# EUROPEAN PATENT SPECIFICATION

(11) **EP 3 413 881 B1**
(45) Date of publication and mention of the grant of the patent: **30.06.2021**
(21) Application number: 17750709.2
(22) Date of filing: 08.02.2017
(51) Int. Cl.: A61K 47/10, A61L 15/44, A61P 17/02, A61K 45/06, A61K 47/22, A61K 9/06, A61L 15/46, A61L 15/60, A61L 26/00, A61K 31/7004, A61K 31/7016

(54) **COMPOSITIONS AND METHODS FOR TREATING CHRONIC WOUNDS**
ZUSAMMENSETZUNGEN UND VERFAHREN ZUR BEHANDLUNG VON CHRONISCHEN WUNDEN
COMPOSITIONS ET PROCÉDÉS DE TRAITEMENT DES PLAIES CHRONIQUES

(30) Priority: 08.02.2016 US 201662292384 P
(43) Date of publication of application: 19.12.2018
(73) Proprietor: Hackensack University Medical Center, Hackensack, NJ 07601 (US)
(72) Inventor: ASLAM, Rummana, Paterson NJ 07504 (US)
(74) Representative: J A Kemp LLP
(86) International application number: PCT/US2017/017044
(87) International publication number: WO 2017/139398

(56) References cited:
- EP-A1- 1 491 185
- WO-A1-2010/100063
- WO-A1-2014/150174
- US-A- 4 401 651
- US-A1- 2003 180 365
- US-A1- 2011 171 284
- CLINICALTRIALS.GOV: "Use of White Granulated Sugar on Wounds. NCT01716273", , 26 October 2012 (2012-10-26), page 1, XP055409050,
- TUMINO, G et al.: "Topical Treatment of Chronic Venous Ulcers With Sucralfate: A Placebo Controlled Randomized Study", International Journal of Molecular Medicine, vol. 22, 2008, page 18, XP002736051,
- SOOD, A et al.: "Wound Dressings and Comparative Effectiveness Data", Advances in Wound Care, vol. 3, no. 8, 2014, page 515, XP055409055,
- KORBER, A et al.: "Three-Dimensional Documentation of Wound Healing: First Results of a New Objective Method for Measurement", Journal der Deutschen Dermatologischen Gesellschaft, vol. 4, no. 10, October 2006 (2006-10), pages 848-854, XP055409056,
- MOUES, CM et al.: "Five Millennia of Wound Care Products - What is New? A Literature Review", Ostomy Wound Management, vol. 55, no. 3, March 2009 (2009-03), pages 16-18,20,22, XP009513106, ISSN: 0889-5899
- BISWAS, A et al.: "Use of Sugar on the Healing of Diabetic Ulcers: A Review", Journal of Diabetes Science and Technology, vol. 4, no. 5, 2010, XP008170382,
- MURANDU, M et al.: "Use of Granulated Sugar Therapy in the Management of Sloughy or Necrotic Wounds: a Pilot Study", Journal of Wound Care, vol. 20, no. 5, 2011, page 206, 208, XP008170378,
- TOPHAM, J: "Why Do Some Cavity Wounds Treated With Honey or Sugar Paste Heal Without Scarring?", Journal of Woundcare, vol. 11, no. 2, 2002, page 3, XP055574768,

## Description

### CROSS-REFERENCE TO RELATED APPLICATION

This application claims the benefit of priority to U.S. Provisional Application No.: 62/292,384, filed on February 8, 2016.

### FIELD OF THE INVENTION

The described invention relates to the fields of cell and molecular biology, therapeutic agents and therapeutic methods of use.

### BACKGROUND

### 1. Anatomy and Physiology of the Skin

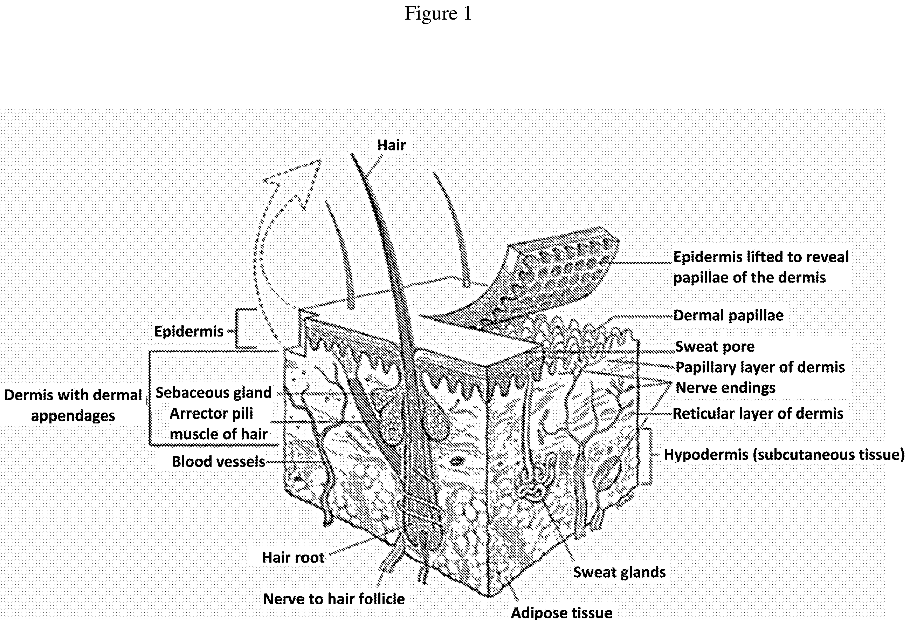

The skin is the largest organ in the body consisting of several layers and plays an important role in biologic homeostasis **(****Figure 1****)**. Its reapproximation over the surface of the wound has long been a primary sign of the completion of a significant portion of wound healing. This reclosure of the defect restores the protective function of the skin, which includes protection from bacteria, toxins, and mechanical forces, as well as providing the barrier to retain essential body fluids. The epidermis, which is composed of several layers beginning with the stratum corneum, is the outermost layer of the skin. The innermost skin layer is the deep dermis. The skin has multiple functions, including thermal regulation, metabolic function (vitamin D metabolism), and immune functions (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

### Epidermis

The epidermis can be considered the body's buffer zone against the environment. It provides protection from trauma, excludes toxins and microbial organisms, and provides a semi-permeable membrane, keeping vital body fluids within the protective envelope. Traditionally, the epidermis has been divided into several layers, of which two represent the most significant ones physiologically. The basal-cell layer, or germinative layer, is of importance because it is the primary source of regenerative cells. In the process of wound healing, this is the area that undergoes mitosis in most instances. The upper epidermis, including stratum and granular layer, is the other area of formation of the normal epidermalbarrier function (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

When the epidermis is injured, the body is subject to invasion by outside agents and loss of body fluids. Epidermal wounds heal primarily by cell migration. Clusters of epidermal cells migrate into the area of damage and cover the defect. These cells are phagocytic and clear the surface of debris and plasma clots. Repair cells originate from local sources that are primarily the dermal appendages and from adjacent intact skin areas. Healing occurs rapidly, and the skin is regenerated and is left unscarred. Blisters are examples of epidermal wounds. They may be small vesicles or larger bullae (blisters greater than 1 cm in diameter) (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

### Stratum Corneum and the Acid Mantle

Stratum corneum is an avascular, multilayer structure that functions as a barrier to the environment and prevents transepidermal water loss. Normal surface skin pH is between 4 and 6.5 in healthy people; it varies according to area of skin on the body. This low pH forms an acid mantle that enhances the skin barrier function. Recent studies have shown that enzymatic activity is involved in the formation of an acid mantle in the stratum corneum. Together, the acid mantle and stratum corneum make the skin less permeable to water and other polar compounds, and indirectly protect the skin from invasion by microorganisms. Damage of the stratum corneum increases the skin pH and, thus, the susceptibility of the skin to bacterial skin infections (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3^{rd} edition, edited by Carrie Sussman and Barbara M. Batesensen, Copyright © 2007, Lippincott Williams & Wilkins, Baltimore MD and Philadelphia PA).

### Other layers of the Epidermis

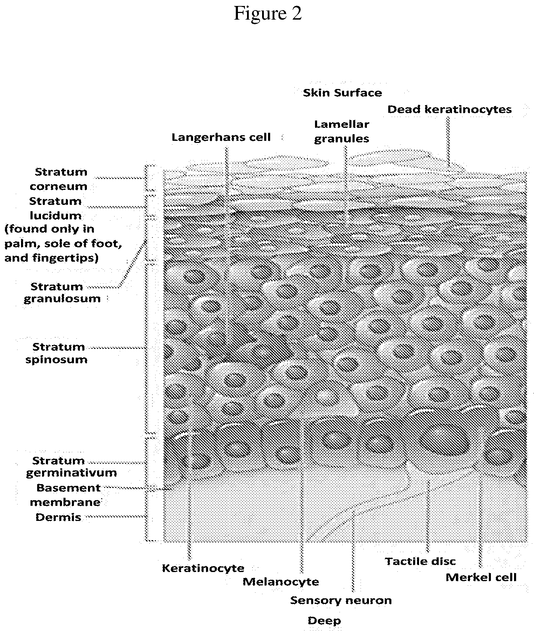

Other layers of the epidermis below the stratum corneum include the stratum lucidum, stratum granulosum, stratum germinativum, and stratum basale. Each contains living cells with specialized functions **(****Figure 2****)**. For example melanin, which is produced by melanocytes in the epidermis, is responsible for the color of the skin. Langerhans cells are involved in immune processing (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

### Dermal Appendages

Dermal appendages, which include hair follicles, sebaceous and sweat glands, fingernails, and toenails, originate in the epidermis and protrude into the dermis **(****Figure 1****)**. Hair follicles and sebaceous and sweat glands contribute epithelial cells for rapid reepithelialization of wounds that do not penetrate through the dermis (termed partial-thickness wounds). The sebaceous glands are responsible for secretions that lubricate the skin, keeping it soft and flexible. They are most numerous in the face and sparse in the palm of the hands and soles of the feet. Sweat gland secretions control skin pH to prevent dermal infections. The sweat glands, dermal blood vessels, and small muscles in the skin (responsible for goose pimples) control temperature on the surface of the body. Nerve endings in the skin include receptors for pain, touch, heat, and cold. Loss of these nerve endings increases the risk for skin breakdown by decreasing the tolerance of the tissue to external forces (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

The basement membrane both separates and connects the epidermis and dermis. When epidermal cells in the basement membrane divide, one cell remains, and the other migrates through the granular layer to the surface stratum corneum. At the surface, the cell dies and forms keratin. Dry keratin on the surface is called scale. Hyperkeratosis (thickened layers of keratin) is found often on the heels and indicates loss of sebaceous gland and sweat gland functions if the patient is diabetic. The basement membrane atrophies with aging; separation between the basement membrane and dermis is one cause for skin tears in the elderly (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

### Dermis

The dermis, or the true skin, is a vascular structure that supports and nourishes the epidermis. In addition, there are sensory nerve endings in the dermis that transmit signals regarding pain, pressure, heat, and cold. The dermis is divided into two layers: the superficial dermis consists of extracellular matrix (collagen, elastin, and ground substances) and contains blood vessels, lymphatics, epithelial cells, connective tissue, muscle, fat, and nerve tissue. The vascular supply of the dermis is responsible for nourishing the epidermis and regulating body temperature. Fibroblasts are responsible for producing the collagen and elastin components of the skin that give it elasticity. Fibronectin and hyaluronic acid are secreted by the fibroblasts (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

The deep dermis is located over the subcutaneous fat; it contains larger networks of blood vessels and collagen fibers to provide tensile strength. It also consists of fibroelastic connective tissue, which is yellow and composed mainly of collagen. Fibroblasts are also present in this tissue layer. The well-vascularized dermis withstands pressure for longer periods of time than subcutaneous tissue or muscle. The collagen in the skin gives the skin its toughness (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

Dermal wounds, e.g., cracks or pustules, involve the epidermis, basal membrane, and dermis. Typically, dermal injuries heal rapidly. Cracks in the dermis can exude serum, blood, or pus, and lead to formation of clots or crusts. Pustules are pus-filled vesicles that often represent infected hair follicle (See, e.g., McLafferty E et al., Nursing Standard 27(3): 35-42; Evans N D et al., Journal of the Mechanical Behavior of Biomedical Materials, Vol. 28, December 2013, 397-409; Wound Care: A Collaborative Practice Manual, 3rd edition, edited by Carrie Sussman and Barbara M. Batesensen, Copyright © 2007, Lippincott Williams & Wilkins, Baltimore MD and Philadelphia PA).

### 2. Wounds and Wound Types

A wound results from damage or disruption to normal anatomical structure and function (Robson MC et al., Curr Probl Surg 2001; 38: 72-140; Velnar T et al., The Journal of International Medical Research 2009; 37: 1528-1542). This can range from a simple break in the epithelial integrity of the skin to deeper, subcutaneous tissue with damage to other structures such as tendons, muscles, vessels, nerves, parenchymal organs and even bone (Alonso JE et al., Surg Clin North Am 1996; 76: 879-903). Irrespective of the cause and form, wounding damages and disrupts the local tissue environment.

Wounds are classified according to various criteria (Robson MC et al., Curr Probl Surg 2001; 38: 72-140; Velnar T et al., The Journal of International Medical Research 2009; 37: 1528-1542). Among these, time plays an important role in injury management and wound repair. Thus, wounds can be clinically categorized as acute or chronic based on their time frame of healing (Velnar T et al., The Journal of International Medical Research 2009; 37: 1528-1542; Robson MC etal., Curr Probl Surg 2001; 38: 72-140; Velnar T et al., The Journal of International Medical Research 2009; 37: 1528-1542; Lazurus GS et al., Arch Dermatol 1994; 130: 489-493).

### 3. Acute Wounds

Wounds that repair themselves and that proceed normally by following a timely and orderly healing pathway, with both functional and anatomical restoration as the end result, are classified as acute wounds. Typically, healing of acute wounds range from 5-10 days or within 30 days. Acute wounds can be acquired, for example, as a result of traumatic loss of tissue or as a result of a surgical procedure.

Acute wounds are a common health problem, with 11 million people affected and approximately 300,000 people hospitalized yearly in the United States (Singer AJ and Dagum AB, N Engl J Med 2008 Sep 4; 359(10): 1037-46; Hostetler SG et al., Wounds 2006; 18: 340-351). Acute wound healing is a well-organized process leading to predictable tissue repair where platelets, keratinocytes, immune surveillance cells, microvascular cells, and fibroblasts play key roles in the restoration of tissue integrity (Singer AJ and Clark RA N Engl J Med 1999 Sep 2; 341(10): 738-746; Falanga V Lancet 2005 Nov 12; 366(9498): 1736-43).

### 4. Chronic Wounds

Chronic wounds are wounds that fail to progress through the normal stages of healing and cannot be repaired in an orderly and timely manner (Robson MC et al., Curr Probl Surg 2001; 38: 72-140; Szycher M and Lee SJ, J Biomater Appl 1992; 7: 142-213; Velnar T et al., The Journal of International Medical Research 2009; 37: 1528-1542). The healing process is incomplete and disturbed by various factors that prolong one or more stages in the phases of hemostasis, inflammation, proliferation or remodeling. These factors include infection, tissue hypoxia, necrosis, exudate and excess levels of inflammatory cytokines (Vanwijck R, Bull Mem Acad R Med Belg 2001; 115: 175-184).

With the estimated number of older adults 65 years or older in the United States almost doubling (from 35 million to 53 million people) by 2030, and the estimated risk of developing diabetes for children born in 2000 as high as 35%, the anticipated risks of diabetes and age-associated nonhealing chronic wounds continue to increase dramatically. Annual chronic wound care costs now exceed $1 billion in the United States alone, and represents ∼2% of total EU financial resources (Gosain A and DiPetro LA, World J Surg 2004 Mar; 28(3): 321-326; Narayan KM et al., JAMA 2003 Oct 8; 290(4): 1884-90; Ramsey SD et al., Diabets Care 1999 Mar; 22(3): 382-387; Bitsch M et al., Scand J Plast Reconstr Surg Hand Surg 2005; 39(3): 162-169).

Chronic wounds can be classified into vascular ulcers (eg, venous and arterial ulcers), pressure ulcers and diabetic ulcers. Some common features shared by each of these include a prolonged or excessive inflammatory phase, persistent infections, formation of drug-resistant microbial biofilms, and the inability of dermal and/or epidermal cells to respond to reparative stimuli (Eming SA et al., J Invest Dermatol 2007 Mar; 127(3): 514-525; Edwards R and Harding KG; Curr Opin Infect Dis 2004 Apr; 17(2): 91-96; Wolcott RD et al., J Wound Care 2008 Aug; 17(8): 333-341). In aggregate, these pathophysiologic phenomena result in the failure of these wounds to heal. The underlying pathologies, however, deviate in different types of chronic wounds.

### Venous Ulcers

Venous ulcers display profound pathological changes that arise secondary to venous valvular incompetence in the deep and superficial veins, which in turn, leads to a constant blood backflow resulting in an increase in venous pressure. Pressure-induced changes in blood vessel wall permeability leads to leakage of fibrin and other plasma components into the perivascular space. Accumulation of fibrin has negative effects on wound healing. Fibrin down-regulates collagen synthesis, leads to formation of pericapillary fibrin cuffs that create a barrier for normal vessel function, and traps blood-derived growth factors (Pardes JB et al., J Cell Physiol 1995 Jan; 162(1): 9-14; Higley HR et al., Br J Dermatol 1995 Jan; 132(1): 79-85; Walker DJ, Orthop Nurs 1999 Sept-Oct; 18(5): 65-74, 95).

### Arterial Ulcers

Arterial ulcers are less common than chronic venous wounds. They are the result of arterial insufficiency caused by atherosclerosis or embolism that can lead to narrowing of arterial lumen and ischemia, which prevents timely healing of minor traumatic injuries (Bonham PA, AACN Clin Issues 2003 Nov; 14(4): 442-456). Unlike venous ulcers, which generally arise between the knee and the ankle, arterial leg wounds may present at any spot distal to arterial perfusion such as a tip of a toe. It is estimated that arterial ulcers affect 100,000 Americans annually (Sieggreen MY and Kline RA, Nurse Pract 2004 Sep; 29(9): 46-52). Unlike venous ulcers that often can be improved with therapeutic compression, chronic wounds linked to arterial insufficiency can be treated successfully only after the restoration of arterial function by revascularization (Bonham PA, AACN Clin Issues 2003 Nov; 14(4): 442-456). Current options for limb revascularization are rather limited and include reconstructive surgery (angioplasty) or pharmaceutical interventions. Because failure of wound revascularization almost inevitably leads to limb amputation in arterial ulcer sufferers, novel techniques allowing for restoration of blood supply to the wound bed are under investigation (Grey JE et al., BMJ 2006 Feb 11: 332(7537): 347-350).

### Pressure Ulcers

Pressure ulcers develop as a result of prolonged unrelieved pressure and shearing force applied to skin and the underlying muscle tissue, leading to a decrease in oxygen tension, ischemia reperfusion injury, and tissue necrosis (Demidova-Rice TN et al., Adv Skin Wound Care 2012 July; 25(7): 304-314). Pressure ulcers are common in patients with compromised mobility and decreased sensory perception (neuropathies) and are exacerbated in individuals with arterial and venous insufficiencies described above (Defloor T, J Clin Nurs 1999 Mar; 8(2): 206-16).

### Diabetic Ulcers

Complications of aging and diabetes can lead to and exacerbate vascular pathologies related to both arterial and venous insufficiencies and worsen pressure ulcers. Other abnormalities leading to development of chronic wounds in diabetic patients (i.e., diabetic ulcers) include neuropathy (often linked to vascular impairment), deficiencies in muscle metabolism, and a number of microvascular pathologies often caused by hyperglycemia (Falanga V, Lancet 2005 Nov 12; 366(9498): 1736-43). Macroscopic pathologies seen in chronic, particularly diabetic, wounds often are linked to cellular phenotypic abnormalities, including low mitogenic/motogenic potential and inability to respond to environmental cues (Demidova-Rice TN et al., Adv Skin Wound Care 2012 July; 25(7): 304-314).

### Chronic Wound Characterization

Even though all of the wounds described may have different origins, each wound is characterized by a chronically inflamed wound bed and a failure to heal (Demidova-Rice TN et al., Adv Skin Wound Care 2012 July; 25(7): 304-314). Excessive recruitment of inflammatory cells to the wound bed often triggered by infection and cell extravasation is facilitated by disproportionate expression of vascular cell adhesion molecule 1 (VCAM1) and interstitial cell adhesion molecule 1 (ICAM1) by resident endothelial cells. Inflammatory cells accumulated inside the chronic wound produce various reactive oxygen species (ROS) that damage structural elements of the extracellular matrix (ECM) and cell membranes and lead to premature cell senescence (Ben-Porath I and Weinberg RA, Int J Biochem Cell Biol 2005 May; 37(5): 861-976). In addition to these direct negative effects, ROS together with proinflammatory cytokines induce production of serine proteinases and matrix metalloproteinases (MMPs) that degrade and inactivate components of the ECM and growth factors necessary for normal cell function (Eming SA et al., J Invest Dermatol 2007 Mar; 127(3): 514-525). Inactivation of proteinase inhibitors by proteolytic degradation augments this process. Although the production of growth factors is often increased in chronic versus acute wounds, their quantity and bio-availability are significantly decreased (Mast BA and Schultz GS, Wound Repair Regen 1996 Oct; 4(4): 411-420; Lauer G et al., J Invest Dermatol 2000 Jul; 115(1): 12-18).

### 5. Phases of Normal (Acute) Wound Healing

Wound healing is a dynamic, interactive process involving soluble mediators, blood cells, extracellular matrix, and parenchymal cells. The wound repair process can be divided into four (4) temporally and spatially overlapping phases: (1) a coagulation phase; (2) an inflammatory phase, (3) a proliferative phase, and (4) a remodeling phase.

### Coagulation Phase

Immediately after injury, platelets adhere to damaged blood vessels, initiate a release reaction, and begin a hemostatic reaction, giving rise to a blood-clotting cascade that prevents excessive bleeding and provides provisional protection for the wounded area. Blood platelets release well over a dozen growth factors, cytokines, and other survival or apoptosis-inducing agents (Weyrich AS and Zimmerman GA, Trends Immunol 2004 Sep; 25(9): 489-495). Key components of the platelet release reaction include platelet-derived growth factor (PDGF) and transforming growth factors A1 and 2 (TGF-A1 and TGF-2), which attract inflammatory cells, such as leukocytes, neutrophils, and macrophages (Singer AF and Clark RA, N Engl J Med 1999 Sep 2; 341(10): 738-746).

### Inflammatory Phase

The inflammatory phase is triggered by capillary damage, which leads to the formation of a blood clot/provisional matrix composed of fibrin and fibronectin. This provisional matrix fills the tissue defect and enables effector cell influx. Platelets present in the clot release multiple cytokines that participate in the recruitment of inflammatory cells (such as neutrophils, monocytes, and macrophages, amongst others), fibroblasts, and endothelial cells (ECs).

### Proliferative Phase

The inflammatory phase is followed by a proliferative phase, in which active angiogenesis creates new capillaries, allowing nutrient delivery to the wound site, notably to support fibroblast proliferation. Fibroblasts present in granulation tissue are activated and acquire a smooth muscle cell-like phenotype, then being referred to as myofibroblasts. Myofibroblasts synthesize and deposit extracellular matrix (ECM) components that replace the provisional matrix. They also have contractile properties mediated by α-smooth muscle actin organized in microfilament bundles or stress fibers. Myofibroblastic differentiation of fibroblastic cells begins with the appearance of the protomyofibroblast, whose stress fibers contain only β- and γ-cytoplasmic actins. Protomyofibroblasts can evolve into differentiated myofibroblasts whose stress fibers contain α-smooth muscle actin.

### Remodeling Phase

The final healing phase involves gradual remodeling of the granulation tissue and reepithelialization. This remodeling process is mediated largely by proteolytic enzymes, especially matrix metalloproteinases (MMPs) and their inhibitors (TIMPs, tissue inhibitors of metalloproteinases). During re-epithelialization, Type III collagen, the main component of granulation tissue, is replaced gradually by type I collagen, the main structural component of the dermis. Elastin, which contributes to skin elasticity and is absent from granulation tissue, also reappears. Cell density normalizes through apoptosis of vascular cells and myofibroblasts (resolution).

### 5.1. Inflammation

Tissue injury causes the disruption of blood vessels and extravasation of blood constituents. A blood clot re-establishes hemostasis and provides a provisional extracellular matrix for cell migration. Platelets not only facilitate the formation of a hemostatic plug but also secrete several mediators of wound healing, such as platelet-derived growth factor, which attract and activate macrophages and fibroblasts (Heldin, C. and Westermark B., In: Clark R., ed. The molecular and cellular biology of wound repair, 2nd Ed. New York, Plenum Press, pp. 249-273, (1996)). It was suggested, however, that, in the absence of hemorrhage, platelets are not essential to wound healing; numerous vasoactive mediators and chemotactic factors are generated by the coagulation and activated-complement pathways and by injured or activated parenchymal cells that were shown to recruit inflammatory leukocytes to the site of injury (*Id.*)*.*

Infiltrating neutrophils cleanse the wounded area of foreign particles and bacteria and then are extruded with the eschar (a dead tissue that falls off (sheds) from healthy skin or is phagocytosed by macrophages). In response to specific chemoattractants, such as fragments of extracellular-matrix protein, transforming growth factor β (TGF-β), and monocyte chemoattractant protein-1 (MCP-1), monocytes also infiltrate the wound site and become activated macrophages that release growth factors (such as platelet-derived growth factor and vascular endothelial growth factor), which initiate the formation of granulation tissue. Macrophages bind to specific proteins of the extracellular matrix by their integrin receptors, an action that stimulates phagocytosis of microorganisms and fragments of extracellular matrix by the macrophages (Brown, E. Phagocytosis, Bioessays, 17:109-117 (1995)). Studies have reported that adherence to the extracellular matrix also stimulates monocytes to undergo metamorphosis into inflammatory or reparative macrophages. These macrophages play an important role in the transition between inflammation and repair (Riches, D., In Clark R., Ed. The molecular and cellular biology of wound repair, 2nd Ed. New York, Plenum Press, pp. 95-141). For example, adherence induces monocytes and macrophages to express Colony-Stimulating Factor-1 (CSF-1), a cytokine necessary for the survival of monocytes and macrophages; Tumor Necrosis Factor-a (TNF-a), a potent inflammatory cytokine; and Platelet-Derived Growth Factor (PDGF), a potent chemoattractant and mitogen for fibroblasts. Other cytokines shown to be expressed by monocytes and macrophages include Transforming Growth Factor (TGF-α), Interleukin-1 (IL-1), Transforming Growth Factor β (TGF-β), and Insulin-like Growth Factor-I (IGF-I) (Rappolee, D. et al., Science, 241, pp. 708-712 (1988)). The monocyte- and macrophage-derived growth factors have been suggested to be necessary for the initiation and propagation of new tissue formation in wounds, because macrophage depleted animals have defective wound repair (Leibovich, S. and Ross, R., Am J Pathol, 78, pp 1-100 (1975)).

Wound healing is a complex biological process that is regulated by numerous growth factors, cytokines, and chemokines. MAPK-activated protein kinase 2 (MK2) is a major regulator of cytokine and chemokine expression, which can recruit local and circulating immunomodulatory cells at wound sites. A recent study with cultured keratinocytes has shown that depleting MK2 through the use of small interfering RNAs severely impairs the ability of the keratinocytes to produce several cytokines, including Tumor Necrosis Factor (TNF) and Interleukin-8 (IL-8) (Johansen et al., J Immunol, 176:1431-1438, 2006). Similarly, in vivo studies have shown that expression levels of several cytokines and chemokines, such as Interleukin-6 (IL-6), Regulated on Activation Normal T cell Expressed and Secreted (RANTES), Tumor Necrosis Factor-alpha (TNF-a), and Interleukin-1 beta (IL-1β), are significantly reduced in the wounds of MK2 knockout mice. These data suggest that MK2 signaling represents an important biochemical pathway that controls the ability of wound infiltrating immunomodulatory cells to produce cytokines and chemokines.

### 5.2. Epithelialization

Reepithelialization of wounds begins within hours after injury. Epidermal cells from skin appendages, such as hair follicles, quickly remove clotted blood and damaged stroma from the wound space. At the same time, the cells undergo phenotypic alteration that includes retraction of intracellular tonofilaments (Paladini, R. et al., J. Cell Biol, 132, pp. 381-397 (1996)); dissolution of most inter-cellular desmosomes, which provide physical connections between the cells; and formation of peripheral cytoplasmic actin filaments, which allow cell movement and migration (Goliger, J. and Paul, D. Mol Biol Cell, 6, pp. 1491-1501 (1995); Gabbiani, G. et al., J Cell Biol, 76, PP. 561-568 (1978)). Furthermore, epidermal and dermal cells no longer adhere to one another, because of the dissolution of hemidesmosomal links between the epidermis and the basement membrane, which allows the lateral movement of epidermal cells. The expression of integrin receptors on epidermal cells allows them to interact with a variety of extracellular-matrix proteins (e.g., fibronectin and vitronectin) that are interspersed with stromal type I collagen at the margin of the wound and interwoven with the fibrin clot in the wound space (Clark, R., J Invest Dermatol, 94, Suppl, pp. 128S-134S (1990)). The migrating epidermal cells dissect the wound, separating desiccated eschar from viable tissue. The path of dissection appears to be determined by the array of integrins that the migrating epidermal cells express on their cell membranes.

The degradation of the extracellular matrix, which is required if the epidermal cells are to migrate between the collagenous dermis and the fibrin eschar, depends on the production of collagenase by epidermal cells (Pilcher, B. et al., J Cell Biol, 137, pp. 1445-1457 (1997)), as well as the activation of plasmin by plasminogen activator produced by the epidermal cells (Bugge, T. et al., Cell, 87, 709-719 (1996)). Plasminogen activator also activates collagenase (matrix metalloproteinase-1) (Mignatti, P. et al., Proteinases and Tissue Remodeling. In Clark, R. Ed. The molecular and cellular biology of wound repair. 2nd Ed. New York, Plenum Press, 427-474 (1996)) and facilitates the degradation of collagen and extracellular-matrix proteins.

One to two days after injury, epidermal cells at the wound margin begin to proliferate behind the actively migrating cells. The stimuli for the migration and proliferation of epidermal cells during reepithelialization have not been determined, but several possibilities have been suggested. The absence of neighbor cells at the margin of the wound (the "free edge" effect) may signal both migration and proliferation of epidermal cells. Local release of growth factors and increased expression of growth-factor receptors may also stimulate these processes. Leading contenders include Epidermal Growth Factor (EGF), Transforming Growth Factor-a (TGF-α), and Keratinocyte Growth Factor (KGF) (Nanney, L. and King, L. Epidermal Growth Factor and Transforming Growth Factor-a. In Clark, R. Ed. The molecular and cellular biology of wound repair. 2nd Ed. New York, Plenum Press, pp. 171-194 (1996); Werner, S. et al., Science, 266, pp. 819--822 (1994); Abraham, J. and Klagsburn, M. Modulation of Wound Repair by Members of the Fibroblast Growth Factor family. In Clark, R. Ed. The molecular and cellular biology of wound repair. 2nd Ed. New York, Plenum Press, pp. 195-248 (1996)). As re-epithelialization ensues, basement-membrane proteins reappear in a very ordered sequence from the margin of the wound inward, in a zipper-like fashion (Clark R. et al., J. Invest Dermatol, 79, pp. 264-269 (1982)). Epidermal cells revert to their normal phenotype, once again firmly attaching to the reestablished basement membrane and underlying dermis.

### 5.3. Formation of Granulation Tissue

New stroma, often called granulation tissue, begins to invade the wound space approximately four days after injury. Numerous new capillaries endow the new stroma with its granular appearance. Macrophages, fibroblasts, and blood vessels move into the wound space at the same time (Hunt, T. ed. Wound Healing and Wound Infection: Theory and Surgical Practice. New York, Appleton-Century-Crofts (1980)). The macrophages provide a continuing source of growth factors necessary to stimulate fibroplasia and angiogenesis; the fibroblasts produce the new extracellular matrix necessary to support cell ingrowth; and blood vessels carry oxygen and nutrients necessary to sustain cell metabolism.

Growth factors, especially Platelet-Derived Growth Factor-4 (PDGF-4) and Transforming Growth Factor β-1 (TGF- β1) (Roberts, A. and Sporn, M, Transforming Growth Factor-1, In Clark, R. ed. The molecular and cellular biology of wound repair. 2nd Ed. New York, Plenum Press, pp. 275-308 (1996)) in concert with the extracellular-matrix molecules (Gray, A. et al., J Cell Sci, 104, pp. 409-413 (1993); Xu, J. and Clark, R., J Cell Biol, 132, pp. 239-149 (1996)), were shown to stimulate fibroblasts of the tissue around the wound to proliferate, express appropriate integrin receptors, and migrate into the wound space. It was reported that platelet-derived growth factor accelerates the healing of chronic pressure sores (Robson, M. et al., Lancet, 339, pp. 23-25 (1992) and diabetic ulcers (Steed, D., J Vasc Surg, 21, pp. 71-78 (1995)). In some other cases, basic Fibroblast Growth Factor (bFGF) was effective for treating chronic pressure sores (Robson, M. et al., Ann Surg, 216, pp. 401-406 (1992).

The structural molecules of newly formed extracellular matrix, termed the provisional matrix (Clark, R. et al., J. Invest Dermatol, 79, pp. 264-269, 1982), contribute to the formation of granulation tissue by providing a scaffold or conduit for cell migration. These molecules include fibrin, fibronectin, and hyaluronic acid (Greiling, D. and Clark R., J. Cell Sci, 110, pp. 861-870 (1997)). The appearance of fibronectin and the appropriate integrin receptors that bind fibronectin, fibrin, or both on fibroblasts was suggested to be the ratelimiting step in the formation of granulation tissue. While the fibroblasts are responsible for the synthesis, deposition, and remodeling of the extracellular matrix, the extracellular matrix itself can have a positive or negative effect on the ability of fibroblasts to perform these tasks, and to generally interact with their environment (Xu, J. and Clark, R., J Cell Sci, 132, pp. 239-249 (1996); Clark, R. et al., J Cell Sci, 108, pp. 1251-1261).

Cell movement into a blood clot of cross-linked fibrin or into tightly woven extracellular matrix requires an active proteolytic system that can cleave a path for cell migration. A variety of fibroblast-derived enzymes, in addition to serum-derived plasmin, are suggested to be potential candidates for this task, including plasminogen activator, collagenases, gelatinase A, and stromelysin (Mignatti, P. et al., Proteinases and Tissue Remodeling. In Clark, R. Ed. The molecular and cellular biology of wound repair. 2nd Ed. New York, Plenum Press, 427-474 (1996); Vaalamo, M. et al., J Invest Dermatol, 109, pp. 96-101 (1997)). After migrating into wounds, fibroblasts commence the synthesis of extracellular matrix. The provisional extracellular matrix is replaced gradually with a collagenous matrix, perhaps in response to Transforming Growth Factor-β1 (TGF-β1) signaling (Clark, R. et al., J Cell Sci, 108, pp. 1251-1261 (1995); Welch, M. et al., J. Cell Biol, 110, pp. 133-145 (1990))

Once an abundant collagen matrix has been deposited in the wound, the fibroblasts stop producing collagen, and the fibroblast-rich granulation tissue is replaced by a relatively acellular scar. Cells in the wound undergo apoptosis triggered by unknown signals. It was reported that dysregulation of these processes occurs in fibrotic disorders, such as keloid formation, hypertrophic scars, morphea, and scleroderma.

### 5.4. Neovascularization

The formation of new blood vessels (neovascularization) is necessary to sustain the newly formed granulation tissue. Angiogenesis is a complex process that relies on extracellular matrix in the wound bed as well as migration and mitogenic stimulation of endothelial cells (Madri, J. et al., Angiogenesis in Clark, R. Ed. The molecular and cellular biology of wound repair. 2nd Ed. New York, Plenum Press, pp. 355-371 (1996)). Neovascularization differs from angiogenesis in that angiogenesis is mainly characterized by protrusion and outgrowth of capillary buds and sprouts from pre-existing blood vessels, whereas, neovascularization is characterized by proliferation of blood vessels in tissue not normally containing blood vessels or proliferation of a different type of blood vessel not normally found in a particular tissue. The induction of angiogenesis was initially attributed to acidic or basic Fibroblast Growth Factor. Subsequently, many other molecules have also been found to have angiogenic activity, including vascular endothelial growth factor (VEGF), Transforming Growth Factor-β (TGF-β), angiogenin, angiotropin, angiopoietin-1, and thrombospondin (Folkman, J. and D'Amore, P, Cell, 87, pp. 1153-1155 (1996)).

Low oxygen tension and elevated lactic acid were suggested also to stimulate angiogenesis. These molecules induce angiogenesis by stimulating the production of basic Fibroblast Growth Factor (FGF) and Vascular Endothelial Growth Factor (VEGF) by macrophages and endothelial cells. For example, it was reported that activated epidermal cells of the wound secrete large quantities of Vascular Endothelial cell Growth Factor (VEGF) (Brown, L. et al., J Exp Med, 176, 1375-1379 (1992)).

Basic fibroblast growth factor was hypothesized to set the stage for angiogenesis during the first three days of wound repair, whereas vascular endothelial-cell growth factor is critical for angiogenesis during the formation of granulation tissue on days 4 through 7 (Nissen, N. et al., Am J Pathol, 152, 1445-1552 (1998)).

In addition to angiogenesis factors, it was shown that appropriate extracellular matrix and endothelial receptors for the provisional matrix are necessary for angiogenesis. Proliferating microvascular endothelial cells adjacent to and within wounds transiently deposit increased amounts of fibronectin within the vessel wall (Clark, R. et al., J. Exp Med, 156, 646-651 (1982)). Since angiogenesis requires the expression of functional fibronectin receptors by endothelial cells (Brooks, P. et al., Science, 264, 569-571 (1994)), it was suggested that perivascular fibronectin acts as a conduit for the movement of endothelial cells into the wound. In addition, protease expression and activity were shown to also be necessary for angiogenesis (Pintucci, G. et al., Semin Thromb Hemost, 22, 517-524 (1996)).

The series of events leading to angiogenesis has been proposed as follows. Injury causes destruction of tissue and hypoxia. Angiogenesis factors, such as acidic and basic Fibroblast Growth Factor (FGF), are released immediately from macrophages after cell disruption, and the production of vascular endothelial-cell growth factor by epidermal cells is stimulated by hypoxia. Proteolytic enzymes released into the connective tissue degrade extracellular-matrix proteins. Fragments of these proteins recruit peripheral-blood monocytes to the site of injury, where they become activated macrophages and release angiogenesis factors. Certain macrophage angiogenesis factors, such as basic fibroblast growth factor (bFGF), stimulate endothelial cells to release plasminogen activator and procollagenase. Plasminogen activator converts plasminogen to plasmin and procollagenase to active collagenase, and in concert these two proteases digest basement membranes. The fragmentation of the basement membrane allows endothelial cells stimulated by angiogenesis factors to migrate and form new blood vessels at the injured site. Once the wound is filled with new granulation tissue, angiogenesis ceases and many of the new blood vessels disintegrate as a result of apoptosis (Ilan, N. et al., J Cell Sci, 111, 3621-3631 (1998)). This programmed cell death has been suggested to be regulated by a variety of matrix molecules, such as thrombospondins 1 and 2, and anti-angiogenesis factors, such as angiostatin, endostatin, and angiopoietin 2 (Folkman, J., Angiogenesis and angiogenesis inhibition: an overview, EXS, 79, 1-8, (1997)).

### 5.5. Wound Contraction and Extracellular Matrix Reorganization

Wound contraction involves a complex and orchestrated interaction of cells, extracellular matrix, and cytokines. During the second week of healing, fibroblasts assume a myofibroblast phenotype characterized by large bundles of actin-containing microfilaments disposed along the cytoplasmic face of the plasma membrane of the cells and by cell-cell and cell-matrix linkages (Welch, M. et al., J Cell Biol, 110, 133-145 (1990); Desmouliere, A. and Gabbiani, G. The role of the myofibroblast in wound healing and fibrocontractive diseases. In Clark, R. Ed. The molecular and cellular biology of wound repair. 2nd Ed. New York, Plenum Press, pp. 391-423 (1996)). The appearance of the myofibroblasts corresponds to the commencement of connective-tissue compaction and the contraction of the wound. This contraction was suggested to require stimulation by Transforming Growth Factor (TGF)-β1 or β2 and Platelet-Derived Growth Factor (PDGF), attachment of fibroblasts to the collagen matrix through integrin receptors, and cross-links between individual bundles of collagen. (Montesano, R. and Orci, Proc Natl Acad Sci USA, 85, 4894-4897 (1988); Clark, R. et al., J Clin Invest, 84, 1036-1040 (1989); Schiro, J. et al., Cell, 67, 403-410 (1991); Woodley, D. et al., J Invest Dermatol, 97, 580-585 (1991)).

Collagen remodeling during the transition from granulation tissue to scar is dependent on continued synthesis and catabolism of collagen at a low rate. The degradation of collagen in the wound is controlled by several proteolytic enzymes, termed matrix metalloproteinases (MMP), which are secreted by macrophages, epidermal cells, and endothelial cells, as well as fibroblasts (Mignatti, P. et al., Proteinases and Tissue Remodeling. In Clark, R. Ed. The molecular and cellular biology of wound repair. 2nd Ed. New York, Plenum Press, 427-474 (1996)). Various phases of wound repair have been suggested to rely on distinct combinations of matrix metalloproteinases and tissue inhibitors of metalloproteinases (Madlener, M. et al, Exp Cell Res, 242, 201-210 (1998)).

Wounds gain only about 20 percent of their final strength in the first three weeks, during which fibrillar collagen has accumulated relatively rapidly and has been remodeled by contraction of the wound. Thereafter, the rate at which wounds gain tensile strength is slow, reflecting a much slower rate of accumulation of collagen and collagen remodeling with the formation of larger collagen bundles and an increase in the number of intermolecular cross-links. Nevertheless, it was suggested that wounds never attain the same breaking strength (the tension at which skin breaks) as uninjured skin, and that, at maximal strength, a scar is only 70 percent as strong as normal skin (Levenson, S. et al., Ann Surg, 161. 293-308 (1965)).

### 6. Chronic Wound Healing

Unlike acute wounds, which generally heal without significant interventions, all types of chronic wounds represent major challenges for patients and caregivers. It is now understood that the inability of the chronic wound to heal is caused by both cellular and molecular abnormalities occurring within the wound bed (Demidova-Rice TN et al., Adv Skin Wound Care 2012 July; 25(7): 304-314). However, proper diagnosis of wound etiology, selection of effective treatment, and prevention of wound reoccurrence remain a problem for chronic wound sufferers and healthcare providers (Bonham PA, AACN Clin Issues 2003 Nov; 14(4): 442-456).

### 6.1 Phenotypic Abnormalities in Chronic Wound Cells

Phenotypic abnormalities of epidermis- and dermis-derived cells residing in chronic wounds include lower density of growth factor receptors and lower mitogenic potential preventing them from responding properly to environmental cues. For example, fibroblasts, isolated from patients with chronic diabetic, chronic non-diabetic wounds, or patients with venous insufficiency, have lower mitogenic response to platelet-derived growth factor (PDGF-AB), insulin-like growth factor (IGF), basic fibroblast growth factor (bFGF), and epidermal growth factor (EGF) applied separately or in combination; likely due to a decrease in receptor density (Demidova-Rice TN et al., Adv Skin Wound Care 2012 July; 25(7): 304-314; Loot MA et al., Eur J Cell Biol 2002 Mar; 81(3): 153-160; Seidman C et al.,Ann Vasc Surg. 2003; 17: 239-244; Vasquez R et al., Vasc Endovascular Surg 2004 Jul-Aug; 38(4): 355-360). Moreover, fibroblasts isolated from leptin receptor-deficient diabetic mice, and from patients with chronic venous insufficiency, have reduced motility, compared with normal fibroblasts (Raffetto JD et al., J Vasc Surg 2001 Jun; 33(6): 1233-1241; Lerman OZ et al., Am J Pathol 2003 Jan; 162(1): 303-312). These cellular abnormalities impede the formation of granulation tissue and ECM deposition, leading to formation of nonhealing wounds.

Keratinocytes derived from chronic ulcers have also been reported to possess a "chronic wound-associated" phenotype (Usui ML et al., J Histochem Cytochem 2008 Jul; 56(7): 687-696). By overexpressing the proliferation marker Ki67, these cells up-regulate expression of several cell cycle-associated genes, such as cyclin-dependent kinase 1 (CDK1) and cyclin B1, suggesting a hyperproliferative status (Stojadinovic O et al., J Cell Mol Med 2008 Dec; 12(6B): 2675-2690). However, these chronic wound-derived keratinocytes exhibit impaired migratory potential. The mechanisms of this impairment are not completely understood but have been linked to decreased production of laminin 332 (formerly known as laminin 5), which is an important epithelial ECM component and substrate for injury-induced keratinocyte migration (Usui ML et al., J Histochem Cytochem 2008 Jul; 56(7): 687-696). These cells possess an increased activation of the A-catenin/c-myc pathway and do not express markers of differentiation, particularly keratin 10 and keratin 2 (Stojadinovic O et al., AM J Pathol 2005 Jul; 167(1): 59-69). Finally, several genes encoding a variety of growth factors are down- or up-regulated. For example, vascular endothelial growth factor (VEGF), epiregulin, and transforming growth factor-A2 (TGF-A2) expression are decreased, whereas PDGF and platelet-derived endothelial growth factor encoding genes are up-regulated (Stojadinovic O et al., J Cell Mol Med 2008 Dec; 12(6B): 2675-2690).

### 6.2 Disturbances in the ECM Microenvironment Contribute to Sustaining a Chronic Wound

The microenvironment of the chronic wound bed is denoted by a matrix. Information about the differences in chemical composition of the ECM found in chronic and acute wounds is scarce and controversial (Demidova-Rice TN et al., Adv Skin Wound Care 2012 July; 25(7): 304-314). It is known, however, that deposition of a number of matrix components is different in chronic as compared with acute wounds. For example, chronic wounds are characterized by prolonged or insufficient expression of fibronectin, chondroitin sulfate, and tenascin, which gives rise to impaired cellular proliferation and migration (Loots MA et al., J Invest Dermatol 1998 Nov; 111(5): 850-857; Agren MS et al., J Invest Dermatol 1999 Apr; 112(4): 463-469). Recently, reduced production of laminin 332 - a basement membrane component that serves as a haptotactic substrate for postinjury keratinocyte motility (meaning migration induced by ECM proteins when substratum-bound) - was found to be one of the reasons for impaired re-epithelialization and wound healing (Usui ML et al., J Histochem Cytochem 2008 Jul; 56(7): 687-696). Changes in the ECM, including posttranslational modification of key structural components, can also negatively influence cellular responses to injury. For instance, matrix glycation is often seen in diabetic patients and is likely to be responsible for or linked to premature cell senescence, apoptosis, inhibition of cell proliferation, migration, and angiogenic sprout formation (Kuo PC et al., In Vitro Cell Dev Biol Anim 2007 Nov-Dec; 43(10): 338-343). Glycation adds to matrix instability and disrupts matrix assembly and interactions between collagen and its binding partners, including heparan sulfate proteoglycans (Reigle KL et al., J Cell Biochem 2008 Aug 1; 104(5): 1684-1698; Liao H et al., Biomaterials 2009 Mar; 30(9): 1689-1696). High glucose has also been shown to stimulate MMP production by fibroblasts, macrophages, and endothelial cells, thus contributing to a cycle of matrix degradation detrimental for cell survival and wound healing (Death AK et al., Atherosclerosis 2003 Jun; 168(2): 263-269). Matrix instability that results from glycation and insufficient intermolecular cross-linking under hypoxic conditions and excessive matrix degradation by MMPs are also detrimental to the healing process (Dalton SJ et al., J Invest Dermatol 2007 Apr; 127(4): 958-968). Matrix instability prevents normal cell-matrix interactions necessary for cell survival and function and, ultimately, injury repair.

### 6.3 Biofilms and Chronic Wound Bed

Infection is an extrinsic factor that causes delay of wound healing, contributing to wound chronicity, morbidity, and mortality (Bader MS, Am Fam Physician 2008 Jul 1; 78(1): 71-79). High bacterial counts of greater than 10⁵ viable bacteria or any number of group A-hemolytic streptococci are considered detrimental. Live bacteria (and subsequently produced bacterial toxins) induce excessive inflammatory responses and tissue damage that can lead to abscess, cellulites, osteomyelitis, or limb loss (in diabetic patients) (Edwards R and Harding KG, Curr Opin Infect Dis 2004 Apr; 17(2): 91-96; Ovington L, Ostomy Wound Manage 2003 July; 49(7A Suppl): 8-12). Furthermore, recruited inflammatory cells produce a number of proteases (e.g., MMPs), which degrade the ECM and growth factors present within the wound bed (Demidova-Rice TN et al., Adv Skin Wound Care 2012 July; 25(7): 304-314). Bacteria that colonize chronic wounds often form polymicrobial communities called biofilms (James GA et al., Wound Repair Regen 2008 Jan-Feb; 16(1): 37-44). These complex structures are composed of microbial cells embedded in a secreted polymer matrix. The polymer matrix provides an optimal environment for bacterial cell survival, enabling the bacteria's escape from host immune surveillance/defense and resistance to antibiotic treatment (Martin JM et al., J Invest Dermatol 2010 Jan; 130(1): 38-48). Although biofilms are prevalent in chronic wounds and significantly delay re-epithelialization in animal models, it remains unclear precisely how they delay healing (Falanga V. Wound healing and its impairment in the diabetic foot. Lancet. 2005;366:1736-43; James GA, Swogger E, Wolcott R, et al. Biofilms in chronic wounds. Wound Repair Regen. 2008;16(1):37-44; Schierle Clark F., De la Garza Mauricio, Mustoe Thomas A., Galiano Robert D. Staphylococcal biofilms impair wound healing by delaying reepithelialization in a murine cutaneous wound model. Wound Repair Regen. 2009;17:354-9). Increased bacterial survival and enhanced production of virulence factors are likely explanations. Nonetheless, it is possible that extracellular biofilm components possess or display a toxic phenotype for host cell functionality and therefore impede healing. It has been demonstrated that hindering biofilm formation by RNAIII-inhibiting peptide reverses wound-healing delays induced by bacterial biofilms (Schierle Clark F., De la Garza Mauricio, Mustoe Thomas A., Galiano Robert D. Staphylococcal biofilms impair wound healing by delaying reepithelialization in a murine cutaneous wound model. Wound Repair Regen. 2009;17:354-9; Cirioni O, Ghiselli R, Minardi D, et al. RNAIII-inhibiting peptide affects biofilm formation in a rat model of staphylococcal ureteral stent infection. Antimicrob Agents Chemother. 2007;51:4518-20).

### 7. Chronic Wound Care

Successful treatment of a chronic wound requires a detailed understanding of the molecular and cellular components present within the wound bed. Currently, chronic (and acute) wounds of different etiologies are treated using a multistep approach based on contemporary knowledge of wound healing and known by the acronym TIME: nonviable tissues (T) from within and around a wound are removed using surgical debridement or debriding agents, such as bacterial collagenase; infection and inflammation (I) are minimized with antibiotics and anti-inflammatory preparations; moisture (M) imbalance is corrected, generally with carefully selected dressings; and epithelialization (E) and granulation tissue formation are promoted by the application of specific therapies, such as growth factors (Demidova-Rice T N et al. Acute and Impaired Wound Healing: Pathophysiology and Current Methods for Drug Delivery, Part 1: Normal and Chronic Wounds: Biology, Causes, and Approaches to Care. Adv Skin Wound Care. 2012 Jul; 25(7): 304-314;Tomic-Canic M, Ayello EA, Stojadinovic O, Golinko MS, Brem H. Using gene transcription patterns (bar coding scans) to guide wound debridement and healing. Adv Skin Wound Care. 2008;21:487-92).

The use of TIME strategy is not always sufficient, with some wounds remaining nonresponsive to current therapies. Therefore, refined methods that will enable personalized therapeutics represent interesting options. To this end, a wound diagnosis technology called "bar coding" of the wound has been proposed (Tomic-Canic M, Ayello EA, Stojadinovic O, Golinko MS, Brem H. Using gene transcription patterns (bar coding scans) to guide wound debridement and healing. Adv Skin Wound Care. 2008;21:487-92). "Bar coding" diagnosis uses sampling of chronic wound fluids and/or collection of tissue biopsies that allow for identification of markers of wound chronicity, such as growth factors and growth factor receptors, matrix metalloproteinases (MMPs), members of the A-catenin/c-myc pathway, and keratinocyte differentiation markers. Wound bar coding can be used for both guiding wound debridement and treatment regimens. Necessary levels of dead tissue removal can be determined, for example, by the presence of A-catenin/c-myc-positive cells that, although they can proliferate, cannot migrate or differentiate and therefore have to be removed (Demidova-Rice T N et al. Acute and Impaired Wound Healing: Pathophysiology and Current Methods for Drug Delivery, Part 1: Normal and Chronic Wounds: Biology, Causes, and Approaches to Care. Adv Skin Wound Care. 2012 Jul; 25(7): 304-314).

In addition, treatment strategies can be adjusted based on the needs of individual patients or bar-coding results. For example, if cells residing within a wound express low levels of growth factors, growth factor receptors, and high levels of MMPs as determined by enzyme-linked immunosorbent assay, growth factors or patient-derived "engineered" cells may be administered to help restore the wound microenvironment to a healing phenotype.

### 7.1 Wound Bed Preparation

The term wound bed preparation refers to management of the wound to accelerate endogenous healing or to facilitate the effectiveness of advanced and adjunctive wound therapies. Wound bed preparation involves processes that change the biochemical, cellular and molecular milieu of a chronic, non-healing wound, to an acute healing wound. Examples of wound bed preparation processes are listed in Table 1.

**Table 1. Wound bed preparation processes**

| **Designation** | **Description** |
|---|---|
| A | Removal of wound and surrounding tissue edema which is secondary to chronic inflammation |
| B | Decrease bacterial burden |
| C | Decrease fungal burden |
| D | Decrease necrotic tissue and slough |
| E | Increase healthy granulation tissue formation (granulation tissue is composed of new collagen formation and new blood vessel growth) |
| F | Decrease hyper-granulation which is secondary to increased water and bacterial content |
| G | Hydration of wound bed |
| H | Absorbing drainage from wound bed |
| I | Achieving moisture balance on wound bed by balance of hydration and absorbance |
| J | Improve wound contraction and closure by promoting healthy collagen and blood vessel growth and epithelialization |
| K | Decrease wound pain |

### Management of Tissue Necrosis

The nonviable necrotic tissue overlying the wound base obscures an assessment of the depth and condition of the wound. In addition, the presence of necrotic tissue is a nidus for bacterial growth and serves as a physical barrier that may obscure the signs of local wound infection. Bacterial colonies in necrotic tissue can produce damaging metalloproteinases that adversely affect extracellular matrix components during the healing process. Bacteria may also compete for the scarce local resources (e.g., oxygen, nutrition and building blocks) that are necessary for wound healing (Halim AS et al. Wound bed preparation from a clinical perspective. Indian J Plast Surg. 2012 May-Aug; 45(2): 193-202).

Tissue management is the process of removing necrotic or devitalized tissue, bacteria and cells that impede the healing process to reduce wound contamination and tissue destruction. The aim is to restore a viable wound base with a functional extracellular matrix. Chronic wounds are converted into acute wounds with the removal of the necrotic burden of senescent cells, the extracellular matrix, inflammatory enzymes and biofilms that contain bacterial colonies (Panuncialman J, Falanga V. The science of wound bed preparation. Clin Plast Surg. 2007;34:621-32).

The options for debridement include surgical, mechanical, autolytic, enzymatic and biological methods. The surgical method is the fastest means and allows the accurate assessment of the severity and extent of the wound. It is particularly important in life- or limb-threatening infections with necrotic eschar or gangrene. Surgical debridement is also indicated for wounds with extensive or adhering eschar, in which the rapid clearance of necrotic tissue is required. However, it is non-selective because normal healthy tissue may be removed at the same time (Panuncialman J, Falanga V. The science of wound bed preparation. Clin Plast Surg. 2007;34:621-32). Surgical debridement may be limited by the bleeding tendency and pain tolerance of the patient and the availability of surgical expertise.

Versajet hydrosurgery represents a technical advance in surgical debridement that uses tangential hydro-dissection. The energy of the saline beam of the Versajet tangentially excises the wound surface with minimal damage to the surrounding healthy tissue. It is as effective in removing bacteria as the high-powered pulse lavage systems (Granick MS, Tenenhaus M, Knox KR, Ulm JP. Comparison of wound irrigation and tangential hydrodissection in bacterial clearance of contaminated wounds: Results of a randomized, controlled clinical study. Ostomy Wound Manage. 2007;53:64-6). It improves treatment outcomes by decreasing the number of surgeries required for the surgical wound bed preparation of acute and chronic wounds. Versajet has been noted to be a feasible, simple and safe technique that hastens surgical debridement of burns and adds more precision in burn wound excision (Granick M, Boykin J, Gamelli R, Schultz G, Tenenhaus M. Toward a common language: surgical wound bed preparation and debridement. Wound Repair Regen. 2006;14()(Suppl 1):S1-10; Gravante G, Delogu D, Esposito G, Montone A. Versajet hydrosurgery versus classic escharectomy for burn debridement: A prospective randomized trial. J Burn Care Res. 2007;28:720-4).

Mechanical debridement involves methods such as wet-to-dry dressing and pressure irrigation. Wet-to-dry dressing is performed by leaving wet gauze in direct contact with wound surfaces and removing it when dry, together with any adhering slough tissue. It causes excessive pain as well as bleeding and removes the new healing epithelium when the dressing is changed. Pressure irrigation is the forced irrigation of saline from a syringe through cannulas to remove necrotic tissues that are loose and superficial. This technique should not be used when the solution may collect and be trapped in dead spaces (Sibbald RG, Goodman L, Woo KY, Krasner DL, Smart H, Tariq G, et al. Special considerations in wound bed preparation 2011: An update(c) Adv Skin Wound Care. 2011;24:415-36).

Edinburg University Solution of Lime (EUSOL), traditionally used for wound debridement, is a cheap bleaching and antiseptic agent containing calcium hypochloride and boric acid. The solution was developed around World War I for the treatment of trench foot (Patton MA. Eusol: The continuing controversy. BMJ. 1992;304:1636). EUSOL is usually administered as wet-to-dry dressing three to four times a day for chemical debridement of a sloughy wound. Although it has been a subject of controversy over the past two to three decades due to its tissue toxicity and delayed healing of clean granulating wound, it still plays a role in wound debridement if properly administered.

Autolytic debridement uses the inherent ability of the body to digest and remove necrotic tissue. A moist-retention dressing is applied to the wound to provide a moist wound healing environment that allows the necrotic tissue to be liquefied by endogenous enzymes or phagocytic cells. The application of a hydrogel that is covered by a polyurethane film is an example of the dressing used for this type of debridement. This method is relatively easy to perform, requires limited technical skills and involves minimal pain. However, it is time consuming and carries the risk of invasive infection and wound edge maceration. Autolytic debridement is indicated in wounds with a minimal necrotic load, wounds that need more aggressive debridement requiring anaesthesia or in patients who are unable to tolerate pain (Knox KR, Datiashvili RO, Granick MS. Surgical wound bed preparation of chronic and acute wounds. Clin Plast Surg. 2007;34:633-41).

Enzymatic debridement uses manufactured enzymes, such as collagenase and papainurea, as debriding agents to dissolve necrotic tissue. Papain is a broad-spectrum enzyme that is useful for bulk debridement, whereas collagenase is gentler on viable cells. Enzymatic debridement is suitable for nonsurgical patients and can be effectively combined with moist wound healing. Enzymatic debridement is expensive and has a limited role in the treatment of selected chronic wounds (Halim AS et al. Wound bed preparation from a clinical perspective. Indian J Plast Surg. 2012 May-Aug; 45(2): 193-202).

In biological debridement (also known as maggot debridement therapy or MDT), the larvae of green butterfly (Lucila serricata) digest necrotic tissue and secrete bactericidal enzymes. These larvae have the ability to debride wound beds, provide antimicrobial activity and also stimulate wound healing (Marineau ML et al. Maggot Debridement Therapy in the Treatment of Complex Diabetic Wounds. Hawaii Med J. 2011; 70(6): 121-124). MDT is effective in wounds infected with methicillin-resistant staphylococcus aureus (MRSA) and/or beta hemolytic streptococcus. Biological debridement is considered to be a secondary debridement method after surgical debridement or for patients who are not fit for surgical debridement. The uncomfortable feeling generated by this treatment makes it unpopular (Halim AS et al. Wound bed preparation from a clinical perspective. Indian J Plast Surg. 2012 May-Aug; 45(2): 193-202).

### Restoration of the Bacterial Balance

Chronic wound beds are often colonized by various species of bacteria or fungal organisms due to the prolonged opening of the wound, poor blood flow and underlying disease process. The bacterial balance is achieved by controlling the bacterial burden in terms of its density and pathogenicity (Panuncialman J, Falanga V. The science of wound bed preparation. Clin Plast Surg. 2007;34:621-32). The presence of bacteria in the wound beds ranges from contamination, colonization and critical colonization to invasive infection. Critical colonization is the presence of replicating microorganisms that are beginning to cause local tissue damage. It is the point at which the host defenses are unable to maintain the balance of organisms at colonization. It is noted clinically by signs such as a change in the color of the wound bed, friable and unhealthy granulation tissue, abnormal odour, increased serous exudate and pain at the wound site. Identifying critical colonization is important because it is the level at which wound healing begins to be delayed, even before the occurrence of invasive infection (Halim AS et al. Wound bed preparation from a clinical perspective. Indian J Plast Surg. 2012 May-Aug; 45(2): 193-202).

Bacterial levels of 10⁶ or more per gram of tissue are generally considered an infection because these levels adversely affect wound healing. The presence of replicating microorganisms in the wound causes injury to the host due to the release of toxins, competitive metabolism and inflammation. In acute and subacute wounds, infection is recognized clinically as local signs of advancing redness, warmth of the skin surrounding the wound, edema, increasing pain and tenderness, a foul odour and increased or purulent drainage. Systemic signs include fever, tachycardia and even changes in mental status in cases of sepsis. The patient also may have an increased white blood cell count. However, chronic wounds more than 3 months old are less likely to have advancing inflammation and constitutional symptoms (Gardner SE, Frantz RA, Doebbeling BN. The validity of the clinical signs and symptoms used to identify localized chronic wound infection. Wound Repair Regen. 2001; 9:178-86). Chronic wound infection is recognized by an increasing ulcer size, increasing exudate production and friable unhealthy granulation tissue (Halim AS et al. Wound bed preparation from a clinical perspective. Indian J Plast Surg. 2012 May-Aug; 45(2): 193-202).

As chronic wounds are often colonized by bacteria, obtaining and interpreting laboratory data should be performed in correlation with clinical findings. Although a tissue biopsy may be more ideal, a properly performed deep wound swab is also useful. Apart from a quantitative bacterial count, the presence of four or more organisms in the wound bed can be predictive of delayed wound healing, especially since certain organisms exhibit synergism (Trengove NJ, Stacey MC, McGechie DF, Mata S. Qualitative bacteriology and leg ulcer healing. J Wound Care. 1996;5:277-80).

The critically colonized wound should be treated with topical antimicrobial dressings. Sustained-release silver dressings have gained in popularity due to their efficacy, low resistance and broad-spectrum antimicrobial actions, especially when *pseudomonas* or MRSA infection is a concern. The wound should be cleansed with low toxicity topical antiseptic solutions (e.g. normal saline or chlorhexidine solution), instead of cytotoxic solutions such as povidone-iodine. Topical antiseptics have the advantages of a broad spectrum of bacterial coverage and delivery in high concentrations directly to the wound bed. Wound debridement is an important adjunct, as it directly reduces the bacterial burden, including biofilms. Biofilms are bacterial colonies surrounded by a protective coat of polysaccharides, more easily resistant to the action of antimicrobials. The periodic release of motile bacteria from these colonies may result in infection. Systemic antibiotics are only indicated in the presence of invasive wound infection or sepsis. Host systemic factors that cause immunosuppression should also be addressed (e.g. in controlling diabetes mellitus). Other factors that have clinical implication on wound bed preparation include bone exposure complicated with underlying osteomyelitis. This may require extended systemic antibiotic and surgical management such as debridement, and adequate soft tissue coverage (Halim AS et al. Wound bed preparation from a clinical perspective. Indian J Plast Surg. 2012 May-Aug; 45(2): 193-202).

### Achieving Moisture Balance

A moist wound environment accelerates wound re-epithelization without increasing the infection rate. These principles are fundamental parts of moisture balance in wound bed preparation (Falanga V. Wound Bed Preparation in Practice. EWMA Position Document. London: Medical Education Partnership Ltd; 2004. Wound bed preparation: Science applied to practice; pp. 2-5). Achieving moisture balance involves the creation and maintenance of a warm, moist wound bed and the stimulation of components in the moisture that have a positive impact on wound healing, such as growth factors. An appropriate amount of moisture is needed for the optimal effects of growth factors and cytokines and for the growth of proliferating cells, such as keratinocytes, endothelial cells and fibroblasts. On the one hand, excessive wound fluids contain matrix metalloproteinases and serine proteases that can break down or damage essential extracellular matrix materials. Excessive moisture also may lead to the maceration of the wound edges. On the other hand, inadequate moisture may inhibit cellular activities and promote eschar formation (Sibbald RG, Goodman L, Woo KY, Krasner DL, Smart H, Tariq G, et al. Special considerations in wound bed preparation 2011: An update(c) Adv Skin Wound Care. 2011;24:415-36). Thus, moisture balance is a delicate process of maintaining a moist healing environment that is optimal for healing. This requirement has led to the development of a vast array of moisture-retentive dressings that promote moist wound healing, ranging from occlusive, semiocclusive, absorptive and hydrating dressings.

Exudates can be managed directly via the use of a number of dressing materials, depending on the moisture status of the wound bed. For example, in a highly exudative wound, an absorptive dressing such as foam is appropriate, whereas in a dry wound eschar, an occlusive or semi-occlusive dressing such as a hydrocolloid is suitable to achieve the appropriate moisture balance (Halim AS et al. Wound bed preparation from a clinical perspective. Indian J Plast Surg. 2012 May-Aug; 45(2): 193-202). The use of compression and limb elevation to eliminate fluid from the wound site should be applied in venous ulcers or in wounds with surrounding edema. Moisture balance can be achieved indirectly, via systemic therapy that reduces edema (such as in heart failure), or the use of medication to reduce the inflammatory response in certain diseases (Attinger CE, Janis JE, Steinberg J, Schwartz J, Al-Attar A, Couch K. Clinical approach to wounds: Debridement and wound bed preparation including the use of dressings and wound-healing adjuvants. Plast Reconstr Surg. 2006;117:72 S-109).

Biological dressings (e.g., skin allograft, autologous skin graft) can also help manage chronic wounds. These dressings form a mechanical barrier against fluid, protein and electrolyte losses. Thus, these dressings prevent tissue desiccation and microbial invasion. A skin allograft can be used as a test prior to autologous skin grafting (Mat Saad AZ, Khoo TL, Dorai AA, Halim AS. The versatility of a glycerol-preserved skin allograft as an adjunctive treatment to free flap reconstruction. Indian J Plast Surg. 2009;42:94-9; Mat Saad AZ, Halim AS, Khoo TL. The use of glycerol-preserved skin allograft in conjunction with reconstructive and flap surgery: Seven years of experience. J Reconstr Microsurg. 2011;27:103-8).

Negative pressure wound therapy (e.g., foam dressing) can be used to manage a heavily exudating wound. Negative pressure wound therapy can also reduce edema, contributing to improved tissue perfusion. It also plays an important role in wound bed preparation by reducing the size and complexity of the wounds. The wound immediately contracts (macro-deformation) once the negative pressure is applied. This type of therapy has micro-deformation effects due to the stretching of small tissue blebs into the foam dressing. These mechanical effects change the cytoskeleton, resulting in cascades of biologic effects, including the stimulation of angiogenesis, reduction of bacterial loads and formation of granulation tissue (Borgquist O, Gustafsson L, Ingemansson R, Malmsjo M. Micro- and macromechanical effects on the wound bed of negative pressure wound therapy using gauze and foam. Ann Plast Surg. 2010;64:789-93). This therapy also can be used as a tool for mechanical and autolytic wound debridement, as the necrotic tissue adhering to the foam dressing is removed during the dressing changes and the moist wound bed enables the autolytic action of endogenous proteinases present within the moist environment.

### Epithelial Advancement

The progression of the wound edge in terms of epidermal cell/keratinocyte migration and wound contraction is one of the key indicators of a healing wound. If an arrest of these processes is observed, clinicians should consider the earlier elements discussed (T.I.M.E.), including cellular dysfunction and biochemical imbalances, as possible reasons for the failure of wound healing. For instance, keratinocytes are unable to proliferate and migrate over necrotic tissue, biofilm, hypergranulation tissue, fibrinous slough, presence of callosity or hyperkeratotic edges. These adverse environments should be removed by proper debridement. Control of infection as well as excessive inflammation must be achieved to reduce the level of proteases to a normal level, therefore maintaining the delicate biochemical balance required for epithelial cell replication. An optimal moisture level of the wound will promote epithelialization as evidence by advancing wound edge. Microscopically, cellular senescence may present at the edge of chronic wounds that need intervention to achieve healing (Halim AS et al. Wound bed preparation from a clinical perspective. Indian J Plast Surg. 2012 May-Aug; 45(2): 193-202).

### Correction of Cellular Dysfunction and Restoration of Biochemical Balance

Other components of wound bed preparation, especially when dealing with chronic, non-healing wounds, are the correction of cellular dysfunction and the restoration of biochemical balance. Chronic wounds are believed to be stuck in a prolonged inflammatory phase, which results from a variety of factors, including cellular dysfunction and dysregulation caused by the abnormal expression of extracellular matrix molecules and growth factors. In chronic wounds, the healing process has failed to proceed in an orderly and timely manner to produce anatomic and functional integrity, and these chronic wounds are characterized by a defective remodelling of the extracellular matrix, prolonged inflammation and failure to re-epithelialize (Mustoe TA, O'Shaughnessy K, Kloeters O. Chronic wound pathogenesis and current treatment strategies: A unifying hypothesis. J Plast Reconstr Surg. 2006;117:35-41; Hasan A, Murata H, Falabella A, Ochoa S, Zhou L, Badiava E, et al. Dermal fibroblasts from venous ulcers are unresponsive to action of transforming growth factor-beta I. J Dermatol Sci. 1997;16:59-66; Agren MS, Steenfos HH, Dabelsteen S, Hansen JB, Dabelsteen E. Proliferation and mitogenic response to PDGF-BB of fibroblasts isolated from chronic leg ulcers is ulcer-dependent. J Invest Dermatol. 1999;112:463-9; Cook H, Davies KJ, Harding KG, Thomas DW. Defective extracellular matrix reorganization by chronic wound fibroblasts is associated with alterations in TIMP-1, TIMP-2 and MMP-2 activity. J Invest Dermatol. 2000;115:225-33). In contrast, the normal wound healing process involves complex interactions between cellular components, extracellular matrix molecules and biochemical mediators/enzymes, such as growth factors, cytokines and proteases. These orderly cellular and biochemical processes are lost in the chronic non-healing wound. These processes are closely regulated, and disorder in one will affect another. The expression of extracellular matrix molecules, such as fibronectin and thrombospondin, follows a defined temporal course. However, they appear to be over-expressed in the chronic wound, which is believed to result from cellular dysfunction and dysregulation (Falanga V, Grinnell F, Gilchrist B, Maddox YT, Moshell A. Workshop on the pathogenesis of chronic wounds. J Invest Dermatol. 1994;102:125-7).

The biochemical content of chronic wound exudates is different from exudates of an acute wound. The balance of inflammatory cytokines, growth factors, enzymes and their inhibitors is altered, slowing down or even completely blocking the proliferation of cells, such as endothelial cells, fibroblasts and keratinocytes. These effects can prevent the progression of wound healing (Schultz GS, Sibbald RG, Falanga V, Ayello EA, Dowsett C, Harding K, et al. Wound bed preparation: A systematic approach to wound management. Wound Repair Regen. 2003; 11(Suppl 1): S 1-28).

Under normal circumstances, the levels of two pro-inflammatory cytokines, tumor necrosis factor-a (TNF-α) and interleukin-1 (IL-1), peak after a few days and return to very low levels in the absence of infection. However, these cytokines are persistently elevated in non-healing wounds. When the chronic wound shows signs of healing, the concentrations of inflammatory cytokines decrease to values approaching those in acute healing wounds, indicating a close correlation between low levels of inflammatory cytokines and the progression of wound healing (Schultz GS, Sibbald RG, Falanga V, Ayello EA, Dowsett C, Harding K, et al. Wound bed preparation: A systematic approach to wound management. Wound Repair Regen. 2003; 11(Suppl 1): S 1-28).

Fibroblasts are believed to play a pivotal role in orchestrating cellular and biochemical interactions in normal wound healing. Fibroblasts synthesize collagen fibers to provide strength to the healing wound and more importantly produce fibronectin, thrombospondin, integrins and vitronectin to form a basal lamina. These molecules are also components of the extracellular matrix, which provides a scaffold and template for endothelial cell and keratinocyte migration, for angiogenesis and for epithelialization. Fibroblasts also play a role in modulating the levels of extracellular matrix molecules by mediating the expression of cytokines and proteases, such as matrix metalloproteinases and elastase. In the chronic wound, fibronectin levels are low due to rapid degradation by proteases whose levels are higher than in acute wounds and due to the absence or low level of tissue inhibitor of metalloproteinases (TIMPs) (Halim AS et al. Wound bed preparation from a clinical perspective. Indian J Plast Surg. 2012 May-Aug; 45(2): 193-202).

Fibroblasts isolated from chronic wounds also display cellular dysfunctions, such as the inability to respond to growth factors (e.g., platelet-derived growth factor (PDGF)-β and transforming growth factor (TGF)-β). Several studies have demonstrated that fibroblasts derived from a variety of wound beds in chronic wounds represent a premature, senescent or undifferentiated phenotype that responds inefficiently to normal stimulatory messages (Mustoe TA, O'Shaughnessy K, Kloeters O. Chronic wound pathogenesis and current treatment strategies: A unifying hypothesis. J Plast Reconstr Surg. 2006;117:35-41; Stanley AC, Park HY, Phillips TJ, Russakovsky V, Menzoian JO. Reduced growth of dermal fibroblasts from chronic venous ulcers can be stimulated with growth factors. J Vasc Surg. 1997;26:994-9; Henderson EA. The potential effect of fibroblast senescence on wound healing and the chronic wound environment. J Wound Care. 2006;15:315-8; Menke NB, Ward KR, Witten TM, Bonchev DG, Diegelmann RF. Impaired wound healing. Clin Dermatol. 2007;25:19-25).

Chronic diabetic wounds increase the activity of fibroblasts, leukocytes and macrophages by creating a local deficiency of glucose and increase the amount of unwanted lactic acid byproducts, which are detrimental to the wound environment (Simonsen L, Holstein P, Larsen K, Bullow J. Glucose metabolism in chronic diabetic foot ulcers measured in vivo usingmicrodialysis. Clin Physiol. 1998;18:355-9; Stolle LB, Riegels-Nielsen P. The metabolism if the diabetic foot. In vivo investigation with microdialysis. Acta Orthop Scand. 2004;75:106-8). Macrophages isolated from the wounds of diabetic mice showed significant impairment in efferocytosis (meaning the process of removal or clearance of apoptotic cells by phagocytes). Impaired efferocytosis was associated with a significantly higher burden of apoptotic cells in the wound tissue as well as a higher expression of pro-inflammatory cytokines and a lower expression of anti-inflammatory cytokines (Khanna S, Biswas S, Shang Y, Collard E, Azad A, Kauh C, et al. Macrophage Dysfunction Impairs Resolution of Inflammation in the Wounds of Diabetic Mice. PLoS ONE. 2010;5:e9539).

Liu *et al.* have shown that there is impaired local angiogenesis in chronic diabetic foot ulcers due to the inadequate presence of endothelial progenitor cells. However, this deficiency is correctable by the use of hyperbaric oxygen therapy, which enhances the mobilization of circulating endothelial progenitor cells to the wound and synergistically works with the administration of exogenous stromal cell-derived factor (SDF)-1α (Liu ZJ, Velazquez OC. Hyperoxia, Endothelial progenitor cell mobilization, and diabetic wound healing. Antioxid Redox Signal. 2008;10:1869-82). The failure of insufficient angiogenesis affects cell migration and the support of the collagen synthesis necessary for the formation of mature granulation tissue and subsequent re-epithelialization (Brem H, Stojadinovic O, Diegelmann RF, Entero H, Lee B, Pastar I. Molecular markers in patients with chronic wounds to guide surgical debridement. Mol Med. 2007;13:30-9).

Similar to other parameters, to correct cellular and biochemical abnormalities, the causative factor(s) should be removed or treated. Such issues, for example, involve glycemic control in diabetes, pressure redistribution in a pressure ulcer, revascularization in arterial insufficiency and compressive treatment in venous ulceration (Sibbald RG, Goodman L, Woo KY, Krasner DL, Smart H, Tariq G, et al. Special considerations in wound bed preparation 2011: An update(c) Adv Skin Wound Care. 2011;24:415-36).

One of the classical ways to manage the chronic wound is by converting the wound to an acute wound by removing the wound edges via surgery. Multiple studies using biopsies and biochemical analyses have found that the cellular dysfunction and chemical abnormalities are found in this region of the wound (Mustoe TA, O'Shaughnessy K, Kloeters O. Chronic wound pathogenesis and current treatment strategies: A unifying hypothesis. J Plast Reconstr Surg. 2006;117:35-41; Stanley AC, Park HY, Phillips TJ, Russakovsky V, Menzoian JO. Reduced growth of dermal fibroblasts from chronic venous ulcers can be stimulated with growth factors. J Vasc Surg. 1997;26:994-9; Henderson EA. The potential effect of fibroblast senescence on wound healing and the chronic wound environment. J Wound Care. 2006;15:315-8; Menke NB, Ward KR, Witten TM, Bonchev DG, Diegelmann RF. Impaired wound healing. Clin Dermatol. 2007;25:19-25; Jude EB, Blakytny R, Bulmer J, Boulton AJ, Ferguson MW. Transforming growth factor-beta 1, 2, 3 and receptor type I and II in diabetic foot ulcers. Diabet Med. 2002;19:440-7; Blakytny R, Jude EB, Martin Gibson J, Boulton AJ, Ferguson MW. Lack of insulin-like growth factor-1 (IGF-1) in the basal keratinocyte layer of diabetic skin and diabetic foot ulcers. J Pathol. 2000;190:589-94). The biochemical imbalance usually can be managed by controlling the exudates by means of different dressing materials (e.g., negative pressure wound therapy).

However, even with adequate wound bed preparation, there are wounds that remain refractory to treatment or slow to progress. This may be the consequence of persistent cellular and biochemical derangement from the inappropriate activities of proteolytic enzymes, cytokines and growth factors produced within the wound, leading to prolonged inflammation, poor angiogenesis, extracellular matrix degradation and failure of cellular proliferation and migration (Herrick SE, Sloan P, McGurk M, Freak L, McCollum CN, Ferguson MW. Sequential changes in histologic pattern and extracellular matrix deposition during the healing of chronic venous ulcers. Am J Pathol. 1992;141:1085-95). Advanced therapies aiming to reverse these abnormalities include engineered skin constructs, platelet-derived growth factor, keratinocyte growth factor, granulocyte-macrophage colony stimulating factor, esterified hyaluronic acid, protease modulating matrix and immunoglobulin (Fivenson D, Scherschun L. Clinical and economic impact of Apligraf for the treatment of non-healing venous leg ulcers. Int J Dermatol. 2003;42:960-5; Da Costa RM, Ribeiro Jesus FM, Aniceto C, Mendes M. Randomised, double-blind, placebo-controlled, dose-ranging study of granulocytemacrophage colony stimulating factor in patients with chronic venous leg ulcers. Wound Repair Regen. 1999;7:17-25; Robson MC, Phillips TJ, Falanga V, Odenheimer DJ, Parish LC, Jensen JL, et al. Randomised trial of topically applied Repifermin (recombinant human keratinocyte growth factor-2) to accelerate wound healing in venous ulcers. Wound Repair Regen. 2001;9:347-52; Colletta V, Dioguardi D, Di Lonardo A, Maggio G, Torasso F. A trial to assess the efficacy and tolerability of Hyalofill-F in non-healing venous leg ulcers. J Wound Care. 2003;12:357-60; Sibbald RG, Torrance G, Hux M, Attard C, Milkovich N. Cost-effectiveness of becaplermin for non-healing neuropathic diabetic foot ulcers. Ostomy Wound Manage. 2003;49:76-84; Vevas A, Sheehan P, Pham HT. A randomised controlled trial of Promogran (a collagen/oxidized regenerated cellulose dressing) vs standard treatment in the management of diabetic foot ulcer. Arch Surg. 2002;137:822-7; Felts AG, Grainger DW, Slunt JB. Locally delivered antibodies combined with systemic antibiotics confer synergistic protection against antibiotic-resistant burn wound infection. J Trauma. 2000;49:873-8). These treatments in general produce/replace growth factors, stimulate angiogenesis, modulate inflammatory cells, promote cell proliferation and control excessive protease activity. However, such treatments are expensive and unavailable to many clinicians and practitioners.

### 8. Treatment of Chronic Non-healing Wounds

### 8.1 Adjuvant Agents

A wide variety of commercially available adjuvants are marketed to facilitate the treatment of chronic wounds. Unfortunately, randomized controlled trials continue to lag behind promotion and application. Improved functional status, ankle/brachial index (ABI), and quality of life have been documented with the use of cilostazol when treating arterial ulcers (Hiatt WR. Pharmacologic therapy for peripheral arterial disease and claudication. J Vas Surg. 2002;36:1283-91). Pentoxifylline and the application of bilayered artificial skin dressings, both utilized in conjunction with elasteric multilayer high-compression bandaging for the treatment of venous ulcers, have been validated as has the application of platelet-derived growth factors for neuropathic ulcers and pressure ulcers therapy (Jull AB, Waters J, Arroll B. Pentoxifylline for treating venous leg ulcers. Cochrane Database Syst Rev. 2002;1:CD001733; Miell JM, Wieman J, Steed DL, Perry BH, Sampson AR, Schwab BH. Efficacy and safety of becaplermin (recombinant human platelet-derived, growth factor-BB) in patients with non-healing, lower extremity diabetic ulcers: a combined analysis of four randomized studies. Wound Rep Reg. 1999;7:335-46; Wound Healing Society. Guidelines for the best care of chronic wounds. Wound Repair Regen. 2006;14:647-710). Recent concerns regarding malignancy with the use of Regranex® (becaplermin) have raised specific concern regarding its use (Werdin F. Eplasty. 2009; 9: e19). Electrical stimulation, ultrasound, low energy laser, spinal cord stimulation, and the application of hyperbaric oxygen therapy are promising therapies with theoretical, rational, and preclinical studies suggesting their use (Wound Healing Society. Guidelines for the best care of chronic wounds. Wound Repair Regen. 2006;14:647-710). Quality randomized controlled trials concerning their efficacy in chronic wound care are at present lacking. There is some support for negative pressure wound therapy as an adjunct for healing challenging wounds (Wound Healing Society. Guidelines for the best care of chronic wounds. Wound Repair Regen. 2006;14:647-710). Statistically, laser therapy and phototherapy have not been shown to improve ulcer healing (Wound Healing Society. Guidelines for the best care of chronic wounds. Wound Repair Regen. 2006;14:647-710).

Honey (or concentrated sugar preparations) have been applied to wounds to help prevent infection and possibly speed healing. Honey is thought to work primarily through its high sugar content by directly killing microorganisms. However, studies have not shown a clear benefit to the use of honey or concentrated sugar preparations in the treatment of chronic, non-healing wounds. A study by Robson et al. found that antibacterial honey (i.e., Medihoney®) did not significantly improve wound healing in 105 patients suffering from leg ulcers (Robson V et al., J. Adv Nurs. 2009; 65: 565-575). Conversely, Jull et al. showed that topical honey improved healing time compared to saline gauze and silver sulfadiazine in 2 trials of 140 patients with skin ulcers or burns (Jull A B et al., Cochrane Database Syst Rev. 2013 Feb 28; 2: CD005083). In addition, Debure and colleagues reported a case of a 64-year-old male who was being treated with granulated sugar for an infected pneumonectomy cavity (Debure A et al., Lancet. 1987; 1(8540): 1034-1035). The patient developed severe hyponatremia and acute renal failure with an osmolar gap and elevated sucrose levels in the urine and blood (Debure A et al., Lancet. 1987; 1(8540): 1034-1035). Once the sugar was removed from the infected cavity, the patient resumed urine flow, and a diagnosis of sucroseinduced osmotic nephrosis was concluded (Debure A et al., Lancet. 1987; 1(8540): 1034-1035). US 2011/0171284 describes a medical dressing for use in the treatment of wounds and comprising granulated table sugar, a povidone-iodine solution, and a gelling agent. As a gelling agent, alginates as well as hydrogels are useful.

### 8.2 Emerging Treatment Technologies

Emerging technologies present novel approaches to future wound care. In the near future, gene therapy may allow genes or gene-derived messengers important in healing to be delivered directly into a wound at directed time points (Hirsch T, Spielmann M, Velander P, et al. Insulin-like growth factor-1 gene therapy and cell transplantation in diabetic wounds. J Gene Med. 2008;10(11): 1247-52). Skin and composite equivalents from embryonic stem cells and application of bone marrow-derived stem cells seem further possible options (Stoff A, Rivera AA, Sanjib Banerjee N, et al. Promotion of incisional wound repair by human mesenchymal stem cell transplantation. Exp Dermatol. 2008). These future developments will depend very much on public and professional support for further research.

Unfortunately, one of the major barriers to effective wound care continues to be the lack of interest, enthusiasm, and knowledge shown by many clinicians and general practitioners for this subject (Werdin F. Eplasty. 2009; 9: e19). Thus, the need exists for a cost-effective, readily available wound healing compound that is safe to use in all types of chronic, non-healing wounds.

The described invention provides pharmaceutical compositions comprising granulated sugar and hydrogel that are effective to treat chronic non-healing wounds and to promote and accelerate wound healing.

### SUMMARY OF THE INVENTION

The present invention and the scope thereof is defined by the appended claims. The more generic description of the invention is provided for illustrative purposes only. Embodiments not falling under these claims are for reference purposes only. Embodiments in the description relating to methods of treatment are not covered by the claims. According to one aspect, the described invention provides a pharmaceutical composition comprising in equal parts 1:1 v/v granulated sugar and a hydrogel biomaterial comprising a water-swollen polymeric material for use in a method of treating a chronic non-healing wound in a subject, wherein the pharmaceutical composition increases average percent change in wound closure compared to a control, wherein the control is the hydrogel biomaterial; wherein: (a) the hydrogel biomaterial comprises purified water, glycerol, hydroxyl ethyl cellulose, sodium lactate, and allantoin, wherein: (i) the purified water is about 70% of the hydrogel biomaterial; and (ii) the glycerol is about 30% of the hydrogel biomaterial; and (b) the pharmaceutical composition is administered: (i) as a topical formulation; or (ii) as a dressing, wherein a surface of the dressing is impregnated with the composition.

The pharmaceutical composition comprising granulated sugar and a hydrogel biomaterial is effective to kill microorganisms in the chronic non-healing wound; or to accelerate wound healing in the chronic non-healing wound; or to increase collagen synthesis and neovascularization in the chronic non-healing wound; or to remove: fibrin; slough; and biofilm in the chronic non-healing wound; or to increase granulation tissue in the chronic non-healing wound; or to accelerate epithelialization in the chronic non-healing wound; or to drain the chronic non-healing wound; or to draw inflammatory fluid away from the chronic non-healing wound; or to control wound bed infection in the chronic non-healing wound; or to achieve wound bed preparation in the chronic non-healing wound.

According to one embodiment, the average percent wound closure ranges from about 50% to about 100% as compared to the control. According to another embodiment, the average percent wound closure is at least 50% greater than the control. According to another embodiment, the average percent wound closure is at least 95% greater than the control. According to another embodiment, the average percent wound closure is at least 99% greater than the control.

According to the claimed invention, the control is the hydrogel biomaterial comprising the purified water, glycerol, hydroxyl ethyl cellulose, sodium lactate and allantoin. The purified water is about 70% of the hydrogel biomaterial and the glycerol is about 30% of the hydrogel biomaterial.

According to the claimed invention, the pharmaceutical composition comprises equal parts (1:1 v/v) of the granulated sugar and the hydrogel biomaterial. According to another embodiment, the pharmaceutical composition is administered topically. According to another embodiment, the pharmaceutical composition is coated on at least one surface of a dressing. According to another embodiment, the dressing comprises an occlusive dressing and a nonadherent pad (Telfa™).

According to one embodiment, the average percent wound closure is average percent change in wound area, average percent change in wound volume or a combination thereof. According to another embodiment, the average percent change in wound area, the average percent change in wound volume or the combination thereof is determined by a 3-dimensional camera system.

According to one embodiment, the pharmaceutical composition further comprises at least one additional therapeutic agent. According to another embodiment, the at least one additional therapeutic agent is selected from the group consisting of an anti-inflammatory agent, an analgesic agent, an anti-infective agent, a growth factor and a combination thereof. According to another embodiment, the anti-infective agent is an antibiotic agent. According to another embodiment, the growth factor is selected from the group consisting of platelet-derived growth factor (PDGF), fibroblast growth factor (FGF) and granulocyte macrophage colony stimulating factor (GM-CSF).

According to one embodiment, the pharmaceutical composition is administered in combination with an adjunct therapy. According to another embodiment, the adjunct therapy is selected from the group consisting of hyperbaric oxygen (HBO), gene therapy, stem cell therapy, bioengineered skin, a skin equivalent, a skin substitute, a skin graft and a combination thereof.

According to one embodiment, the chronic non-healing wound is selected from the group consisting of a diabetic wound, a venous wound, a surgical wound, a cancer wound, a pressure ulcer, an arterial ulcer and a combination thereof.

According to one embodiment, the pharmaceutical composition is effective to decrease: wound and tissue edema; necrotic tissue; and pain in the chronic non-healing wound.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
**FIGURE 1** shows a diagram depicting skin anatomy (taken from Stedman's Medical Dictionary, 27th Ed., Lippincott, Williams & Wilkins, Baltimore, MD (2000), at 1647).
**FIGURE 2** shows a diagram depicting the layers of the epidermis.

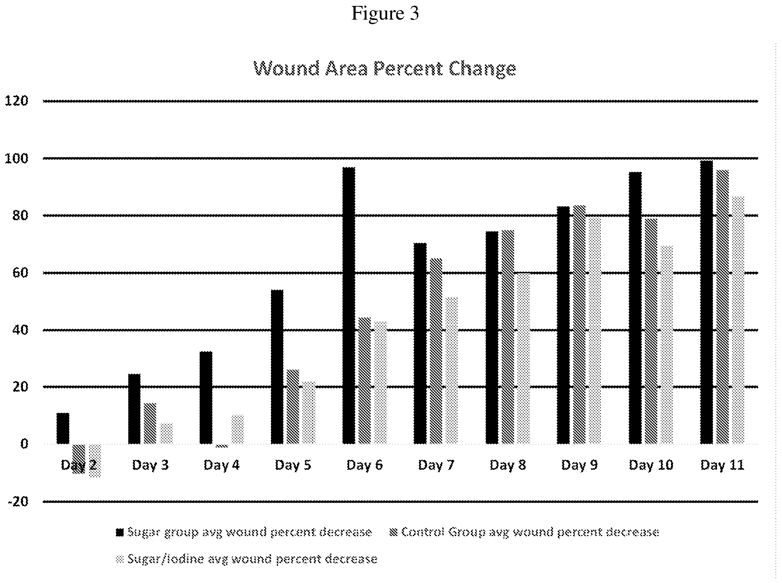

**FIGURE 3** shows a bar graph representing wound area percent change.

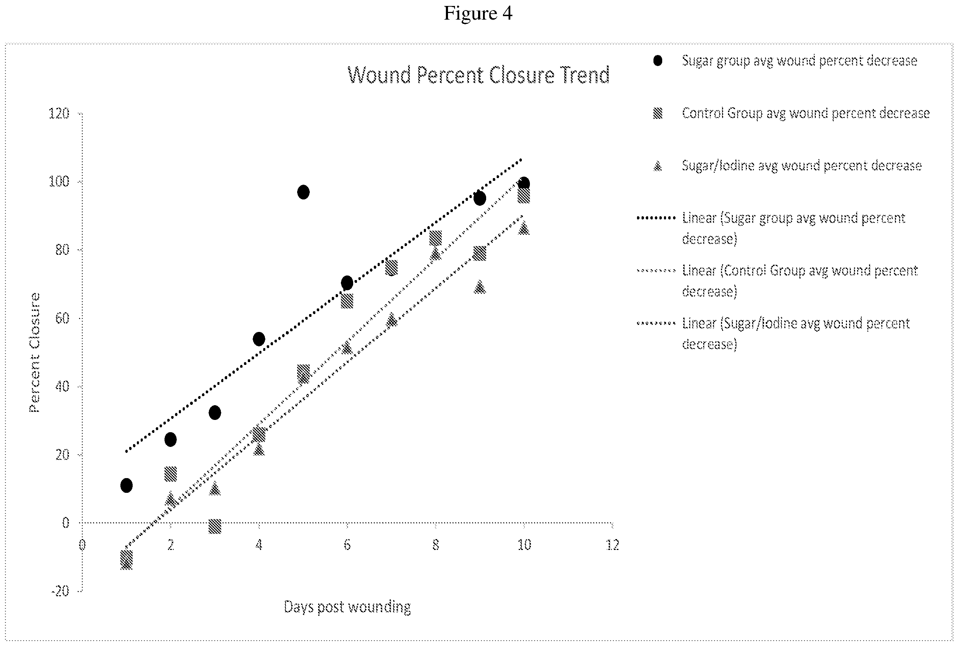

**FIGURE 4** shows a linear plot of wound percent closure trend.

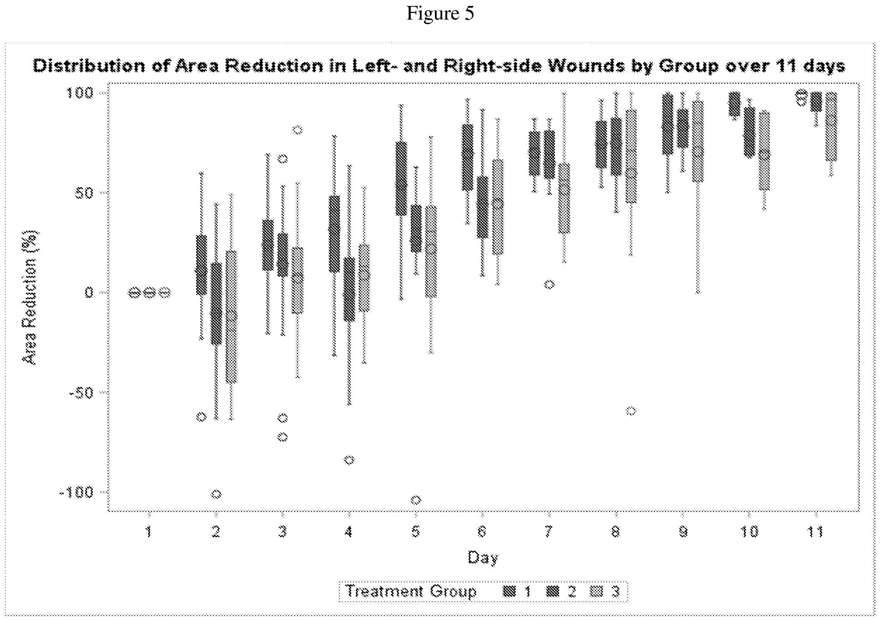

**FIGURE 5** shows comparison boxplots of area reduction of left- and right-side wounds from animals in Group 1 (granulated sugar + wound gel; n=12), Group 2 (wound gel only; n=12) and Group 3 (granulated sugar +wound gel + betadine; n=12) during an 11 day follow with 3 mice sacrificed in each group on days 3, 6, and 9 post-wounding before the end-of study evaluation on Day 11. By Day 2, wounds treated with granulated sugar +wound gel (Group 1) achieved faster area reduction than wounds in the other two groups. This trend persisted until Day 7, when wounds treated with wound gel only achieved area reduction that was not significantly different from Group 1 but significantly higher than those in Group 3 (Day 1: P=1.000; Day 2 P=0.0284; Day 3: P=0.0343; Day 4:P=0.0653; Day 5: P=0.0035; Day 6: P= 0.0147; Day 7: P= 0.0804; Day 8:P=0.9004; Day 9: P=0.7756; Day 10: P=0.0290; Day 11: P=0.1720).

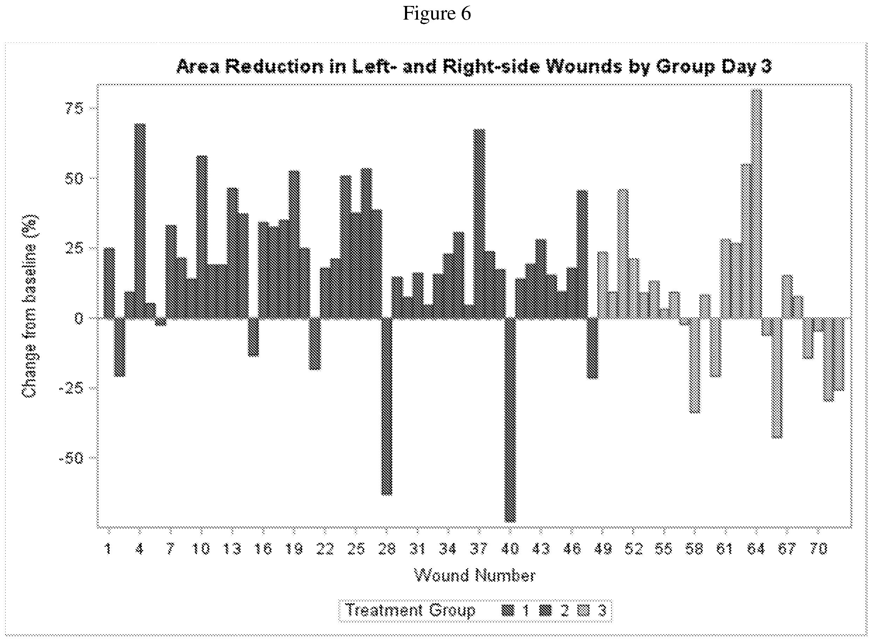

**FIGURE 6** shows a waterfall plot of area reduction in left- and right- side wounds on Day 3. Left- and right-side wounds 1 - 24 are from animals in Group 1 (granulated sugar + wound gel; n=12) on Day 3; Left-and right-side wounds 25 - 48 are from animals in Group 2 (wound gel only; n=12) on Day 3; left- and right-side wounds 49 - 72 are from animals in Group 3 (granulated sugar +wound gel + betadine; n=12) on Day 3. Reduction in area (change from baseline) in wounds in Group 1 was larger than that reported in wounds from Group 2. Sparse positive area reduction was observed in Group 3.

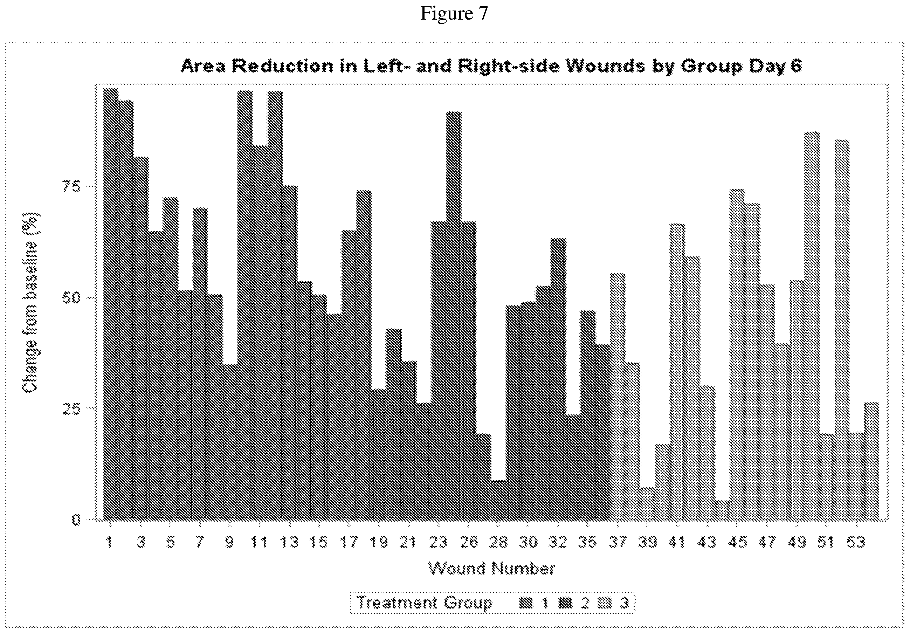

**FIGURE 7** shows a waterfall plot of area reduction in left- and right- side wounds on Day 6. Left- and right-side wounds 1 - 18 are from animals in Group 1 (granulated sugar + wound gel; n=9) on Day 6; Left-and right-side wounds 19 - 36 are from animals in Group 2 (wound gel only; n=9) on Day 6; left- and right-side wounds 37 - 54 are from animals in Group 3 (granulated sugar +wound gel + betadine; n=9) on Day 6. Reduction in area (change from baseline) in wounds in Group 1 was larger than that reported in wounds from Group 2. Sparse positive area reduction was observed in Group 3.

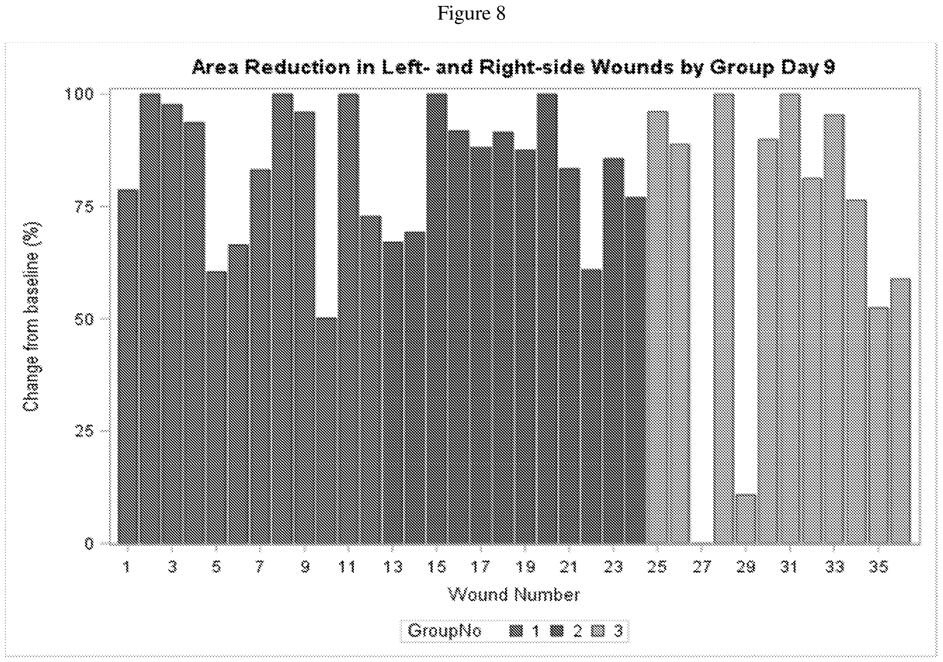

**FIGURE 8** shows a waterfall plot of area reduction in left- and right- side wounds on Day 9. Left- and right-side wounds 1 - 12 are from animals in Group 1 (granulated sugar + wound gel; n=6) on Day 9; Left-and right-side wounds 13 - 24 are from animals in Group 2 (wound gel only; n=6) on Day 9; left- and right-side wounds 25 - 36 are from animals in Group 3 (granulated sugar +wound gel + betadine; n=6) on Day 9. 6/12 wounds in Group 1 had achieved 90% area reduction by Day 9; 4/12 wounds in Group 2 had achieved at least 90% area reduction by Day 9 and 4/12 wounds in Group 3 had achieved 90% area reduction.

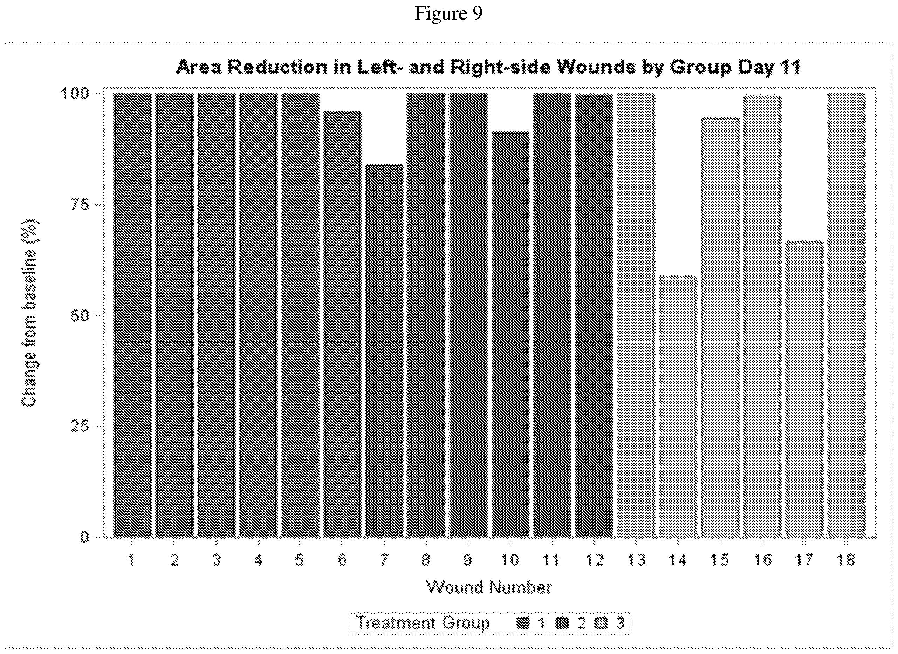

**FIGURE 9** shows a waterfall plot of area reduction in left- and right- side wounds on Day 11. Left- and right-side wounds 1 - 6 are from animals in Group 1 (granulated sugar + wound gel; n=3) on Day 11; Left-and right-side wounds 7 - 12 are from animals in Group 2 (wound gel only; n=3) on Day 11; left- and right-side wounds 13 - 18 are from animals in Group 3 (granulated sugar +wound gel + betadine; n=3) on Day 11. 5/6 wounds in Group 1 healed by Day 11, with 100% area reduction; 4/6 wounds in Group 2 achieved 100% area reduction by Day 11. 3/6 wounds in Group 3 achieved 100% area reduction by Day 11, with 2 of the wounds at less than 70% area reduction.

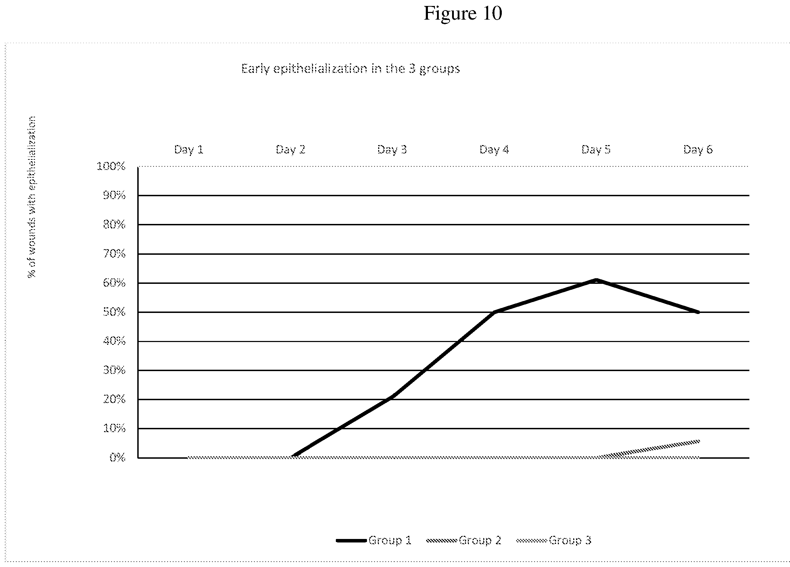

**FIGURE 10** shows a graph of percentage of wounds with epithelialization over time (Day 1 to Day 6) for animals in Group 1 (granulated sugar + wound gel), Group 2 (wound gel only) and Group 3 (granulated sugar +wound gel + betadine).

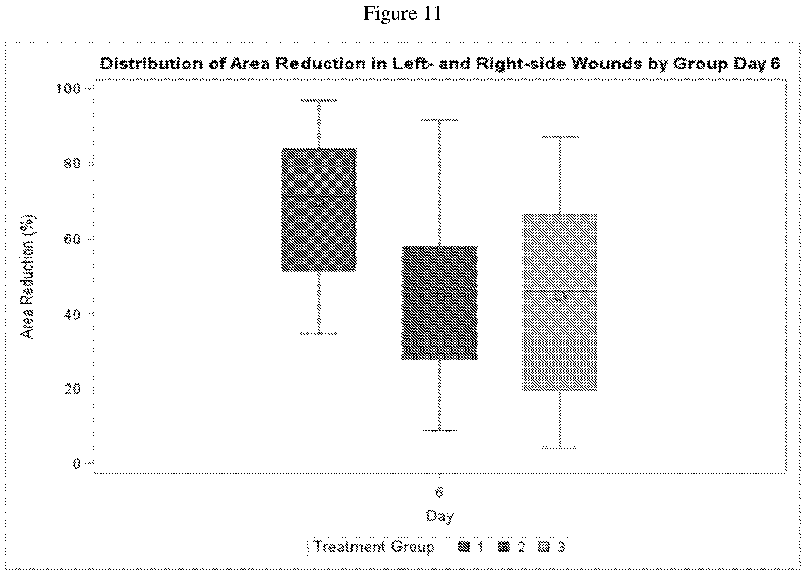

**FIGURE 11** shows boxplots of area reduction (%) in left- and right- side wounds from animals in Group 1 (granulated sugar + wound gel; n=9); Group 2 (wound gel only; n=9); and Group 3 (granulated sugar +wound gel + betadine; n=9) on Day 6. Area reduction was significantly different between the three groups (Wilcoxon P-value=0.0147).

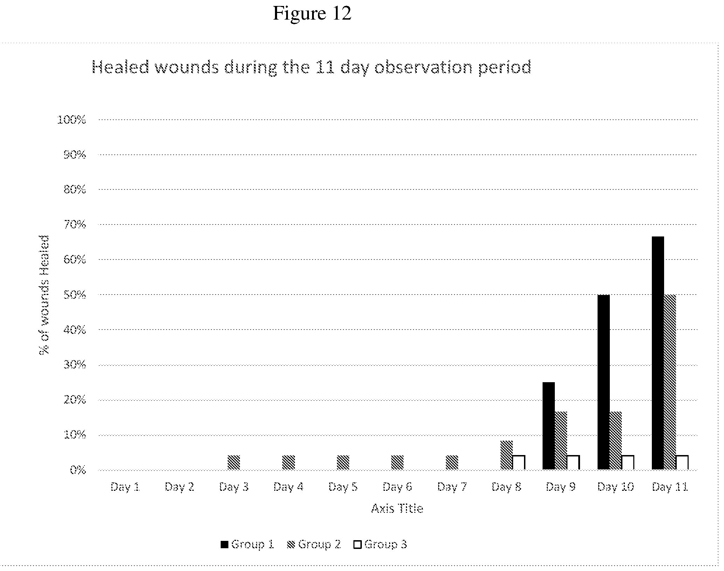

**FIGURE 12** shows a graph of the percentage of wounds healed during the 11 day observation period. The graph shows that the absolute number of healed wounds was higher in the sugar wound gel group (Group 1) compared to all other groups.

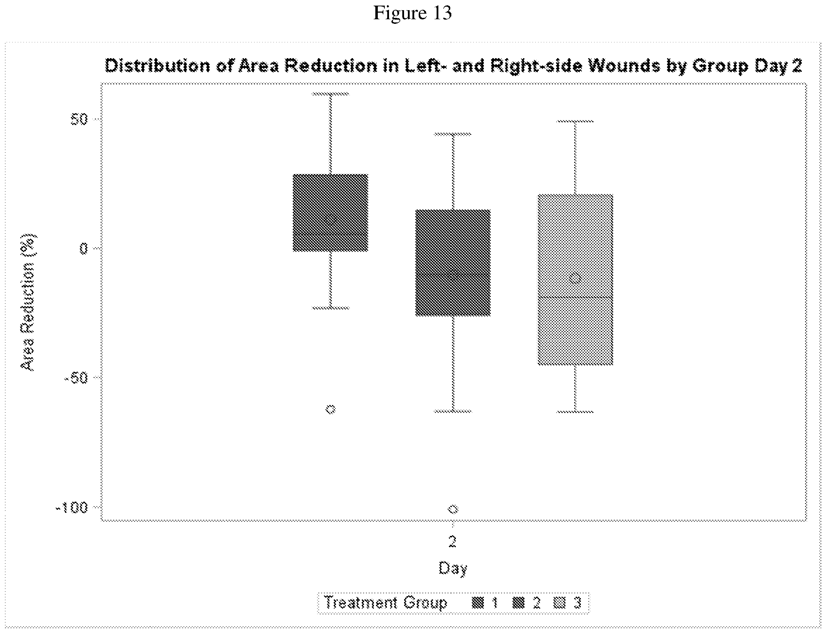

**FIGURE 13** shows boxplots of area reduction (%) in left- and right- side wounds from animals in Group 1 (granulated sugar + wound gel; n=9); Group 2 (wound gel only; n=9); and Group 3 (granulated sugar +wound gel + betadine; n=9) on Day 2.

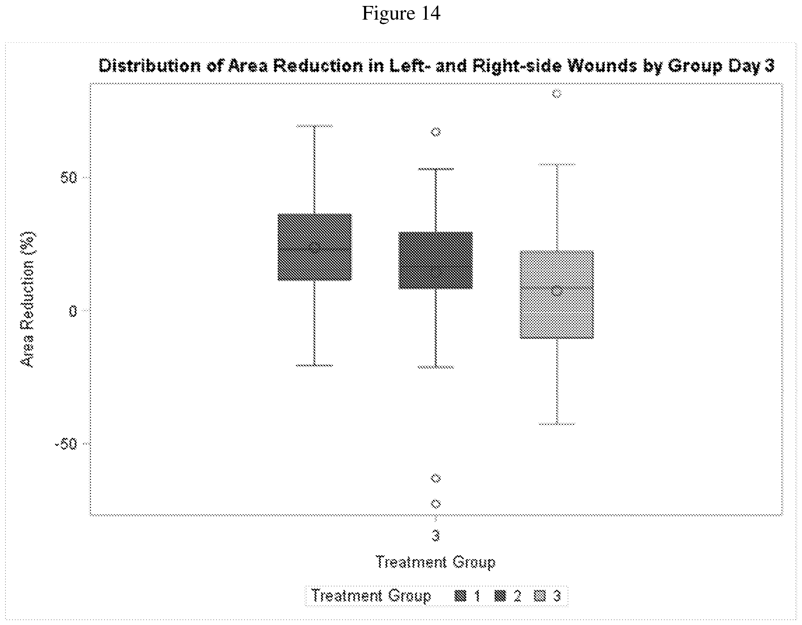

**FIGURE 14** shows boxplots of area reduction (%) in left- and right- side wounds from animals in Group 1 (granulated sugar + wound gel; n=9); Group 2 (wound gel only; n=9); and Group 3 (granulated sugar +wound gel + betadine; n=9) on Day 3.

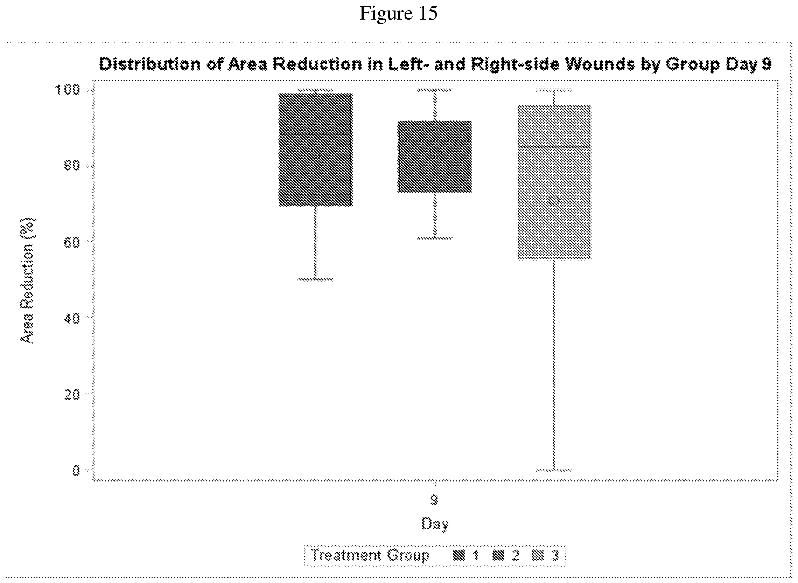

**FIGURE 15** shows boxplots of area reduction (%) in left- and right- side wounds from animals in Group 1 (granulated sugar + wound gel; n=9); Group 2 (wound gel only; n=9); and Group 3 (granulated sugar +wound gel + betadine; n=9) on Day 9.

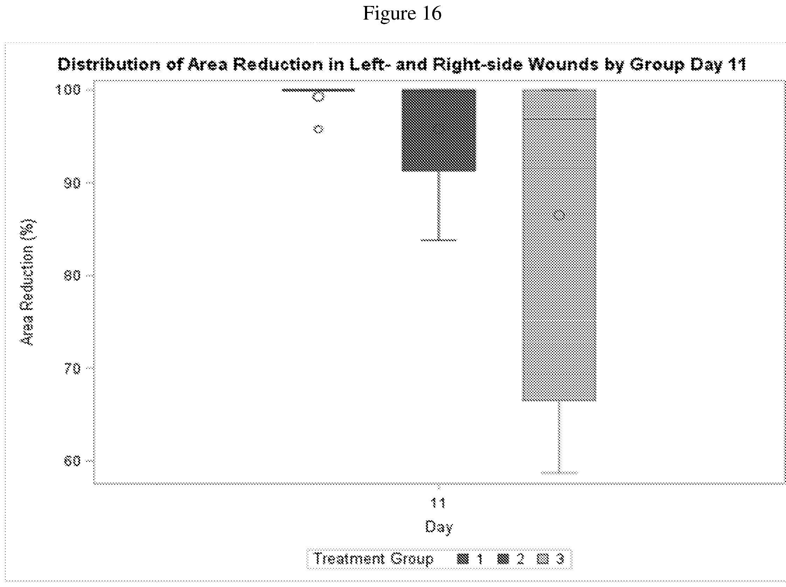

**FIGURE 16** shows boxplots of area reduction (%) in left- and right- side wounds from animals in Group 1 (granulated sugar + wound gel; n=9); Group 2 (wound gel only; n=9); and Group 3 (granulated sugar +wound gel + betadine; n=9) on Day 11.

### DETAILED DESCRIPTION OF THE INVENTION

### Glossary

The term "abrasion" as used herein refers to a scraping or rubbing away of a body surface by friction.

The term "administer" as used herein means to give or to apply. The term "administering" as used herein includes *in vivo* administration, as well as administration directly to tissue ex vivo. Generally, administration may be topically, in dosage unit formulations containing conventional nontoxic pharmaceutically acceptable carriers, adjuvants, and vehicles as desired, or may be local by means such as, but not limited to, injection, implantation, grafting, or parenterally.

The term "allantoin" as used herein refers to a chemical compound (5-ureidohydantoin), C₄H₆N₄O₃, that occurs as a white crystallizable substance found in many plants and in the allantoic and amniotic fluids and fetal urine of primates. It is also present in the urine of mammals other than primates as a product of purine metabolism and can be produced synthetically by the oxidation of uric acid.

As used herein, the term "antibody" includes, by way of example, both naturally occurring and non-naturally occurring antibodies. Specifically, the term "antibody" includes polyclonal antibodies and monoclonal antibodies, and fragments thereof. Furthermore, the term "antibody" includes chimeric antibodies and wholly synthetic antibodies, and fragments thereof.

Antibodies are serum proteins the molecules of which possess small areas of their surface that are complementary to small chemical groupings on their targets. These complementary regions (referred to as the antibody combining sites or antigen binding sites) of which there are at least two per antibody molecule, and in some types of antibody molecules ten, eight, or in some species as many as 12, may react with their corresponding complementary region on the antigen (the antigenic determinant or epitope) to link several molecules of multivalent antigen together to form a lattice.

The basic structural unit of a whole antibody molecule consists of four polypeptide chains, two identical light (L) chains (each containing about 220 amino acids) and two identical heavy (H) chains (each usually containing about 440 amino acids). The two heavy chains and two light chains are held together by a combination of noncovalent and covalent (disulfide) bonds. The molecule is composed of two identical halves, each with an identical antigen-binding site composed of the N-terminal region of a light chain and the N-terminal region of a heavy chain. Both light and heavy chains usually cooperate to form the antigen binding surface.

Human antibodies show two kinds of light chains, κ and λ; individual molecules of immunoglobulin generally are only one or the other. In normal serum, 60% of the molecules have been found to have κ determinants and 30 percent λ. Many other species have been found to show two kinds of light chains, but their proportions vary. For example, in the mouse and rat, λ chains comprise but a few percent of the total; in the dog and cat, κ chains are very low; the horse does not appear to have any κ chain; rabbits may have 5 to 40% λ, depending on strain and b-locus allotype; and chicken light chains are more homologous to λ than κ.

In mammals, there are five classes of antibodies, IgA, IgD, IgE, IgG, and IgM, each with its own class of heavy chain- α (for IgA), δ (for IgD), ε (for IgE), γ (for IgG) and µ (for IgM). In addition, there are four subclasses of IgG immunoglobulins (IgG1, IgG2, IgG3, IgG4) having γ1, γ2, γ3, and γ4 heavy chains respectively. In its secreted form, IgM is a pentamer composed of five four-chain units, giving it a total of 10 antigen binding sites. Each pentamer contains one copy of a J chain, which is covalently inserted between two adjacent tail regions.

All five immunoglobulin classes differ from other serum proteins in that they show a broad range of electrophoretic mobility and are not homogeneous. This heterogeneity - that individual IgG molecules, for example, differ from one another in net charge - is an intrinsic property of the immunoglobulins.

The principle of complementarity, which often is compared to the fitting of a key in a lock, involves relatively weak binding forces (hydrophobic and hydrogen bonds, van der Waals forces, and ionic interactions), which are able to act effectively only when the two reacting molecules can approach very closely to each other and indeed so closely that the projecting constituent atoms or groups of atoms of one molecule can fit into complementary depressions or recesses in the other. Antigen-antibody interactions show a high degree of specificity, which is manifest at many levels. Brought down to the molecular level, specificity means that the combining sites of antibodies to an antigen have a complementarity not at all similar to the antigenic determinants of an unrelated antigen. Whenever antigenic determinants of two different antigens have some structural similarity, some degree of fitting of one determinant into the combining site of some antibodies to the other may occur, and that this phenomenon gives rise to cross-reactions. Cross reactions are of major importance in understanding the complementarity or specificity of antigen-antibody reactions. Immunological specificity or complementarity makes possible the detection of small amounts of impurities/contaminations among antigens.

Monoclonal antibodies (mAbs) can be generated by fusing mouse spleen cells from an immunized donor with a mouse myeloma cell line to yield established mouse hybridoma clones that grow in selective media. A hybridoma cell is an immortalized hybrid cell resulting from the *in vitro* fusion of an antibody-secreting B cell with a myeloma cell. *In vitro* immunization, which refers to primary activation of antigen-specific B cells in culture, is another well-established means of producing mouse monoclonal antibodies.

Diverse libraries of immunoglobulin heavy (VH) and light (Vκ and Vλ) chain variable genes from peripheral blood lymphocytes also can be amplified by polymerase chain reaction (PCR) amplification. Genes encoding single polypeptide chains in which the heavy and light chain variable domains are linked by a polypeptide spacer (single chain Fv or scFv) can be made by randomly combining heavy and light chain V-genes using PCR. A combinatorial library then can be cloned for display on the surface of filamentous bacteriophage by fusion to a minor coat protein at the tip of the phage.

The technique of guided selection is based on human immunoglobulin V gene shuffling with rodent immunoglobulin V genes. The method entails (i) shuffling a repertoire of human λ light chains with the heavy chain variable region (VH) domain of a mouse monoclonal antibody reactive with an antigen of interest; (ii) selecting half-human Fabs on that antigen (iii) using the selected λ light chain genes as "docking domains" for a library of human heavy chains in a second shuffle to isolate clone Fab fragments having human light chain genes; (v) transfecting mouse myeloma cells by electroporation with mammalian cell expression vectors containing the genes; and (vi) expressing the V genes of the Fab reactive with the antigen as a complete IgG1, λ antibody molecule in the mouse myeloma.

The term "antibiotic agent" as used herein means any of a group of chemical substances having the capacity to inhibit the growth of, or to destroy bacteria, and other microorganisms, used chiefly in the treatment of infectious diseases. Examples of antibiotic agents include, but are not limited to, Penicillin G; Methicillin; Nafcillin; Oxacillin; Cloxacillin; Dicloxacillin; Ampicillin; Amoxicillin; Ticarcillin; Carbenicillin; Mezlocillin; Azlocillin; Piperacillin; Imipenem; Aztreonam; Cephalothin; Cefaclor; Cefoxitin; Cefuroxime; Cefonicid; Cefmetazole; Cefotetan; Cefprozil; Loracarbef; Cefetamet; Cefoperazone; Cefotaxime; Ceftizoxime; Ceftriaxone; Ceftazidime; Cefepime; Cefixime; Cefpodoxime; Cefsulodin; Fleroxacin; Nalidixic acid; Norfloxacin; Ciprofloxacin; Ofloxacin; Enoxacin ; Lomefloxacin; Cinoxacin; Doxycycline; Minocycline; Tetracycline; Amikacin; Gentamicin; Kanamycin; Netilmicin; Tobramycin; Streptomycin; Azithromycin; Clarithromycin; Erythromycin; Erythromycin estolate ; Erythromycin ethyl succinate; Erythromycin glucoheptonate; Erythromycin lactobionate; Erythromycin stearate; Vancomycin; Teicoplanin; Chloramphenicol; Clindamycin; Trimethoprim; Sulfamethoxazole; Nitrofurantoin; Rifampin; Mupirocin; Metronidazole; Cephalexin; Roxithromycin; Co-amoxiclavuanate; combinations of Piperacillin and Tazobactam; and their various salts, acids, bases, and other derivatives. Anti-bacterial antibiotic agents include, but are not limited to, penicillins, cephalosporins, carbacephems, cephamycins, carbapenems, monobactams, aminoglycosides, glycopeptides, quinolones, tetracyclines, macrolides, and fluoroquinolones.

The term "anti-fungal agent" as used herein means any of a group of chemical substances having the capacity to inhibit the growth of or to destroy fungi. Anti-fungal agents include but are not limited to Amphotericin B, Candicidin, Dermostatin, Filipin, Fungichromin, Hachimycin, Hamycin, Lucensomycin, Mepartricin, Natamycin, Nystatin, Pecilocin, Perimycin, Azaserine, Griseofulvin, Oligomycins, Neomycin, Pyrrolnitrin, Siccanin, Tubercidin, Viridin, Butenafine, Naftifine, Terbinafine, Bifonazole, Butoconazole, Chlordantoin, Chlormidazole, Cloconazole, Clotrimazole, Econazole, Enilconazole, Fenticonazole, Flutrimazole, Isoconazole, Ketoconazole, Lanoconazole, Miconazole, Omoconazole, Oxiconazole, Sertaconazole, Sulconazole, Tioconazole, Tolciclate, Tolindate, Tolnaftate, Fluconawle, Itraconazole, Saperconazole, Terconazole, Acrisorcin, Amorolfine, Biphenamine, Bromosalicylchloranilide, Buclosamide, Calcium Propionate, Chlorphenesin, Ciclopirox, Cloxyquin, Coparaffinate, Diamthazole, Exalamide, Flucytosine, Halethazole, Hexetidine, Loflucarban, Nifuratel, Potassium Iodide, Propionic Acid, Pyrithione, Salicylanilide, Sodium Propionate, Sulbentine, Tenonitrozole, Triacetin, Ujothion, Undecylenic Acid, and Zinc Propionate.

The term "anti-infective agent" as used herein refers to an agent that is capable of inhibiting the spread of an infectious agent such as an infectious microorganism, e.g., a bacteria, a virus, a nematode, a parasite, etc. Exemplary anti-infective agents may include antibiotic agent, antifungal agent, anti-viral agent, anti-protozoal agent, etc.

The term "anti-inflammatory agent" as used herein refers to an agent that reduces inflammation. The term "steroidal anti-inflammatory agent", as used herein, refer to any one of numerous compounds containing a 17-carbon 4-ring system and includes the sterols, various hormones (as anabolic steroids), and glycosides. The term "non-steroidal anti-inflammatory agents" refers to a large group of agents that are aspirin-like in their action, including ibuprofen (Advil)®, naproxen sodium (Aleve)®, and acetaminophen (Tylenol)®.

The term "biodegradable", as used herein, refers to material that will break down actively or passively over time by simple chemical processes, by action of body enzymes or by other similar biological activity mechanisms.

The term "biomimetic" as used herein refers to materials, substances, devices, processes, or systems that imitate or "mimic" natural materials made by living organisms.

The term "burn" as used herein refers to an injury to tissues caused by the contact with heat, flame, chemicals, electricity, or radiation. First degree burns show redness; second degree burns show vesication (a blistered spot); third degree burns show necrosis (cell death) through the entire skin. Burns of the first and second degree are partial-thickness burns, those of the third degree are full-thickness burns.

The term "carrier" as used herein describes a material that does not cause significant irritation to an organism and does not abrogate the biological activity and properties of the peptide of the composition of the described invention. Carriers must be of sufficiently high purity and of sufficiently low toxicity to render them suitable for administration to the mammal being treated. The carrier can be inert, or it can possess pharmaceutical benefits. The terms "excipient", "carrier", or "vehicle" are used interchangeably to refer to carrier materials suitable for formulation and administration of pharmaceutically acceptable compositions described herein. Carriers and vehicles useful herein include any such materials know in the art which are nontoxic and do not interact with other components.

The term "clotting agent" as used herein refers to an agent that promotes the clotting of blood. Exemplary clotting agents include but are not limited to thrombin, prothrombin, fibrinogen, etc.

The term "component" as used herein refers to a constituent part, element or ingredient.

The term "condition", as used herein, refers to a variety of health states and is meant to include disorders or diseases caused by any underlying mechanism or disorder, injury, and the promotion of healthy tissues and organs.

The term "contact" and all its grammatical forms as used herein refers to a state or condition of touching or of immediate or local proximity.

The term "cytokine" as used herein refers to small soluble protein substances secreted by cells which have a variety of effects on other cells. Cytokines mediate many important physiological functions including growth, development, wound healing, and the immune response. They act by binding to their cell-specific receptors located in the cell membrane, which allows a distinct signal transduction cascade to start in the cell, which eventually will lead to biochemical and phenotypic changes in target cells. Generally, cytokines act locally, but, to use hormone terminology, may have autocrine, paracrine or even endocrine effects. They include type I cytokines, which encompass many of the interleukins, as well as several hematopoietic growth factors; type II cytokines, including the interferons and interleukin-10; tumor necrosis factor ("TNF")-related molecules, including TNF-α and lymphotoxin; immunoglobulin super-family members, including interleukin 1 ("IL-1"); and the chemokines, a family of molecules that play a critical role in a wide variety of immune and inflammatory functions. The same cytokine can have different effects on a cell depending on the state of the cell; inflammatory cytokines may be produced by virtually all nucleated cells, for example, endo/epithelial cells and macrophages. Cytokines often regulate the expression of, and trigger cascades of, other cytokines.

The term "immunomodulatory cell(s)" as used herein refer(s) to cell(s) that are capable of augmenting or diminishing immune responses by expressing chemokines, cytokines and other mediators of immune responses.

The term "inflammatory cytokines" or "inflammatory mediators" as used herein refers to the molecular mediators of the inflammatory process, which may modulate being either pro- or anti-inflammatory in their effect. These soluble, diffusible molecules act both locally at the site of tissue damage and infection and at more distant sites. Some inflammatory mediators are activated by the inflammatory process, while others are synthesized and/or released from cellular sources in response to acute inflammation or by other soluble inflammatory mediators. Examples of inflammatory mediators of the inflammatory response include, but are not limited to, plasma proteases, complement, kinins, clotting and fibrinolytic proteins, lipid mediators, prostaglandins, leukotrienes, platelet-activating factor (PAF), peptides and amines, including, but not limited to, histamine, serotonin, and neuropeptides, pro-inflammatory cytokines, including, but not limited to, interleukin-1-beta (IL-1β), interleukin-4 (IL-4), interleukin-6 (IL-6), interleukin-8 (IL-8), tumor necrosis factor-alpha (TNF-a), interferon-gamma (IF-y), and interleukin-12 (IL-12).

Among the pro-inflammatory mediators, IL-1, IL-6, and TNF-α are known to activate hepatocytes in an acute phase response to synthesize acute-phase proteins that activate complement. Complement is a system of plasma proteins that interact with pathogens to mark them for destruction by phagocytes. Complement proteins can be activated directly by pathogens or indirectly by pathogen-bound antibody, leading to a cascade of reactions that occurs on the surface of pathogens and generates active components with various effector functions. IL-1, IL-6, and TNF-α also activate bone marrow endothelium to mobilize neutrophils, and function as endogenous pyrogens, raising body temperature, which helps eliminating infections from the body. A major effect of the cytokines is to act on the hypothalamus, altering the body's temperature regulation, and on muscle and fat cells, stimulating the catabolism of the muscle and fat cells to elevate body temperature. At elevated temperatures, bacterial and viral replications are decreased, while the adaptive immune system operates more efficiently.

The term "interleukin (IL)" as used herein refers to a cytokine secreted by, and acting on, leukocytes. Interleukins regulate cell growth, differentiation, and motility, and stimulates immune responses, such as inflammation. Examples of interleukins include interleukin-1 (IL-1), interleukin-1β (IL-1β), interleukin-6 (IL-6), interleukin-8 (IL-8), and interleukin-12 (IL-12).

The term "Tumor Necrosis Factor" or "TNF" as used herein refers to a cytokine made by white blood cells in response to an antigen or infection, which induce necrosis (death) of tumor cells and possesses a wide range of pro-inflammatory actions. Tumor necrosis factor also is a multifunctional cytokine with effects on lipid metabolism, coagulation, insulin resistance, and the function of endothelial cells lining blood vessels.

The term "disease" or "disorder", as used herein, refers to an impairment of health or a condition of abnormal functioning.

The terms "dose" and "dosage" are used interchangeably to refer to the quantity of a drug or other remedy to be taken or applied all at one time or in fractional amounts within a given period.

The term "drug" as used herein refers to a therapeutic agent or any substance, used in the prevention, diagnosis, alleviation, treatment, or cure of disease.

The term "effective amount" refers to the amount necessary or sufficient to realize a desired biologic effect.

The term "excisional wound" as used herein refers to a wound resulting from surgical removal or cutting away of tissue. The term "excisional wound" includes, but is not limited to, tears, abrasions, cuts, punctures, or lacerations in the epithelial layer of the skin that may extend into the dermal layer and even into subcutaneous fat and beyond.

The term "extracellular matrix" as used herein refers to a tissue derived or biosynthetic material that is capable of supporting the growth of a cell or culture of cells.

The term "extracellular matrix deposition" as used herein refers to the secretion of fibrous elements (e.g., collagen, elastin, and reticulin), link proteins (e.g., fibronectin, laminin), and space filling molecules (e.g., glycosaminoglycans) by cells.

The term "formulation" as used herein refers to a mixture prepared according to a formula, recipe or procedure.

The term "granulation" as used herein refers to a process whereby small red, grain-like prominences form on a raw surface in the process of healing.

The term "granulomatous inflammation" as used herein refers to an inflammation reaction characterized by a predominance of regular to epithelioid macrophages with or without multinucleated giant cells and connective tissue.

The term "hydrophilic" as used herein refers to a material or substance having an affinity for polar substances, such as water. The term "lipophilic" as used herein refers to preferring or possessing an affinity for a non-polar environment compared to a polar or aqueous environment.

The term "high tension wound" as used herein refers to a wound occurred in areas at or near a joint, including areas at or near elbow or knee. Other areas of the "high tension wound" include midsternal chest and post cesarean section wound.

The term "IC₅₀ value" as used herein refers to the concentration of an inhibitor that is needed to inhibit 50% of a given biological process or component of a process (i.e., an enzyme, cell, or cell receptor).

The term "in close proximity" as used herein refers to a distance very near.

The term "incisional wound" as used herein refers to a wound made by a clean cut, as with a sharp instrument.

The term "inflammation" as used herein refers to the physiologic process by which vascularized tissues respond to injury. See, e.g., FUNDAMENTAL IMMUNOLOGY, 4th Ed., William E. Paul, ed. Lippincott-Raven Publishers, Philadelphia (1999) at 1051-1053. During the inflammatory process, soluble inflammatory mediators of the inflammatory response work together with cellular components in a systemic fashion in the attempt to contain and eliminate the agents causing physical distress. The term "inflammatory mediators" as used herein refers to the molecular mediators of the inflammatory process. These soluble, diffusible molecules act both locally at the site of tissue damage and infection and at more distant sites. Some inflammatory mediators are activated by the inflammatory process, while others are synthesized and/or released from cellular sources in response to acute inflammation or by other soluble inflammatory mediators. Examples of inflammatory mediators of the inflammatory response include, but are not limited to, plasma proteases, complement, kinins, clotting and fibrinolytic proteins, lipid mediators, prostaglandins, leukotrienes, platelet-activating factor (PAF), peptides and amines, including, but not limited to, histamine, serotonin, and neuropeptides, proinflammatory cytokines, including, but not limited to, interleukin-1, interleukin-4, interleukin-6, interleukin-8, tumor necrosis factor (TNF), interferon-gamma, and interleukin 12.

The terms "inhibiting", "inhibit" or "inhibition" are used herein to refer to reducing the amount or rate of a process, to stopping the process entirely, or to decreasing, limiting, or blocking the action or function thereof. Inhibition may include a reduction or decrease of the amount, rate, action function, or process of a substance by at least 5%, at least 10%, at least 15%, at least 20%, at least 25%, at least 30%, at least 40%, at least 45%, at least 50%, at least 55%, at least 60%, at least 65%, at least 70%, at least 75%, at least 80%, at least 85%, at least 90%, at least 95%, at least 98%, or at least 99%. The terms "further inhibiting", "further inhibit" or "further inhibition are used herein to refer to reducing the amount or rate of a second process, to stopping the second process entirely, or to decreasing, limiting, or blocking the action or function thereof in addition to reducing the amount or rate of a first process, to stopping the first process entirely, or to decreasing, limiting, or blocking the action or function thereof. The term "inhibitory profile" as used herein refers to the characteristic pattern of reduction of the amount or rate or decrease, blocking or limiting of the action of more than one protein or enzyme. The terms "substantially inhibiting", "substantially inhibit", "substantially inhibited", or "substantially inhibition" are used here to refer to inhibition of kinase activity by at least 65%, by at least 70%, by at least 75%, by at least 80%, by at least 85%, by at least 90%, by at least 95%, by at least 98% or by at least 99%.

The term "injury," as used herein, refers to damage or harm to a structure or function of the body caused by an outside agent or force, which may be physical or chemical.

The term "laceration" as used herein refers to a torn and ragged wound or an accidental cut wound.

The term "manifestation" as used herein refers to the display or disclosure of characteristic signs or symptoms of an illness. Thus, the term "skin manifestation" as used herein refers to the display or disclosure of characteristic signs or symptoms of an illness on the skin.

The term "mechano-active dressing" as used herein refers to a medical or surgical covering for a wound that is configured to be removably secured to a skin surface near a wound in order to apply tension to the wound.

The term "modulate" as used herein means to regulate, alter, adapt, or adjust to a certain measure or proportion.

The term "neovascularization" as used herein refers to the new growth of blood vessels with the result that the oxygen and nutrient supply is improved. Similarly, the term "angiogenesis" refers to the vascularization process involving the development of new capillary blood vessels.

The term "pharmaceutically acceptable carrier" as used herein refers to one or more compatible solid or liquid filler, diluent or encapsulating substance which is/are suitable for administration to a human or other vertebrate animal. The components of the pharmaceutical compositions also are capable of being commingled in a manner such that there is no interaction which would substantially impair the desired pharmaceutical efficiency.

The term "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio. When used in medicine, the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts may be prepared as alkaline metal or alkaline earth salts, such as sodium, potassium or calcium salts of the carboxylic acid group. By "pharmaceutically acceptable salt" is meant those salts which are, within the scope of sound medical judgment, suitable for use in contact with the tissues of humans and lower animals without undue toxicity, irritation, allergic response and the like and are commensurate with a reasonable benefit/risk ratio. Pharmaceutically acceptable salts are well-known in the art. For example, P. H. Stahl, et al. describe pharmaceutically acceptable salts in detail in "Handbook of Pharmaceutical Salts: Properties, Selection, and Use" (Wiley VCH, Zurich, Switzerland: 2002). The salts may be prepared *in situ* during the final isolation and purification of the compounds described within the present invention or separately by reacting a free base function with a suitable organic acid. Representative acid addition salts include, but are not limited to, acetate, adipate, alginate, citrate, aspartate, benzoate, benzenesulfonate, bisulfate, butyrate, camphorate, camphorsufonate, digluconate, glycerophosphate, hemisulfate, heptanoate, hexanoate, fumarate, hydrochloride, hydrobromide, hydroiodide, 2-hydroxyethansulfonate(isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogencontaining groups may be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid. Basic addition salts may be prepared *in situ* during the final isolation and purification of compounds described within the invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like. Pharmaceutically acceptable salts also may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium or magnesium) salts of carboxylic acids may also be made.

The term "pharmaceutical composition" as used herein refers to a composition (meaning a product that contains all active and inactive ingredients) that is employed to prevent, reduce in intensity, cure or otherwise treat a target condition or disease.

The term "prevent" as used herein refers to the keeping, hindering or averting of an event, act or action from happening, occurring, or arising.

The term "puncture" as used herein refers to a wound in which the opening is relatively small as compared to the depth, as produced by a narrow pointed object.

The term "reduce" or "reducing" or "reduced" or "to reduce" as used herein refer to a diminution, a decrease, an attenuation or abatement of the degree, intensity, extent, size, amount, density or number. For example, the term "reduce" or "reducing" or "reduced" or "to reduce" as used herein refer to a diminution, a decrease, an attenuation or abatement of the degree, intensity, extent, size, amount, density or number of disorder in individuals at risk of developing the disorder.

The term "reepithelialization" as used herein refers to the reformation of epithelium over a denuded surface (e.g., wound).

The term "release" and its various grammatical forms, refers to dissolution of an active drug component and diffusion of the dissolved or solubilized species by a combination of the following processes: (1) hydration of a matrix, (2) diffusion of a solution into the matrix; (3) dissolution of the drug; and (4) diffusion of the dissolved drug out of the matrix.

The term "remodeling" as used herein refers to the replacement of and/or devascularization of granulation tissue.

The term "scaffold" as used herein refers to a substance or structure used to enhance or promote the growth of cells and/or the formation of tissue. A scaffold is typically a three dimensional porous structure that provides a template for cell growth.

The term "similar" is used interchangeably with the terms analogous, comparable, or resembling, meaning having traits or characteristics in common.

The terms "subject" or "individual" or "patient" are used interchangeably to refer to a member of an animal species of mammalian origin, including but not limited to, a mouse, a rat, a cat, a goat, sheep, horse, hamster, ferret, platypus, pig, a dog, a guinea pig, a rabbit and a primate, such as, for example, a monkey, ape, or human.

The phrase "subject in need of such treatment" is used to refer to a patient who has or will suffer a wound that can result in cutaneous scarring unless the context and usage of the phrase indicates otherwise.

The term "substantially pure", as used herein, refers to a condition of a therapeutic agent such that it has been substantially separated from the substances with which it may be associated in living systems or during synthesis. According to some embodiments, a substantially pure therapeutic agent is at least 70% pure, at least 75% pure, at least 80% pure, at least 85% pure, at least 90% pure, at least 95% pure, at least 96% pure, at least 97% pure, at least 98% pure, or at least 99% pure.

The term "susceptible" as used herein refers to a member of a population at risk.

The term "symptom" as used herein refers to a phenomenon that arises from and accompanies a particular disease or disorder and serves as an indication of it.

The term "syndrome," as used herein, refers to a pattern of symptoms indicative of some disease or condition.

The term "moiety" as used herein refers to a functional group of a molecule. The term "targeting moiety" as used herein refers to a functional group attached to a molecule that directs the molecule to a specific target, cell type or tissue.

The term "therapeutic agent" as used herein refers to a drug, molecule, nucleic acid, protein, composition or other substance that provides a therapeutic effect. The term "active" as used herein refers to the ingredient, component or constituent of the compositions of the present invention responsible for the intended therapeutic effect. The terms "therapeutic agent" and "active agent" are used interchangeably. The term "therapeutic component" as used herein refers to a therapeutically effective dosage (i.e., dose and frequency of administration) that eliminates, reduces, or prevents the progression of a particular disease manifestation in a percentage of a population. An example of a commonly used therapeutic component is the ED₅₀ which describes the dose in a particular dosage that is therapeutically effective for a particular disease manifestation in 50% of a population.

The terms "therapeutic amount", "therapeutic effective amount" or an "amount effective" of one or more active agents is an amount that is sufficient to provide the intended benefit of treatment. An effective amount of the active agents that can be employed ranges from generally 0.1 mg/kg body weight and about 50 mg/kg body weight. However, dosage levels are based on a variety of factors, including the type of injury, the age, weight, sex, medical condition of the patient, the severity of the condition, the route of administration, and the particular active agent employed. Thus the dosage regimen may vary widely, but can be determined routinely by a surgeon using standard methods.

The term "therapeutic effect" as used herein refers to a consequence of treatment, the results of which are judged to be desirable and beneficial. A therapeutic effect may include, directly or indirectly, the arrest, reduction, or elimination of a disease manifestation. A therapeutic effect also may include, directly or indirectly, the arrest reduction or elimination of the progression of a disease manifestation.

The term "topical" as used herein refers to administration of a composition at, or immediately beneath, the point of application. The phrase "topically applying" describes application onto one or more surfaces(s) including epithelial surfaces. Topical administration also may involve the use of transdermal administration such as transdermal patches or iontophoresis devices which are prepared according to techniques and procedures well known in the art. The terms "transdermal delivery system", transdermal patch" or "patch" refer to an adhesive system placed on the skin to deliver a time released dose of a drug(s) by passage from the dosage form through the skin to be available for distribution via the systemic circulation. Transdermal patches are a well-accepted technology used to deliver a wide variety of pharmaceuticals, including, but not limited to, scopolamine for motion sickness, nitroglycerin for treatment of angina pectoris, clonidine for hypertension, estradiol for post-menopausal indications, and nicotine for smoking cessation. Patches suitable for use in the described invention include, but are not limited to, (1) the matrix patch; (2) the reservoir patch; (3) the multi-laminate drug-in-adhesive patch; and (4) the monolithic drug-in-adhesive patch; TRANSDERMAL AND TOPICAL DRUG DELIVERY SYSTEMS, pp. 249-297 (Tapash K. Ghosh et al. eds., 1997). These patches are generally available commercially.

The term "traumatic wound" as used herein refers to a wound that is the result of an injury.

The term "treat" or "treating" includes abrogating, substantially inhibiting, slowing or reversing the progression of a disease, condition, disorder or injury, substantially ameliorating clinical or esthetical symptoms of a disease, condition, disorder or injury, substantially preventing the appearance of clinical or esthetical symptoms of a disease, condition, disorder or injury, and protecting from harmful or annoying symptoms. The term "treat" or "treating" as used herein further refers to accomplishing one or more of the following: (a) reducing the severity of the disease, condition, disorder or injury; (b) limiting development of symptoms characteristic of the disease, condition, disorder or injury being treated; (c) limiting worsening of symptoms characteristic of the disease, condition, disorder or injury being treated; (d) limiting recurrence of the disease, condition, disorder or injury in patients that have previously had the disease, condition, disorder or injury; and (e) limiting recurrence of symptoms in patients that were previously symptomatic for the disease, condition, disorder or injury.

The term "ulcer" as used herein refers to a lesion of the skin that is characterized by the formation of pus and necrosis (death of surrounding tissue) usually resulting from inflammation or ischemia.

The term "wound" as used herein refers to a disruption of the normal continuity of structures caused by a physical (e.g., mechanical) force, a biological or a chemical means. The term "wound" includes, but is not limited to, incisional wounds, excisional wounds, traumatic wounds, lacerations, punctures, cuts, and the like. The term "wound size" as used herein refers to a physical measure of disruption of the normal continuity of structures caused by a physical (e.g., mechanical) force, a biological or a chemical means.

The term "full-thickness wound" as used herein refers to destruction of tissue extending through the second layer of skin (dermis) to involve subcutaneous tissue under and possibly muscle or bone; the tissue can appear snowy white, gray, or brown, with a firm leathery texture.

The term "partial-thickness wound" as used herein refers to destruction of tissue through the first layer of skin (epidermis), extending into, but not through, the dermis.

The term "vitamin" as used herein, refers to any of various organic substances essential in minute quantities to the nutrition of most animals act especially as coenzymes and precursors of coenzymes in the regulation of metabolic processes. Non-limiting examples of vitamins usable in context of the present invention include vitamin A and its analogs and derivatives: retinol, retinal, retinyl palmitate, retinoic acid, tretinoin, iso-tretinoin (known collectively as retinoids), vitamin E (tocopherol and its derivatives), vitamin C (L-ascorbic acid and its esters and other derivatives), vitamin B₃ (niacinamide and its derivatives), alpha hydroxy acids (such as glycolic acid, lactic acid, tartaric acid, malic acid, citric acid, etc.) and beta hydroxy acids (such as salicylic acid and the like).

The term "wound closure" as used herein refers to the healing of a wound such that the edges of the wound are rejoined to form a continuous barrier.

The term "wound healing" as used herein refers to a regenerative process with the induction of a temporal and spatial healing program, including, but not limited to, the processes of inflammation, granulation, neovascularization, migration of fibroblast, endothelial and epithelial cells, extracellular matrix deposition, reepithelialization, and remodeling.

### Compositions

The described invention provides a pharmaceutical composition comprising equal parts (1:1 v/v) of granulated sugar and a hydrogel biomaterial, as defined in independent claim 1.

The term "hydrogel" refers to a water-swollen polymeric material resulting in a solid, semisolid, pseudoplastic or plastic structure containing a necessary aqueous component to produce a gelatinous or jelly-like mass. Hydrogels generally comprise a variety of polymers, including hydrophilic polymers, acrylic acid, acrylamide and 2-hydroxyethylmethacrylate (HEMA). Hydrogels provide moisture balance to a wound bed by balancing hydration with absorption of excessive fluid. Examples of hydrogels include, but are not limited to, synthetic, stimuli-sensitive, polypeptide-based, hybrid and DNA-based hydrogels.

Synthetic hydrogels may be produced, for example, by controlled radical polymerization, such as atom transfer radical polymerization (ATRP), reversible addition-fragmentation transfer (RAFT) polymerization, and nitroxide-mediated polymerization; and by chain crosslinking. Chain crosslinking employs sliding crosslinking agents. By chemically crosslinking two cyclodextrin molecules, each threaded on a different polyethylene glycol (PEG) chain (end-capped with a bulky group, such as adamantan), a sliding double ring crosslinking agent is produced. These agents are characterized by a high degree of swelling in water and a high stretching ratio without fracture.

Non-limiting examples of synthetic hydrogels include Double networks (DN) and nanocomposite (clay filled) hydrogels. Double networks (DN) are a subset of interpenetrating networks (IPNs) formed by two hydrophilic networks, one highly crosslinked, the other loosely crosslinked. For example, a DN composed of two mechanically weak hydrophilic networks, poly(2-acrylamido-2-methylpropanesulfonic acid) and polyacrylamide, provides a hydrogel with mechanical properties demonstrating both hardness and toughness.

Nanocomposite (clay filled) hydrogels are organic-inorganic hybrids based on *N*-isopropylacrylamide (NIPAAm) with hectorite, [Mg_{5.34}Li_{0.66}Si₈O₂₀(OH)₄]Na_{0.66}, as multifunctional crosslinker. Exfoliated clay platelets are uniformly dispersed in an aqueous medium containing the NIPAAm monomer; polyNIPAAm chains are grafted on the clay surface by one or two ends.

Stimuli-sensitive hydrogels undergo continuous or discontinuous changes in swelling that are mediated by external stimuli such as changes in pH, temperature, ionic strength, solvent type, electric and magnetic fields, light, and the presence of chelating species. The majority of stimuli responsive hydrogels are created using conventional (traditional) methods of synthesis of a relatively small number of synthetic polymers, especially (meth)acrylate derivatives and their copolymers. Stimuli-sensitive hydrogels may also be polypeptide based. These include, but are not limited to, hydrogels formed from block copolypeptides recombinant segments of natural structural proteins like elastin and silk, tandemly repeated silk-like and elastin-like peptide blocks, and recombinant triblock copolymers of a random polypeptide sequence flanked by two coiled-coil blocks. Peptide and/or protein segments have been used to introduce degradability, temperature-induced phase transition, and sensitivity to the presence of biologically active molecules into the hydrogel structure. Water-soluble synthetic polymers have been crosslinked with biological molecules, such as oligopeptides, oligodeoxyribonucleotides, stereospecific D,L-lactic acid oligomers, through antigen-antibody binding, or by intact native proteins.

Hydrogels may self-assemble from block and graft copolymers driven by hydrophobic interactions, e.g. of A blocks in ABA triblock copolymers containing a hydrophilic B block capped with (short) hydrophobic A blocks. Designing hydrogel-forming copolymers, using recognition motifs found in nature, such as coiled-coils, enhances the potential for the formation of precisely defined three-dimensional structures. The coiled-coil, a supercoil formed by two or more strands of α-helices, is an example of a hydrogel self-assembled from block and graft copolymers. The primary sequence of a typical coiled-coil is composed of 7-residue repeats, designated as heptads. The amino acid residues in a heptad are conventionally denoted as "a, b, c, d, e, f, g". Hydrophobic residues at positions "a" and "d" form an inter-helical hydrophobic core, providing a stabilizing interface between the helices. Charged residues at positions "e" and "g" form electrostatic interactions, which contribute to coiled-coil stability and mediate specific association among helices.

ABA block copolymers, where the block A is a coiled-coil forming peptide and block B a random coil, also self-assemble into hydrogels. The self-assembly occurs as a balance between the oligomerization of the helical ends and swelling of the central water-soluble polyelectrolyte segment. Temperature and/or pH-responsiveness may be achieved by manipulating the amino acid sequence of the coiled-coil domains. Minor modifications in their structure have a strong impact on the stimuli-sensitivity of self-assembled hydrogels. For example, the *thermal stability of the coiled-coil* containing proteins can be manipulated in a predictable way by substituting amino acids in the coiled-coil domain.

Hybrid hydrogels are hydrogel systems that possess components from at least two distinct classes of molecules, for example, synthetic polymers and biological macromolecules, interconnected either covalently or non-covalently. Compared to synthetic polymers, proteins and protein modules have well-defined and homogeneous structures, consistent mechanical properties, and cooperative folding/unfolding transitions. The peptide domain may impose a level of control over the structure formation at the nanometer level; the synthetic part may contribute to the biocompatibility of the hybrid material.

Hybrid hydrogel synthesis may entail, for example, the non-covalent attachment of genetically engineered coiled-coil protein motifs to a hydrophilic synthetic N-(2-hydroxypropyl) methacrylamide (HPMA) copolymer backbone. The physical crosslinking is established by self-assembly of the coiled-coil domains. A temperature-induced hydrogel collapse corresponds to the structural transition of the coiled-coil domains from an elongated helix to an unfolded state. Hydrogels formed by crosslinking of HPMA copolymer precursors with coiled-coil modules undergo dramatic volume transitions (de-swelling up to 10-fold) at the melting temperature of the coiled-coil modules.

Graft copolymers also self-assemble into hybrid hydrogels. This hybrid hydrogel system is based on HPMA copolymers consisting of a hydrophilic polymer backbone and a pair of oppositely charged peptide grafts. Two distinct pentaheptad peptides create a dimerization motif and serve as physical crosslinkers.

Self-assembly of hydrogels may also be mediated by DNA recognition. DNA sequences have been used as biorecognition motifs in the design of biomaterials. For example, biorecognition of complementary oligonucleotides grafted on a hydrophilic polymer backbone mediates self-assembly of graft copolymers into hydrogels. In another design, hydrogels may be synthesized by copolymerizing acrylamide with dimethacryloylated 5',3'-diaminoalkyl-modified (single strand) ssDNAs as crosslinking agents. DNA motifs may be used to assemble nanoparticles into macroscopic materials, and dendrimers into hydrogels. An enzyme-catalyzed process may be used to mediate hydrogel formation. For example, branched DNA molecules containing complementary sticky ends with palindromic sequences are hybridized, followed by ligation catalyzed with T4 DNA ligase. DNA also may impose changes in the supramolecular structure of hybrid copolymers. For example, a diblock copolymer, ssDNA-*b*-polypropyleneoxide, forms spherical micelles in aqueous solutions. Using molecular recognition mediated by long DNA templates, these micelles can be transformed into amphiphilic rods.

According to a reference embodiment, the hydrogel comprises purified water, glycerol, hydroxyl ethyl cellulose, sodium lactate and aloe vera gel. According to the claimed invention, the hydrogel comprises purified water, glycerol, hydroxyl ethyl cellulose, sodium lactate and allantoin. Uses of allantoin include, but are not limited to, a skin protectant, an anti-irritant, to increase water content of the extracellular matrix, to promote cell replication, to promote wound healing, to promote scar healing and to promote healing of burns. According to the claimed invention, the purified water is about 70% of the hydrogel, and the glycerol is about 30% of the hydrogel.

According to one embodiment, the pharmaceutical composition may be delivered topically. Topical forms of the pharmaceutical composition can include, but are not limited to, lotions, ointments, salves, gels, pastes powders and creams. The pharmaceutical composition may be applied by pouring, dropping, or spraying, if a liquid; rubbing on, if an ointment, lotion, cream, gel, or the like; dusting, if a powder; spraying, if a liquid or aerosol composition; or by any other appropriate means. Topical administration generally provides a local rather than a systemic effect.

According to another embodiment, the administering is by means of a dressing comprising the pharmaceutical composition. According to another embodiment, at least one surface of the dressing is impregnated with the pharmaceutical composition. According to another embodiment, at least one surface of the dressing is coated with the pharmaceutical composition. According to another embodiment, the dressing is selected from the group consisting of a gauze dressing, a tulle dressing, an alginate dressing, a polyurethane dressing, a silicone foam dressing, a synthetic polymer scaffold dressing, a non-adherent pad dressing and a combination thereof. According to another embodiment, the dressing is an occlusive dressing selected from the group consisting of a film dressing, a semi-permeable film dressing, a hydrogel dressing, a hydrocolloid dressing, and a combination thereof. According to another embodiment, the administering is by means of a dermal substitute, wherein the pharmaceutical composition is embedded in a dermal substitute that provides a three dimensional scaffold. According to another embodiment, the dermal substitute is made of a natural biological material, a constructive biological material, or a synthetic material. According to another embodiment, the natural biological material comprises human cadaver skin, porcine cadaver skin, or porcine small intestine submucosa. According to another embodiment, the natural biological material comprises a matrix. According to another embodiment, the natural biological material consists essentially of a matrix that is sufficiently devoid of cell remnants. According to another embodiment, the constructive biological material comprises collagen, glycosaminoglycan, fibronectin, hyaluronic acid, elastine, or a combination thereof. According to another embodiment, the constructive biological material is a bilayer, non-cellularized dermal regeneration template or a single layer, cellularized dermal regeneration template. According to another embodiment, the synthetic dermal substitute comprises a hydrogel. According to another embodiment, the synthetic dermal substitute further comprises an RGD peptide with amino acid sequence Arginine-Glycine-Aspartate.

Gauze dressings can stick to the wound surface and disrupt the wound bed when removed. As a result gauze dressings are used generally on minor wounds or as secondary dressings.

Tulle dressings do not stick to wound surfaces. They are suitable for use in flat, shallow wounds, and are useful in patients with sensitive skin. Examples of tulle dressings include, but are not limited to, Jelonet® and Paranet®.

Alginate dressings are composed of calcium alginate (a seaweed component). When in contact with a wound, calcium in the dressing is exchanged with sodium from wound fluid and this turns the dressing into a gel that maintains a moist wound environment. These dressings are good for exudating wounds and help in debridement of sloughing wounds. In general, alginate dressings are not used on low exudating wounds as this will cause dryness and scabbing. Alginate dressings are changed daily. Examples of alginate dressings include, but are not limited to, Kaltostat® and Sorbsan®.

Polyurethane or silicone foam dressings are designed to absorb large amounts of exudates. They maintain a moist wound environment but are not as useful as alginates or hydrocolloids for debridement. In general, they are not used on low exudating wounds as this will cause dryness and scabbing. Examples of polyurethane or silicone foam dressings include, but are not limited to, Allevyn® and Lyofoam®.

Collagen dressings are generally provided in the form of pads, gels or particles. They promote the deposit of newly formed collagen in the wound bed, and absorb exudate and provide a moist environment.

Non-adherent pad dressings are suitable for use in lightly exudating wounds, acute wounds and skin tears. They provide wound protection to any lightly draining wound without sticking to the wound surface. Examples of non-adherent pad dressings include, but are not limited to, Telfa™ Pads and Telfa™ Island Dressing.

Other suitable dressings include occlusive dressings. The term "occlusive dressing" as used herein refers to a dressing that prevents air or bacteria from reaching a wound or lesion and that retains moisture, heat, body fluids, and medication. Traditional dressings such as gauze and Telfa™ pads (non-adhesive pads) promote desiccation of the wound surface and adhere to it as well. When used to treat severe injuries (e.g., ulcers), removal of the dressing strips away newly formed epithelium, causing bleeding and prolongation of the healing process. Since the wound is dry and cracked, movement is often painful and inhibited. Studies have shown that occlusive dressings prevent desiccation and eschar formation by trapping moisture next to the wound bed. According to some such embodiments, the occlusive dressings are totally occlusive dressings. According to some other embodiments, the occlusive dressings are semi-permeable dressings. Examples of occlusive dressings for the purpose of the invention include, but are not limited to, film dressings (totally occlusive dressings), semi-permeable film dressings, hydrogel dressings, hydrocolloid dressings, or a combination thereof.

Film dressings and semi-permeable film dressings comprise sheets of materials that may be used to cover wounds. Such dressings may comprise sterile materials. Suitable materials, from which such films may be manufactured, include polyurethane and chitin. Film dressing (or semi-permeable film dressings) may be coated with adhesives, such as acrylic adhesives, in order to assist their retention at sites where they are required. Dressings of this type may be transparent, and therefore allow the progress of wound healing to be checked. These dressings are generally suitable for shallow wounds with low exudate. Examples of film or semi-permeable film dressings include, but are not limited to, OpSite® and Tegaderm®

Hydrogel dressings are composed mainly of water in a complex network of fibers that keep the polymer gel intact. Water is released to keep the wound moist. These dressings may be used for necrotic or sloughy wound beds to rehydrate and remove dead tissue. They are not used for moderate to heavily exudating wounds. Examples of hydrogel dressings include, but are not limited to, Tegagel®, Intrasite®.

Hydrocolloid dressings are composed of hydrophilic particles, such as gelatin and pectin, connected together with a hydrophobic adhesive matrix, and are covered by an outer film or foam layer. When hydrocolloids are applied to a wound, any exudate in the wound contact area is absorbed to form a swollen gel, which fills the wound and provides a controlled absorption gradient to the rest of the dressing. This creates a warm, moist environment that promotes debridement and healing. Depending on the hydrocolloid dressing chosen, they may be suitable for use in wounds with light to heavy exudate, sloughing or granulating wounds. Dressings of this sort are available in many forms (adhesive or non-adhesive pad, paste, powder) but most commonly as self-adhesive pads. Examples of hydrocolloid dressings include, but are not limited to, DuoDERM® and Tegasorb®.

A suitable wound healing dressing to be used on a particular wound may be selected with reference to the type of the wound, size of the wound, and healing progression of the wound.

According to some embodiments, the dressing comprises an occlusive and a non-adherent pad. Non-adherent pads include, but are not limited to, Telfa™ pads.

Topical administration also may involve the use of transdermal administration such as transdermal patches or iontophoresis devices which are prepared according to techniques and procedures well known in the art. The terms "transdermal delivery system", transdermal patch" or "patch" refer to an adhesive system placed on the skin to deliver a time released dose of a drug(s) by passage from the dosage form through the skin to be available for distribution via the systemic circulation. Transdermal patches are a well-accepted technology used to deliver a wide variety of pharmaceuticals, including, but not limited to, scopolamine for motion sickness, nitroglycerin for treatment of angina pectoris, clonidine for hypertension, estradiol for post-menopausal indications, and nicotine for smoking cessation. Patches suitable for use in the described invention include, but are not limited to, (1) the matrix patch; (2) the reservoir patch; (3) the multi-laminate drug-in-adhesive patch; and (4) the monolithic drug-in-adhesive patch; TRANSDERMAL AND TOPICAL DRUG DELIVERY SYSTEMS, pp. 249-297 (Tapash K. Ghosh et al. eds., 1997). These patches are well known in the art and generally available commercially.

According to one embodiment, the pharmaceutical composition of the described invention comprises a carrier. The carrier can include, but is not limited to, a release agent, such as a sustained release or delayed release carrier. According to such embodiments, the carrier can be any material capable of sustained or delayed release of the granulated sugar and hydrogel to provide a more efficient administration, e.g., resulting in less frequent and/or decreased dosage of the polypeptide, improving ease of handling, and extending or delaying effects on diseases, disorders, conditions, syndromes, and the like. Non-limiting examples of such carriers include liposomes, microsponges, microspheres, or microcapsules of natural and synthetic polymers and the like. Liposomes may be formed from a variety of phospholipids, including, but not limited to, cholesterol, stearylamines or phosphatidylcholines.

### Methods for Treating Chronic non-healing Wounds

According to some embodiments, the pharmaceutical composition is effective to treat chronic non-healing wounds. Non-limiting examples of chronic non-healing wounds include diabetic wounds, venous wounds, surgical wounds, cancer wounds, pressure ulcers, arterial ulcers and the like. Diabetic wounds include, but are not limited to, diabetic foot ulcers.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to decrease wound and tissue edema in the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to decrease necrotic tissue.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to kill microorganisms in the chronic non-healing wound. According to some embodiments, the pharmaceutical composition is effective to kill microorganisms without risk of bacterial resistance.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to accelerate wound healing in the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to increase collagen synthesis and neovascularization in the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to remove fibrin, slough and biofilm from the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to increase granulation tissue in the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to accelerate epithelialization in the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to drain the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to draw inflammatory fluid away from the chronic non-healing wound.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to decrease pain in the chronic non-healing wound.

Without being bound by theory, the osmotic properties of the granulated sugar of the pharmaceutical compositions are effective to remove excessive fluid from tissue and from cellular bodies of microorganisms. This decreases wound and tissue edema and dehydrates and kills microorganisms. Because microorganisms are killed by a physical rather than a chemical means, there is a reduced risk of bacterial resistance to the pharmaceutical composition. The osmotic property of granulated sugar also is effective to draw chronic inflammatory fluid with excess of harmful and destructive chemokine's away from the wound, thus preventing destruction of healing granulation tissue and allows the wound cells to build healthy granulation tissue. The osmotic property of sugar also is effective to promote gentle debridement and removal of slough and necrotic tissue and hypergranulation and to reduce pain in a chronic non-healing wound. The wound and surrounding edema results from increased inflammation, chemokines, and bacterial load seen in chronic wounds, and this is the reason for pain associated with chronic wounds. By drawing water away from the wound with osmosis, granulated sugar decreases edema and hence decreases wound related inflammatory pain.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to control wound bed infection in the chronic non-healing wound.

Wound bed preparation involves processes that change the biochemical, cellular and molecular environment of a chronic non-healing to an acute healing wound. As shown in Table 2, the pharmaceutical composition of the described invention achieves the full spectrum of wound bed preparation, as opposed to other wound healing products.

**Table 2. Wound bed preparation processes: the pharmaceutical composition compared to other commonly used wound care products**

| | **Designation of Wound Bed Preparation Processes Listed in Table 1** | | | | | | | | | | |
|---|---|---|---|---|---|---|---|---|---|---|---|
| **Wound Care Products** | **A** | **B** | **C** | **D** | **E** | **F** | **G** | **H** | **I** | **J** | **K** |
| Pharmaceutical Formulation (granulated sugar + hydrogel biomaterial) | x | x | x | x | x | x | x | x | x | x | x |
| Hydrogel biomaterial | | | | | | | x | | | | |
| Collagenase based cream (Santyl®) | | | | x | x | | | | | x | |
| Honey based dressings and gels | x | x | x | | x | x | x | x | x | x | |
| Iodosorb® and similar gels | x | x | x | x | | | x | x | x | | |
| Silvadene® | | x | x | | | | | | | | |
| Antibiotic cream/ointment | x | | | | | x | | | | | |
| Gels with growth factors (Regranex®) | | | | | x | | | | | x | |
| Hydrocolloid dressing | | | | | | | | | | | |
| Hydrofiber dressing | | | | | | x | | x | | | |
| Foam dressing | | | | | | x | | x | | | |
| Petroleum based dressing | | | | | | | | | | | |
| Biologic dressings with growth factors | | | | | x | | | | | X | |
| Dressings with collagen | | | | | x | | | | | x | |
| Skin substitutes | | | | | x | | | X | | X | |

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to increase average percent wound closure as compared to a control.

According to some embodiments, the average percent wound closure ranges from about 50% to about 100% greater than a control. According to some embodiments, average percent wound closure by the pharmaceutical composition is about 50% greater than a control. According to some embodiments, average percent wound closure by the pharmaceutical composition is about 60% greater than a control. According to some embodiments, average percent wound closure by the pharmaceutical composition is about 70% greater than a control. According to some embodiments, average percent wound closure by the pharmaceutical composition is about 75% greater than a control. According to some embodiments, average percent wound closure by the pharmaceutical composition is about 80% greater than a control. According to some embodiments, average percent wound closure by the pharmaceutical composition is about 85% greater than a control. According to some embodiments, average percent wound closure by the pharmaceutical composition is about 90% greater than a control. According to some embodiments, average percent wound closure by the pharmaceutical composition is about 95% greater than a control. According to some embodiments, average percent wound closure by the pharmaceutical composition is about 99% greater than a control. According to some embodiments, average percent wound closure by the pharmaceutical composition is about 100% greater than a control.

According to some embodiments, the average percent wound closure is measurable as average percent change in wound area. According to some embodiments, the average percent wound closure is the average percent change in wound volume. According to some embodiments, the average percent wound closure is a combination of the average percent change in wound area and the average percent change in wound volume.

According to some embodiments, wound closure is measurable as a decrease in wound area. According to some embodiments, wound closure is a decrease in wound volume. According to some embodiments, wound closure is a combination of a decrease in wound area and a decrease in wound volume.

According to some embodiments, the percent change in wound area and/or the percent change in wound volume may be determined by using a 3-dimensional camera system such as the ARANZ Silhouette Camera system from ARANZ Medical. The ARANZ Silhouette Camera system is a 3D measurement, imaging and documentation system that provides precise measurement and healing trends along with comprehensive wound surveillance support. The ARANZ Silhouette Camera system is made up of three main parts - a point of care imaging device, smart software and a wound information database. The specialized camera captures the wound image. The camera has no user-adjustable settings - the camera has its own light source; laser lines position the camera for optimum focus and composition; and there is only one moving part - the button which captures the wound image. The software creates a 3D model of the wound based on the data acquired by the camera, derives measurements from the model, and records standardized notes. The information database is a secure Internet-accessible database that stores and consolidates the information obtained from the camera and software. Derived measurements from the ARANZ Silhouette Camera system are likely to be within approximately 2% for area, 1% for perimeter, 5% for average depth and 5% for volume (95% confidence interval). Inter- and intra-rater variability is <1% for area and perimeter, and <2% for average depth and volume. This indicates that repeated measurements over time - even by different assessors - will detect small differences as a wound changes in size and dimensions and the healing trends that are presented will be statistically meaningful and clinically useful.

According to some embodiments, the control is a hydrogel biomaterial.

According to some embodiments, the described invention provides a pharmaceutical composition as defined in the claims for use in a method of treating a chronic non-healing wound in a subject, wherein the method comprises administering to the subject a pharmaceutical composition comprising granulated sugar and a hydrogel biomaterial, wherein the pharmaceutical composition is effective to heal the chronic non-healing wound.

According to some embodiments, the pharmaceutical composition is effective to heal about 50% of chronic non-healing wounds. According to some embodiments, the pharmaceutical composition is effective to heal about 60% of chronic non-healing wounds. According to some embodiments, the pharmaceutical composition is effective to heal about 70% of chronic non-healing wounds. According to some embodiments, the pharmaceutical composition is effective to heal about 75% of chronic non-healing wounds. According to some embodiments, the pharmaceutical composition is effective to heal about 80% of chronic non-healing wounds. According to some embodiments, the pharmaceutical composition is effective to heal about 90% of chronic non-healing wounds. According to some embodiments, the pharmaceutical composition is effective to heal about 95% of chronic non-healing wounds. According to some embodiments, the pharmaceutical composition is effective to heal about 99% of chronic non-healing wounds. According to some embodiments, the pharmaceutical composition is effective to heal about 100% of chronic non-healing wounds.

### Combination Therapy

According to some embodiments, the pharmaceutical composition further comprises at least one additional therapeutic agent.

According to some embodiments, the additional therapeutic agent is an anti-inflammatory agent.

According to some embodiments, the anti-inflammatory agent is a steroidal anti-inflammatory agent. The term "steroidal anti-inflammatory agent", as used herein, refer to any one of numerous compounds containing a 17-carbon 4-ring system and includes the sterols, various hormones (as anabolic steroids), and glycosides. Representative examples of steroidal anti-inflammatory drugs include, without limitation, corticosteroids such as hydrocortisone, hydroxyltriamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflucortolone valerate, fluadrenolone, fluclorolone acetonide, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflorosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof.

According to some embodiments, the anti-inflammatory agent is a nonsteroidal anti-inflammatory agent. The term "non-steroidal anti-inflammatory agent" as used herein refers to a large group of agents that are aspirin-like in their action, including, but not limited to, ibuprofen (Advil®), naproxen sodium (Aleve®), and acetaminophen (Tylenol®). Additional examples of non-steroidal anti-inflammatory agents that are usable in the context of the described invention include, without limitation, oxicams, such as piroxicam, isoxicam, tenoxicam, sudoxicam, and CP-14,304; disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; fenamates, such as mefenamic, meclofenamic, flufenamic, niflumic, and tolfenamic acids; propionic acid derivatives, such as benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone. Mixtures of these non-steroidal anti-inflammatory agents also may be employed, as well as the dermatologically acceptable salts and esters of these agents. For example, etofenamate, a flufenamic acid derivative, is particularly useful for topical application.

According to another embodiment, the anti-inflammatory agent includes, without limitation, Transforming Growth Factor- beta3 (TGF-β3), an anti-Tumor Necrosis Factor-alpha (TNF-α) agent, or a combination thereof.

According to some embodiments, the additional agent is an analgesic agent. According to some embodiments, the analgesic agent relieves pain by elevating the pain threshold without disturbing consciousness or altering other sensory modalities. According to some such embodiments, the analgesic agent is a non-opioid analgesic. "Non-opioid analgesics" are natural or synthetic substances that reduce pain but are not opioid analgesics. Examples of non-opioid analgesics include, but are not limited to, etodolac, indomethacin, sulindac, tolmetin, nabumetone, piroxicam, acetaminophen, fenoprofen, flurbiprofen, ibuprofen, ketoprofen, naproxen, naproxen sodium, oxaprozin, aspirin, choline magnesium trisalicylate, diflunisal, meclofenamic acid, mefenamic acid, and phenylbutazone. According to some other embodiments, the analgesic is an opioid analgesic. "Opioid analgesics", "opioid", or "narcotic analgesics" are natural or synthetic substances that bind to opioid receptors in the central nervous system, producing an agonist action. Examples of opioid analgesics include, but are not limited to, codeine, fentanyl, hydromorphone, levorphanol, meperidine, methadone, morphine, oxycodone, oxymorphone, propoxyphene, buprenorphine, butorphanol, dezocine, nalbuphine, and pentazocine.

According to another embodiment, the additional agent is an anti-infective agent. According to another embodiment, the anti-infective agent is an antibiotic agent. The term "antibiotic agent" as used herein means any of a group of chemical substances having the capacity to inhibit the growth of, or to destroy bacteria, and other microorganisms, used chiefly in the treatment of infectious diseases. Examples of antibiotic agents include, but are not limited to, Penicillin G; Methicillin; Nafcillin; Oxacillin; Cloxacillin; Dicloxacillin; Ampicillin; Amoxicillin; Ticarcillin; Carbenicillin; Mezlocillin; Azlocillin; Piperacillin; Imipenem; Aztreonam; Cephalothin; Cefaclor; Cefoxitin; Cefuroxime; Cefonicid; Cefmetazole; Cefotetan; Cefprozil; Loracarbef; Cefetamet; Cefoperazone; Cefotaxime; Ceftizoxime; Ceftriaxone; Ceftazidime; Cefepime; Cefixime; Cefpodoxime; Cefsulodin; Fleroxacin; Nalidixic acid; Norfloxacin; Ciprofloxacin; Ofloxacin; Enoxacin ; Lomefloxacin; Cinoxacin; Doxycycline; Minocycline; Tetracycline; Amikacin; Gentamicin; Kanamycin; Netilmicin; Tobramycin; Streptomycin; Azithromycin; Clarithromycin; Erythromycin; Erythromycin estolate ; Erythromycin ethyl succinate; Erythromycin glucoheptonate; Erythromycin lactobionate; Erythromycin stearate; Vancomycin; Teicoplanin; Chloramphenicol; Clindamycin; Trimethoprim; Sulfamethoxazole; Nitrofurantoin; Rifampin; Mupirocin; Metronidazole; Cephalexin; Roxithromycin; Co-amoxiclavuanate; combinations of Piperacillin and Tazobactam; and their various salts, acids, bases, and other derivatives. Anti-bacterial antibiotic agents include, but are not limited to, penicillins, cephalosporins, carbacephems, cephamycins, carbapenems, monobactams, aminoglycosides, glycopeptides, quinolones, tetracyclines, macrolides, and fluoroquinolones.

Other examples of at least one additional therapeutic agent include, but are not limited to, rose hip oil, vitamin E, 5-fluorouracil, bleomycin, onion extract, pentoxifylline, prolyl-4-hydroxylase, verapamil, tacrolimus, tamoxifen, tretinoin, colchicine, a calcium antagonist, tranilst, zinc, a growth factor, and a combination thereof. Examples of growth factors include, but are not limited to, platelet-derived growth factor (PDGF), fibroblast growth factor (FGF) and granulocyte macrophage colony stimulating factor (GM-CSF).

### Adjunct Therapy

According to some embodiments, the pharmaceutical composition is administered in combination with an adjunct therapy. Non-limiting examples of adjunct therapies include, hyperbaric oxygen (HBO), gene therapy, stem cell therapy, bioengineered skin, skin equivalents, skin substitutes, skin grafts and the like. Bioengineered skin, skin equivalents and skin substitutes may be derived, for example, from human tissue (autologous or allogeneic), non-human tissue (xenographic), synthetic materials and composite materials. Examples of skin grafts include, but are not limited to, split skin grafts and full thickness skin grafts. Split skin grafts are taken by shaving the surface layers (epidermis and a variable thickness of dermis) of the skin with a large knife called a dermatome. The shaved piece of skin is then applied to the wound. This type of skin graft is often taken from the leg. Full thickness skin grafts are taken by removing all the layers of the skin with a scalpel (a Wolfe graft). It is done in a similar way to skin excision. The piece of skin is cut into the correct shape, then applied to the wound. This type of skin graft is often taken from the arm, neck or behind the ear.

Unless defined otherwise, all technical and scientific terms used herein have the same meaning as commonly understood by one of ordinary skill in the art to which this invention belongs. Although any methods and materials similar or equivalent to those described herein also can be used in the practice or testing of the described invention, the preferred methods and materials are now described.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

It must be noted that as used herein and in the appended claims, the singular forms "a", "and", and "the" include plural references unless the context clearly dictates otherwise. All technical and scientific terms used herein have the same meaning.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1. Clinical Observational Study

Data was collected from a wound -care specific electronic medical record (EMR) which has allowed us to assess real-world outcomes in 41wounds. We used data from the wound EMR data base Intellicure, Inc. to perform a retrospective analysis on wounds treated with topical granulated sugar. Intellicure, Inc. is a wound specific EMR used widely in wound centers across United States. Treatment records include patient demographics, wound type, location, underlying etiology, area measurement, wound specific characteristics, percentage closure recorded at each visit, etc. The EMR is designed to accurately record wound size and characteristics at each visit and percent change in wound area over time. The EMR also records other treatment and procedures or complications or infections as they occur and treatment records. Using the EMR to capture data on healing outcomes has the advantage of capturing progress of healing in real time. Treatment records of wounds from October 2013 to October 2015 were analyzed. In these wounds, granulated sugar was used as topical treatment 75-100% of the time. Granulated sugar was mixed with a hydrogel biomaterial (1:1 mixture) in most cases with a few drops of povidone iodine or mixed with topical antibiotic or petroleum ointment (Topical sugar mixture). However the main ingredient in all cases was granulated sugar. The etiology of wounds included surgical, diabetic, venous, osteomyelitis, traumatic and radiation. These wounds represent the typical mix of wounds seen in a hospital outpatient wound center. The primary analyses were time to healing and safety of the sugar mixture. Wounds with 75-100% healing were included.

Prior to starting treatment at our wound center, most wounds were non-healing for 1-3 months, some wounds for more than a year, and one wound for 36 years. Our results show that 37 wounds healed 100%, 3 wounds healed 99%, and one wound healed 90% after being treated with topical sugar mixture 75-100% of the time. Except for one radiation ulcer, all ulcers healed within 7 months. Most of the ulcers that healed 100% healed within 1-3 months. The other ulcers healed between 5 and 7 months. The ulcers that healed rapidly or within 1 month were either traumatic or surgical or had tissue edema successfully managed. The ulcers that took more than 3 months to heal had necrosis, osteomyelitis or were non-healing for over a year prior to initiating treatment in our wound center.

In addition to topical sugar treatment with the described pharmaceutical composition, 13 ulcers underwent hyperbaric oxygen (HBO) adjunct therapy, 3 ulcers had skin substitutes as adjunct therapy and one ulcer had a combination of HBO and epidermal graft as adjunct therapy.

The following observations were made in all the cases: (i) there were no allergies or local or systemic side effects; (ii) no infection on wound was noted after initiation of sugar therapy if wound was adequately debrided of necrotic tissue; (iii) fibrin and biofilms resolved without any other debridement and granulation tissue grew and filled the wound area; and (iv) for the ulcers that had application of skin substitutes, the sugar treatment prepared the ulcer with good granulation and as a result, other adjunctive treatment such as skin substitutes were effective and quickly epithelized the ulcer bed prepared by the described pharmaceutical composition.

In this study, all ulcers showed progressive improvement with an increase in granulation tissue and accelerated epithelialization throughout the healing period. A decrease in necrotic tissue, odor, pain, and drainage was also observed. None of the patients developed new necrosis, infection, skin irritation, pain, or deceleration in the rate of healing. Safety of the treatment was concluded based on the lack of observed and reported local or systemic adverse effects.

The results of this study indicate that a topical pharmaceutical composition containing granulated sugar mixed with a wound hydrogel, or topical antibiotic can be safely applied to non-healing wounds of any etiology without producing harmful side effects. The pharmaceutical composition improved, enhanced and accelerated all wound healing processes including removal of wound fibrin, slough and biofilms, building of new healthy granulation tissue, control of wound infection, contraction of the wound and building of epithelial coverage. In many of the wounds treated, the pharmaceutical composition progressively and completely healed the wounds. In cases that required adjunctive wound therapies, the pharmaceutical composition accelerated healing and made adjunctive therapies more effective by improving all wound healing processes.

In addition to the retrospective analysis, two clinical observational studies were performed.

In the first clinical observational study, daily dressings using antibiotic/wound gel mixed with granulated sugar (1:1) were used as the primary contact dressing on non-healing wounds which included wounds with diabetic, venous, surgical and cancer etiology. Thirteen patients were enrolled. Of the thirteen enrolled patients, 9 patients had complete healing; 3 patients were lost to follow-up (having at least 50% healing on last documented follow-up; and 1 patient was 90% healed. The time to complete healing was 1 to 3 months in 7 patients and 12 months in 2 patients. With respect to the 2 patients that healed in 12 months, 1 patient had a gangrenous diabetic ulcer and was saved by below knee amputation, and 1 patient had a breast wound with breast cancer treated with ongoing chemotherapy.

In the second clinical observational study, wound gel with granulated sugar (1:1) was used as the primary dressing in eight non-healing diabetic foot ulcers (DFUs) as an adjunct to standard therapy. Of the eight patients studied, 7 patients had 100% healing while 1 patient was lost to follow-up. Time to complete healing was 2 to 4 months in 3 patients and 7 to 11 months in four patients. None of the patients required amputation or major debridement.

Based on these observations, the following animal experiments were conducted in order to demonstrate the effects of sugar in an animal wound model.

### Example 2. In vivo Mouse Study

In this study, Swiss Webster retired breeder female mice were used (i) to demonstrate the difference in rate of wound closure between wounds treated with dressings comprising a composition containing granulated sugar and a hydrogel biomaterial and standard of care hydrogel biomaterial dressing; (ii) to compare and assess development of infection in wounds receiving dressings comprising a composition containing granulated sugar and a hydrogel biomaterial vs. control dressing; and (iii) to determine the difference in rate of progression and the quantification of key elements in tissue repair between dressings comprising a composition containing granulated sugar and a hydrogel biomaterial and control. Histological analyses is performed 3, 6, 9 and 11 days post-wounding based on previous studies in which lactate supplemented subcutaneous matrigel implants in mice showed an inflammatory response by day 3, early granulation tissue by day 9, and mature granulation tissue by day 11 (Hunt T K et al., Antioxid Redox Signal, 2007. 9(8): 1115-1124; Berry D P et al., Plast Reconstr Surg, 1998. 102(1): 124-131; discussion 132-134).

Swiss Webster retired breeder mice were divided into the following 5 groups:
1. Experimental group 1 (n=3) with dressing comprising a composition containing granulated sugar and a hydrogel biomaterial on the wounds per each time point. A total of n=12 for this sub-group;
2. Experimental group 2 (n=3) with dressing comprising a composition containing granulated sugar and a hydrogel biomaterial and iodine on the wounds per each time point. A total of n=12 for this sub-group;
3. Experimental group 3 (n=3) with wound bacterial swab receiving dressing comprising a composition containing granulated sugar and a hydrogel biomaterial on the wounds per each time point. A total of n=12 for this sub-group;
4. Control group 1 (n=3) with wound bacterial swab receiving control dressing on the wounds per each time point. A total of n=12 for this sub-group; and
5. Control group 2 (n=3) with control dressing on the wounds per each time point. A total of n=12 for this sub-group.

Sugar dressings were prepared by covering the composition of the described invention with non-adherent telfa dressing (Covidien) secured with transparent film and coban. The composition of the described invention was compounded by mixing 1:1 v/v granulated sugar (table sugar) with a hydrogel biomaterial. The hydrogel biomaterial ("wound gel") comprised purified water 70%, glycerol 30 % and hydroxy ethyl cellulose. A small amount of allantoin and sodium lactate were also added. The hydrogel biomaterial was prepared at an outside pharmacy. Sugar iodine dressings were prepared by covering a mixture of the composition of the described invention and iodine with non-adherent telfa dressing secured with transparent film and coban. The sugar iodine mixture was compounded by mixing a hydrogel biomaterial, granulated sugar (table sugar) and iodine in a ratio of 1:1:0.17 (14.7ml/14.7ml/2.5ml). Control dressing was hydrogel biomaterial alone with non-adherent telfa dressing secured with transparent film and coban.

Mice were housed individually in standard cages in an AAALAC-accredited animal care facility. Mice had free access to water and chow diet. Mice were anesthetized using inhalant anesthesia. Post-operative analgesia was done by injecting Buprenorphine subcutaneously 0.8ml immediate post-operatively and six hours later. Any additional analgesia was given on an as needed basis every 12 hours. Need was to be assessed by monitoring pain behavior which was defined as decreased eating and drinking and/or change in activity. In the case of a severe response that appeared hazardous to the mice, the mice were euthanized. Signs of pain or distress severe enough to indicate need for euthanasia included weight loss > 10%, moribund and an inability to eat or drink. The method of euthanasia was CO₂ asphyxiation followed by cervical dislocation.

Wounding procedure and wound analysis were performed in a manner similar to that described in the study by Keylock et al. (Am J Physiol Regul Integr Comp Physiol, 2008. 294(1): R179-184). Animals were anesthetized using an inhalant anesthetic via cone mask under a flow hood with isofluorane (Isoflo) in 100% oxygen at a flow rate of 2 to 3 1/min continuously. After being anesthetized, animals were shaved and treated with three alternating scrubs of povidone-iodine and isopropyl alcohol. Two full-thickness circular wounds on the upper dorsal area of each animal were created using a 3.5-mm sterile, disposable punch biopsy instrument. The skin removed with the punch biopsy was fixed in 4% formaldehyde for histopathology. Animals were immediately photographed for assessment of baseline wound area and then returned to their cage after application of appropriate dressing according to their experimental group. After isofluorane anesthesia, animals returned to consciousness in about 3-5 minutes. Animals were monitored until full recovery from the procedure. From each group, a subgroup (n=3) of animals were sacrificed on day 3, day 6, day 9, and day 11, post-wounding by rapid CO₂ asphyxiation followed by cervical dislocation, and wounds and surrounding tissue were harvested. As each animal had 2 wounds, one wound along with surrounding tissue was harvested and fixed in 4% formaldehyde for histopathology and immunohistochemistry; the second wound along with surrounding tissue was harvested and frozen for further testing.

A 0.1-ml saline suspension containing 10⁶ organisms/ml of *Pseudomonas aeruginosa (P. aeruginosa)* (ATCC 27853) was applied to the wounds and gently rubbed in with a sterile teflon rod and allowed to dry. The site was allowed to dry, and then immediately covered with the appropriate dressing. To minimize the risk of spread of infection and contamination, animals were held in individual micro-cages in a quarantined animal facility. Personal protective equipment (PPE) and general protective and hygienic measures were followed according to the standards set by the Centers for Disease Control and Prevention (CDC). The room housing the animals had restricted entry of only study related personnel observing appropriate PPE and general protective and hygienic measures as per CDC guidelines. As per the SDS for *P. aeruginosa,* after the product was used, disinfection was done with bleach (sodium hypochlorite).

Animals were photographed each day after wounding. Each animal had 2 wounds which were both photographed. The wound area was assessed and a new dressing applied. Animals were anesthetized with isofluorane administered with oxygen at a flow rate of 2-3 l/min and the wounds were photographed before recovery from anesthesia. Wound photography and assessment of wound area was performed by an ARANZ (ARANZ Medical) Silhouette Camera which provides an accurate 3-D measurement and assessment of wound area. Visual (i.e., naked eye) assessment of wound characteristics was also performed and recorded. The ARANZ system includes an imaging device that precisely and consistently measures the area, depth and volume of wounds and their healing progress, and software to store and manage wound informatics. The software calculates and stores the percent change (decrease or increase) in area/volume of wound with every photograph. The ARANZ silhouette camera enables wound assessment activity to be monitored on each day starting from day of wounding to day of sacrifice.

The clinical reaction to inoculation was graded on the following scale:
0 = no reaction
1 = definite edema, mild erythema
2 = intense edema and intense erythema
3 = edema and erythema extending beyond the borders of the wound
4 = edema and erythema extending beyond the borders of the wounds with pustule formation

Grades 2, 3 and 4 were categorized as development of infection (Leyden et al., Journal of the Society of Cosmetic Chemists, Vol. 31, No. 1, 19-28).

To assess bacterial burden in animals with bacterial contamination of wounds, quantification of bacteria is determined by histopathology. Samples are sectioned and stained. Using histopathology and immunohistochemistry, the rate of collagen synthesis, the rate of neovascularization, inflammatory wound response, tissue organization, the rate of epithelialization, and the quantity of angiogenesis is assessed. Frozen samples are used for biochemical analysis at a later stage based on histopathology results if needed.

The wounds in groups 1 (Experimental group with sugar and hydrogel dressing- sugar group), 2 (experimental group with sugar hydrogel and iodine dressing - sugar/iodine group) and 5 (group 5 control group with hydrogel dressing alone-control group) were photographed each day with the ARANZ camera and the ARANZ software calculated percent change in the area or volume of the wound. Day of wounding was day 1. The percent change in area for days 2 to 11 post-wounding were recorded for all wounds (2 wounds per animal).

The percent change for all wounds for each group for each day post-wounding were averaged to give average percent closure. Positive percent closure reflected a decrease in wound area and negative percent closure reflected an increase in wound area.

The results of this study are as follows: (i) on days 2,3,4,5,6 post-wounding, average percent wound closure was higher in the sugar dressing group as compared to control and sugar/iodine dressing groups; (ii) the largest difference was at day 6 post-wounding, when percent closure in the sugar dressing group was more than 50% higher as compared to control and sugar/iodine dressing groups; (iii) on days 7,8,9 post-wounding, the average percent closure was similar between the sugar dressing group and the control group, however average percent closure of both the sugar dressing and the control groups was higher than the sugar/iodine dressing group; (iv) at day 10 post-wounding, most wounds in the sugar dressing group were healed (average closure 95%) which was 1 day earlier than both the control group (average closure79%) and sugar /iodine dressing group (average closure 69%); (v) at final day 11 post wounding, wounds in the sugar dressing group healed 99 percent; the control group healed 96 percent and sugar/iodine dressing group healed 86 percent; and (vi) overall, the average percent closure in the sugar/iodine dressing group was less than both the sugar dressing group and the control group with a greater difference between the sugar and the sugar/iodine dressing groups as compared to the difference between the sugar dressing group and the control group.

Visual (i.e., naked eye) observations of the wounds showed early epithelialization in the sugar dressing group at day 4-5 post-wounding as compared with the control and the sugar/iodine dressing group. It was also noted that once wounds epithelized in the sugar dressing group, the epithelialization was progressive and maintained until the last day post-wounding. In contrast, the epithelialization in the control and the sugar/iodine dressing group was not progressive and had intermittently regressed.

None of the animals showed pain behavior which would have required additional analgesia after the wounding day. Likewise, no animals developed any signs of stress or loss of weight. All animals were euthanized at the time points per study protocol.

The results of this study indicate that the wounds healed with contraction from wound edges as well as with epithelialization of the wound bed. The rate of wound closure or contraction between the sugar dressing group, the control group and the sugar/iodine dressing group was highest in the sugar dressing group. Visual (i.e., naked eye) observation of wound epithelialization showed earlier and progressive epithelialization in the sugar dressing group as compared to the control group and the sugar/iodine dressing group.

In this study, Swiss Webster retired breeder female mice were used to demonstrate that sugar accelerates wound healing in experimental wounds. A total of 36 mice were divided evenly (n=12) into 3 groups. Two full thickness circular wounds were created per animal treated daily with (i) granulated sugar plus wound gel (1:1) (Group 1); (ii) wound gel only (Group 2); and (iii) granulated sugar plus wound gel plus iodine (1:1:0.17) and each group was sacrificed on days 3, 6, 9 and 11 for subgroup analyses. The protocol for this experiment was reviewed and approved by the IACUUC Committee Hackensack University Medical Center, NJ

Sugar dressings were prepared using a sugar mixture covered with a non-adherent dressing pad secured with transparent film and self-adhering elastic support. The sugar mixture was compounded by mixing 1:1 v/v granulated sugar (e.g., table sugar) with wound gel comprising purified water 70%, glycerol 30 % and hydroxy ethyl cellulose. A small amount of allantoin and sodium lactate was also added. The wound gel was prepared at an outside pharmacy for the entire experiment with an expiration of one month. Sugar was added to the wound gel on each dressing day to prepare the sugar mixture. Sugar iodine dressing was prepared using a sugar iodine mixture covered with a non-adherent dressing pad secured with transparent film and self-adhering elastic support wrap. Sugar iodine mixture was compounded by mixing wound gel, granulated sugar (e.g., table sugar) and iodine (povidone - iodine USP in a ratio of 1:1:0.17 (14.7ml/14.7ml/2.5ml). Sugar and iodine were added to the wound gel on each dressing day to prepare the sugar iodine mixture. Control dressings were prepared using wound gel alone with a non-adherent dressing pad secured with transparent film and self-adhering elastic support wrap.

Mice were divided into 3 groups as follows:
Experimental group 1: n=12 - with sugar dressing on the wounds per each time point.

Control group 2: n=12 with control dressing on the wounds per each time point.

Experimental group 3 n=12 with sugar and iodine dressing on the wounds per each time point.

Mice were housed individually in standard cages in an AAALAC-accredited animal care facility. Mice had free access to water and chow diet. Mice were anesthetized using inhalant anesthesia as described in the wounding procedure. Post-operative analgesia was done by injecting Buprenorphine subcutaneously 0.08 ml immediate post operatively. Per protocol, additional analgesia was to be given 6 hours post-operatively and subsequently 12 hourly on as needed basis. Need was to be assessed by monitoring pain behavior which is defined as decreased eating and drinking and / or change in activity. Per protocol if there was a severe response that appears hazardous to the mice, the mice were to be euthanized. Signs of pain or distress severe enough to indicate need for euthanasia include visible weight loss, moribund and unable to eat or drink. Method of euthanasia was CO₂ asphyxiation followed by cervical dislocation.

Wounding procedure and wound analysis was done in a similar manner described in the study by Keylock et al (Exercise accelerates cutaneous wound healing and decreases wound inflammation in aged mice. Am J Physiol Regul Integr Comp Physiol, 2008. 294(1): p. R179-84). Animals were anesthetized using a transparent closed box with an inflow of isoflurane (IsofloTM) in 100% oxygen @ 3-4 1/min for 2-3 minutes. Once the animal stopped moving, anesthesia was administered via cone mask under a flow hood with a flow rate of 2 to 3 1/min continuously. After being anesthetized, animals were shaved and treated with alternating scrubs of povidone-iodine and isopropyl alcohol. Two full-thickness circular wounds on the upper dorsal area of each animal were created using a 3.5-mm sterile, disposable punch biopsy instrument. The skin removed with the punch biopsy was fixed in 4% formaldehyde for histopathology. Animals were immediately photographed for assessment of baseline wound area and then returned to their cage after application of appropriate dressing according to their group. After isoflurane anesthesia, animals returned to consciousness in about 3-5 minutes. Animals were monitored until full recovery from the procedure.

From each group, a subgroup (n=3) of animals were sacrificed on day 3, day 6, day 9, and day 11 post wounding by rapid CO₂ asphyxiation, and wounds and surrounding tissue were harvested. As each animal had 2 wounds, one wound harvested with surrounding tissue was fixed in 4% formaldehyde for histopathology and immunohistochemistry; the second wound harvested with surrounding tissue was frozen for further testing. Days 3, 6, 9, 11 post-wounding were selected for histologic analyses. This time-line was based on a previous study in which lactate supplemented subcutaneous matrigel implants in mice showed an inflammatory response by day 3, early granulation tissue by day 9 and mature granulation tissue by day 11 (Hunt, T.K., et al., Aerobically derived lactate stimulates revascularization and tissue repair via redox mechanisms. Antioxid Redox Signal, 2007. 9(8): p. 1115-24). Similar observation by Berry et. al. (Human wound contraction: collagen organization, fibroblasts, and myofibroblasts. Plast Reconstr Surg, 1998. 102(1): p. 124-31; discussion 132-4) in wound punch biopsies showed acute inflammation day 3, early granulation day 6 and mature granulation day 11.

Animals were photographed each day after wounding. Since each animal had 2 wounds, both were photographed. The wound area was assessed and a new dressing applied. Animals were anesthetized with isoflurane administered with 100% oxygen at a flow rate of 2-3 L/min and the wounds were photographed before recovery from anesthesia. Each wound was photographed using an ARANZ (Aranz Medical) Silhouette Camera which provides an accurate 3-D measurement of wound area. The photograph of the wound was traced manually taking care to accurately outline the wound bed - skin interface. The tracings were reviewed by at least one independent reviewer and, where there was conflict, the tracings were reviewed and reconciled by two reviewers. Based on the tracings, the software calculated the 3-D measurements of the wound area, and calculated and stored the percent change (decrease or increase) in area/volume of wound with each photographed wound. The ARANZ silhouette camera enabled wound assessment activity to be monitored on each day starting from day of wounding to day of sacrifice.

Visual (i.e., naked eye) assessment of wound characteristics was also performed and recorded on a daily basis on each wound. These included wound bed assessment including: (i) granulation vs. necrotic/fibrin; (ii) wound drainage; (iii) peri-wound area; and (iv) any other unusual findings. Epithelialization and healing in addition to wound characteristics were also recorded. The visual observations were recorded by at least two observers.

After performing an assessment of wound characteristics, no notable difference in the three groups was observed in the wound bed composition; wound drainage and peri-wound area, with the exception of epithelialization. No unusual signs suggesting wound necrosis or infection were observed. The visual observations of the wounds showed early epithelialization in the sugar group at day 4-5 post-wounding. In Group 1, early epithelialization was seen as early as day 3, with 50% of the wounds showing epithelialization on Day 4. No epithelialization was observed in any of the wounds in group 2 and 3 until Day 5. Only 6% of the wounds showed epithelialization in group 2 by day 6, and 0% of the wounds showed epithelization in group 3. A third independent observer reported epithelialization in the three groups after review of pictures from the ARANZ camera. These results matched the results reported by visual observation. It was also noted on visual observation that more wounds healed in group 1 as compared to groups 2 and 3.

None of the animals showed pain behavior which required additional analgesia after the wounding day and no animal developed any signs of stress or lost weight. All animals were euthanized at the time points per study protocol.

The results of this study indicate that the wounds healed with contraction from wound edges as well as with epithelialization of the wound bed. The rate of wound closure or contraction between the sugar, control and sugar/iodine groups was highest in the sugar group. Visual (i.e. naked eye) observation of wound epithelialization showed earlier epithelialization in the sugar group as compared to the control and the sugar/iodine groups.

## Claims

1. A pharmaceutical composition comprising in equal parts 1:1 v/v granulated sugar and a hydrogel biomaterial comprising a water-swollen polymeric material
for use in a method of treating a chronic non-healing wound,
wherein the pharmaceutical composition increases average percent change in wound closure compared to a control, wherein the control is the hydrogel biomaterial; wherein:
(a) the hydrogel biomaterial comprises purified water, glycerol, hydroxyl ethyl cellulose, sodium lactate, and allantoin, wherein:
(i) the purified water is about 70% of the hydrogel biomaterial; and
(ii) the glycerol is about 30% of the hydrogel biomaterial; and
(b) the pharmaceutical composition is administered:
(i) as a topical formulation; or
(ii) as a dressing, wherein a surface of the dressing is impregnated with the composition.

2. The pharmaceutical composition for use according to claim 1, wherein the pharmaceutical composition increases the average percent change in wound closure as compared to the control, preferably wherein the average percent change in wound closure ranges from 50% to 100% as compared to the control, such as at least 50%, 95%, or 99% greater than the control.

3. The pharmaceutical composition for use according to claim 1, wherein the pharmaceutical composition is impregnated on at least one surface of the dressing, preferably wherein the dressing comprises:
(a) an occlusive dressing selected from the group consisting of: a film dressing, a semi-permeable film dressing, a hydrogel dressing, a hydrocolloid dressing, and a combination thereof; and
(b) a non-adherent pad.

4. The pharmaceutical composition for use according to claim 1, wherein the average percent wound closure is average percent change in wound area, average percent change in wound volume or a combination thereof, preferably wherein the average percent change in wound area, the average percent change in wound volume, or the combination thereof is determined by a 3-dimensional camera system.

5. The pharmaceutical composition for use according to claim 1, wherein the pharmaceutical composition further comprises at least one additional therapeutic agent.

6. The pharmaceutical composition for use according to claim 5, wherein the at least one additional therapeutic agent is an anti-inflammatory agent, an analgesic agent, an anti-infective agent, a growth factor or a combination thereof, preferably wherein the anti-infective agent is an antibiotic agent.

7. The pharmaceutical composition for use according to claim 6, wherein the growth factor is selected from the group consisting of platelet-derived growth factor (PDGF), fibroblast growth factor (FGF) and granulocyte macrophage colony stimulating factor (GM-CSF).

8. The pharmaceutical composition for use according to claim 1, wherein the pharmaceutical composition is administered in combination with an adjunct therapy.

9. The pharmaceutical composition for use according to claim 8, wherein the adjunct therapy is hyperbaric oxygen (HBO), gene therapy, stem cell therapy, bioengineered skin, a skin equivalent, a skin substitute, a skin graft or a combination thereof.

10. The pharmaceutical composition for use according to claim 1, wherein the chronic non-healing wound is a diabetic wound, a venous wound, a surgical wound, a cancer wound, a pressure ulcer, an arterial ulcer or a combination thereof.

11. The pharmaceutical composition for use according to claim 1, wherein the pharmaceutical composition decreases:
(a) wound and tissue edema;
(b) necrotic tissue; and
(c) pain
in the chronic non-healing wound.

12. The pharmaceutical composition for use according to claim 1, wherein the pharmaceutical composition:
(a) kills microorganisms in the chronic non-healing wound;
(b) accelerates wound healing in the chronic non-healing wound;
(c) increases collagen synthesis and neovascularization in the chronic non-healing wound;
(d) removes:
(i) fibrin;
(ii) slough; and
(iii) biofilm in the chronic non-healing wound;
(e) increases granulation tissue in the chronic non-healing wound;
(f) accelerates epithelialization in the chronic non-healing wound;
(g) drains the chronic non-healing wound;
(h) draws inflammatory fluid away from the chronic non-healing wound;
(i) controls wound bed infection in the chronic non-healing wound; or
(j) achieves wound bed preparation in the chronic non-healing wound.

## Patentansprüche

1. Pharmazeutische Zusammensetzung, die zu gleichen Teilen 1:1 Vol./Vol. granulierten Zucker und ein Hydrogel-Biomaterial umfasst, das ein wassergequollenes Polymermaterial zur Verwendung in einem Verfahren zur Behandlung einer chronisch nicht heilenden Wunde umfasst, wobei die pharmazeutische Zusammensetzung die durchschnittliche prozentuale Änderung des Wundverschlusses im Vergleich zu einer Kontrolle erhöht, wobei die Kontrolle das Hydrogel-Biomaterial ist; wobei:
(a) das Hydrogel-Biomaterial gereinigtes Wasser, Glycerin, Hydroxylethylcellulose, Natriumlactat und Allantoin umfasst, wobei:
(i) das gereinigte Wasser etwa 70% des Hydrogel-Biomaterials ausmacht; und
(ii) das Glycerin etwa 30% des Hydrogel-Biomaterials ausmacht; und
(b) die pharmazeutische Zusammensetzung
(i) als topische Formulierung oder
(ii) als Verband wird verabreicht, wobei eine Oberfläche des Verbands mit der Zusammensetzung imprägniert ist.

2. Pharmazeutische Zusammensetzung zur Verwendung nach Anspruch 1, wobei die pharmazeutische Zusammensetzung die durchschnittliche prozentuale Änderung des Wundverschlusses im Vergleich zur Kontrolle vorzugsweise erhöht, wobei die durchschnittliche prozentuale Änderung des Wundverschlusses im Vergleich zur Kontrolle zwischen 50% und 100%, beispielsweise mindestens 50%, 95% oder 99% höher als bei der Kontrolle, liegt.

3. Pharmazeutische Zusammensetzung zur Verwendung nach Anspruch 1, wobei die pharmazeutische Zusammensetzung vorzugsweise auf mindestens einer Oberfläche des Verbands imprägniert ist, wobei der Verband Folgendes umfasst:
(a) einen Okklusivverband, der aus der Gruppe ausgewählt ist, die aus Folgendem besteht: einem Filmverband, einem semipermeablen Filmverband, einem Hydrogelverband, einem Hydrokolloidverband und einer Kombination davon; und
(b) eine nicht anhaftende Kompresse.

4. Pharmazeutische Zusammensetzung zur Verwendung nach Anspruch 1, wobei der durchschnittliche prozentuale Wundverschluss eine durchschnittliche prozentuale Änderung des Wundbereichs, eine durchschnittliche prozentuale Änderung des Wundvolumens oder eine Kombination davon ist, wobei die durchschnittliche prozentuale Änderung der Wundfläche, die durchschnittliche prozentuale Änderung des Wundvolumens oder die Kombination davon vorzugsweise durch ein dreidimensionales Kamerasystem bestimmt wird.

5. Pharmazeutische Zusammensetzung zur Verwendung nach Anspruch 1, wobei die pharmazeutische Zusammensetzung ferner mindestens ein zusätzliches therapeutisches Mittel umfasst.

6. Pharmazeutische Zusammensetzung zur Verwendung nach Anspruch 5, wobei das mindestens eine zusätzliche therapeutische Mittel ein entzündungshemmendes Mittel, ein Analgetikum, ein antiinfektiöses Mittel, ein Wachstumsfaktor oder eine Kombination davon ist, wobei das antiinfektiöse Mittel vorzugsweise ein Antibiotikum ist.

7. Pharmazeutische Zusammensetzung zur Verwendung nach Anspruch 6, wobei der Wachstumsfaktor ausgewählt ist aus der Gruppe bestehend aus einem von Blutplättchen abgeleiteten Wachstumsfaktor (Platelet-Derived Growth Factor, PDGF), einem Fibroblasten-Wachstumsfaktor (Fibroblast Growth Factor, FGF) und einem Granulozyten-Makrophagen-Kolonie-stimulierendem Faktor (Granulocyte Macrophage-Colony Stimulating Factor, GM-CSF).

8. Pharmazeutische Zusammensetzung zur Verwendung nach Anspruch 1, wobei die pharmazeutische Zusammensetzung in Kombination mit einer Zusatztherapie verabreicht wird.

9. Pharmazeutische Zusammensetzung zur Verwendung nach Anspruch 8, wobei die Zusatztherapie hyperbarer Sauerstoff (HyperBaric Oxygen, HBO), Gentherapie, Stammzelltherapie, biotechnologisch hergestellte Haut, ein Hautäquivalent, ein Hautersatz, ein Hauttransplantat oder eine Kombination davon ist.

10. Pharmazeutische Zusammensetzung zur Verwendung nach Anspruch 1, wobei die chronische nicht heilende Wunde eine diabetische Wunde, eine venöse Wunde, eine chirurgische Wunde, eine Krebswunde, ein Druckgeschwür, ein arterielles Geschwür oder eine Kombination davon ist.

11. Pharmazeutische Zusammensetzung zur Verwendung nach Anspruch 1, wobei die pharmazeutische Zusammensetzung Folgendes verringert:
(a) Wund- und Gewebeödem;
(b) nekrotisches Gewebe; und
(c) Schmerz
in der chronischen nicht heilenden Wunde.

12. Pharmazeutische Zusammensetzung zur Verwendung nach Anspruch 1, wobei die pharmazeutische Zusammensetzung:
(a) Mikroorganismen in der chronisch nicht heilenden Wunde abtötet;
(b) die Wundheilung in der chronisch nicht heilenden Wunde beschleunigt;
(c) die Kollagensynthese und Neovaskularisation in der chronischen nicht heilenden Wunde erhöht;
(d) Folgendes entfernt:
(i) Fibrin;
(ii) Schorf; und
(iii) Biofilm in der chronischen nicht heilenden Wunde;
(e) das Granulationsgewebe in der chronisch nicht heilenden Wunde erhöht;
(f) die Epithelisierung in der chronischen nicht heilenden Wunde beschleunigt;
(g) die chronisch nicht heilende Wunde entwässert;
(h) der chronischen nicht heilenden Wunde entzündliche Flüssigkeit entzieht;
(i) die Wundbettinfektion in der chronischen nicht heilenden Wunde kontrolliert; oder
(j) eine Wundbettvorbereitung in der chronisch nicht heilenden Wunde erreicht.

## Revendications

1. Composition pharmaceutique comprenant à parts égales 1 : 1 v / v de sucre granulé et un biomatériau hydrogel comprenant un matériau polymère gonflé à l'eau
à utiliser dans un procédé de traitement d'une plaie chronique non cicatrisante,
dans laquelle la composition pharmaceutique augmente le pourcentage moyen de changement dans la fermeture de la plaie par rapport à un témoin, le témoin étant le biomatériau hydrogel ;
dans laquelle :
(a) le biomatériau hydrogel comprend de l'eau purifiée, du glycérol, de l'hydroxyléthylcellulose, du lactate de sodium et de l'allantoïne, dans laquelle :
(i) l'eau purifiée représente environ 70% du biomatériau hydrogel ; et
(ii) le glycérol représente environ 30% du biomatériau hydrogel ; et
(b) la composition pharmaceutique est administrée :
(i) sous la forme d'une formulation topique ; ou
(ii) en tant que pansement, dans lequel une surface du pansement est imprégnée de la composition.

2. Composition pharmaceutique à utiliser selon la revendication 1, dans laquelle la composition pharmaceutique augmente le pourcentage moyen de changement de la fermeture de la plaie par rapport au témoin, de préférence dans lequel le pourcentage moyen de changement de la fermeture de la plaie va de 50% à 100% par rapport au témoin, tel qu'au moins 50%, 95% ou 99% de plus que le témoin.

3. Composition pharmaceutique à utiliser selon la revendication 1, dans laquelle la composition pharmaceutique est imprégnée sur au moins une surface du pansement, de préférence dans laquelle le pansement comprend :
(a) un pansement occlusif choisi dans le groupe constitué par : un pansement film, un pansement film semi-perméable, un pansement hydrogel, un pansement hydrocolloïde et une combinaison de ceux-ci ; et
(b) un tampon non adhérent.

4. Composition pharmaceutique à utiliser selon la revendication 1, dans laquelle le pourcentage moyen de fermeture de la plaie est le pourcentage moyen de changement de la surface de la plaie, le pourcentage moyen de changement du volume de la plaie ou une combinaison de ceux-ci, de préférence dans lequel le pourcentage moyen de changement de la surface de la plaie, le pourcentage moyen le changement du volume de la plaie, ou leur combinaison, est déterminé par un système de caméra tridimensionnel.

5. Composition pharmaceutique à utiliser selon la revendication 1, dans laquelle la composition pharmaceutique comprend en outre au moins un agent thérapeutique supplémentaire.

6. Composition pharmaceutique à utiliser selon la revendication 5, dans laquelle l'au moins un agent thérapeutique supplémentaire est un agent anti-inflammatoire, un agent analgésique, un agent anti-infectieux, un facteur de croissance ou une combinaison de ceux-ci, de préférence dans lequel l'agent anti infectieux est un agent antibiotique.

7. Composition pharmaceutique à utiliser selon la revendication 6, dans laquelle le facteur de croissance est choisi dans le groupe constitué du facteur de croissance dérivé des plaquettes (PDGF), du facteur de croissance des fibroblastes (FGF) et du facteur de stimulation des colonies de macrophages granulocytaires (GM-CSF).

8. Composition pharmaceutique à utiliser selon la revendication 1, dans laquelle la composition pharmaceutique est administrée en combinaison avec une thérapie d'appoint.

9. Composition pharmaceutique à utiliser selon la revendication 8, dans laquelle la thérapie d'appoint est l'oxygène hyperbare (HBO), la thérapie génique, la thérapie par cellules souches, une peau bio-conçue, un équivalent de peau, un substitut de peau, une greffe de peau ou une combinaison de ceux-ci.

10. Composition pharmaceutique à utiliser selon la revendication 1, dans laquelle la plaie chronique non cicatrisante est une plaie diabétique, une plaie veineuse, une plaie chirurgicale, une plaie cancéreuse, une escarre, un ulcère artériel ou une de leurs combinaisons.

11. Composition pharmaceutique à utiliser selon la revendication 1, dans laquelle la composition pharmaceutique diminue :
(a) l'œdème des plaies et des tissus ;
(b) le tissu nécrotique ; et
(c) la douleur dans la plaie chronique non cicatrisante.

12. Composition pharmaceutique à utiliser selon la revendication 1, dans laquelle la composition pharmaceutique :
(a) tue les microorganismes dans la plaie chronique non cicatrisante ;
(b) accélère la cicatrisation de la plaie chronique non cicatrisante ;
(c) augmente la synthèse du collagène et la néovascularisation dans la plaie chronique non cicatrisante ;
(d) élimine :
(i) la fibrine ;
(ii) les escarres ; et
(iii) le biofilm dans la plaie chronique non cicatrisante ;
(e) augmente le tissu de granulation dans la plaie chronique non cicatrisante ;
(f) accélère l'épithélialisation dans la plaie chronique non cicatrisante ;
(g) draine la plaie chronique non cicatrisante ;
(h) éloigne le liquide inflammatoire de la plaie chronique non cicatrisante ;
(i) contrôle l'infection du lit de la plaie dans la plaie chronique non cicatrisante ; ou
(j) réalise la préparation du lit de la plaie dans la plaie chronique non cicatrisante.
